# EUROPEAN PATENT SPECIFICATION

(11) **EP 3 986 879 B1**
(45) Date of publication and mention of the grant of the patent: **02.10.2024**
(21) Application number: 20734185.0
(22) Date of filing: 22.06.2020
(51) Int. Cl.: C07D 275/06, C07D 279/08, C07D 513/04, A61K 31/428, A61K 31/429, A61P 3/10, A61P 11/06, A61P 25/28, A61P 29/00, A61P 19/02, A61P 25/00, A61P 25/16

(54) **FUSED 1,2-THIAZOLES AND 1,2-THIAZINES WHICH ACT AS NLRP3 MODULATORS**
KONDENSIERTE 1,2-THIAZOLE UND 1,2-THIAZINE, DIE ALS NLRP3-MODULATOREN WIRKEN
1,2-THIAZOLES ET 1,2-THIAZINES FUSIONNÉS QUI AGISSENT EN TANT QUE MODULATEURS DE NLRP3

(30) Priority: 21.06.2019 EP 19181731
(43) Date of publication of application: 27.04.2022
(73) Proprietor: AC Immune SA, 1015 Lausanne (CH)
(72) Inventor: GABELLIERI, Emanuele, 1052 Le Mont-sur-Lausanne (CH); MOLETTE, Jérôme, 01280 Prévessin Moens (FR)
(74) Representative: Vossius & Partner Patentanwälte Rechtsanwälte mbB
(86) International application number: PCT/EP2020/067388
(87) International publication number: WO 2020/254697

(56) References cited:
- WO-A1-2016/131098
- WO-A1-2017/140778
- WO-A1-2018/215818
- WO-A1-2020/102100
- MAXIMILIAN BREMERICH ET AL: "Additions to N -Sulfinylamines as an Approach for the Metal-free Synthesis of Sulfonimidamides: O -Benzotriazolyl Sulfonimidates as Activated Intermediates", ANGEWANDTE CHEMIE INT ED, vol. 58, no. 52, 19 December 2019 (2019-12-19), DE, pages 19014 - 19020, XP055722907, ISSN: 1433-7851, DOI: 10.1002/anie.201911075
- YANTAO CHEN ET AL: "Saccharin Aza Bioisosteres-Synthesis and Preclinical Property Comparisons", ACS MEDICINAL CHEMISTRY LETTERS, vol. 8, no. 6, 8 June 2017 (2017-06-08), US, pages 672 - 677, XP055722909, ISSN: 1948-5875, DOI: 10.1021/acsmedchemlett.7b00137

## Description

### Field of the invention

The present invention relates to novel compounds that can be employed in the treatment, alleviation or prevention of a group of diseases, disorders and abnormalities responsive to modulation or inhibition of the activation of a component of the inflammasome pathway. In particular the component of the inflammasome pathway is from the NOD-like receptor (NLR) family such as pyrin domain-containing protein 3 (NLRP3) inflammasome. More particularly, the compounds of the present invention have the capability to inhibit the NLRP3 inflammasome. Further, the compounds of the present invention modulate, in particular decrease, IL-1 beta and/or IL-18 levels.

### Background of the invention

Inflammasome protein complexes are the key components of inflammatory signaling. These complexes assemble in response to various danger signals such as molecules from infectious agents (pathogen-associated molecular patterns, PAMPs) as well as altered host molecules, products of sterile tissue damage and environmental factors (danger associated molecular patterns, DAMPs). The inflammasome family consists of NALP1-14, IPAF, and NAIP 1-6 with each family member providing specificity towards different PAMPs/DAMPs including nucleic acids, bacterial proteins, metabolites, protein aggregates and the activity of toxins (Sharma, D. & Kanneganti, T.D. The cell biology of inflammasomes: mechanisms of inflammasome activation and regulation. J. Cell Biol. 213, 617-629 (2016)). Inflammasomes are typically composed of a sensor (a cytosolic pattern-recognition receptor, PRR) and an adaptor protein called apoptosis associated speck-like protein containing a caspase-recruitment domain (CARD) (ASC), and an effector such as the protease caspase-1 (Broz, P.; Dixit, V. M. Inflammasomes: Mechanism of Assembly, Regulation and Signalling. Nat. Rev. Immunol. 2016, 16, 407-420).

NLRP3 (NOD-like receptor (NLR) family, pyrin domain-containing protein 3) inflammasome is one of the best-described family members. It is a tripartite protein of the NLR family and contains an amino-terminal PYRIN (PYD) domain, a nucleotide-binding NACHT domain and acarboxy-terminal leucine-rich repeat (LRR) domain. In response to various agents including aggregated proteins, crystals and altered cellular ion homeostasis the NLRP3 sensor molecule assembles into a multimolecular complex with apoptosis-associated speck-like protein containing a caspase activation and recruitment domain (ASC also known as PYCARD) adaptor protein. ASC protein polymerization into a large complex (ASC speck) leads to activation of caspase-1 effector protein and subsequent cleavage of pro-IL1beta and pro-IL18 into their active secreted forms and mediates pyroptosis (Heneka et al., 2018 Nat Rev Neurosci). IL-1beta acts through IL-1beta receptors and induces secondary pro-inflammatory signals including IL-6 and TNF-alpha secretion and attracts and activates cells of adaptive immune system at the sites of infection. NLRP3/ASC complexes can be released into the extracellular environment where they can propagate inflammation.

Multiple genetic and pharmacological evidence highlights the importance of NLRP3 inflammasome in human disease. NLRP3 gain-of-function mutations lead to the inherited cryopyrin-associated periodic syndromes (CAPS) including Muckle-Wells syndrome (MWS), familial cold autoinflammatory syndrome and neonatal-onset multisystem inflammatory disease. Accumulation of tissue damage products associated with ageing results in activation of NLRP3 inflammasome in multiple diseases including metabolic disorders, Alzheimer's disease, Parkinson's disease, amyotrophic lateral sclerosis, atherosclerosis, obesity, lung diseases, liver diseases and gout among others.

Vast experimental evidence from animal models points out the detrimental role of excessive NLRP3 activation in a wide spectrum of diseases. NLRP3-inflammasome genetic or pharmacological downregulation showed protection in models of Alzheimer's disease, Parkinson's disease, amyotrophic lateral sclerosis, demyelination, viral encephalitis, epilepsy, stroke, atherosclerosis, asthma, allergic inflammation, CAPS, gout, inflammatory bowel disease, nonalcoholic fatty liver disease, nonalcoholic steatohepatitis, hypertension, myocardial infarction, oxalate-induced nephropathy, graft-versus host disease, type 1 and type 2 diabetes, rheumatoid arthritis, myelodysplastic syndrome among others (Heneka et al., Nat Rev Neurosci. 2018 Oct;19(10):610-621; Mangan et al., Nat Rev Drug Discov. 2018 Aug;17(8):588-606).

For the reasons described above modulation of NLRP3 inflammasome activity represents a promising therapeutic approach.

Current treatments for NLRP3-related diseases include biologics targeting IL-1. These are the recombinant IL-1 receptor antagonist anakinra, the neutralizing IL-1β antibody canakinumab and the soluble decoy IL-1 receptor rilonacept. These approaches have proven successful in the treatment of CAPS, and these biologic agents have been used in clinical trials for other IL-β-associated diseases. However their activity is limited to downstream, effectors of inflammasome and their bioavailability for central nervous system (CNS) applications is limited.

Several small molecules have been shown to inhibit the NLRP3 inflammasome (Baldwin, A. G., Brough, D. & Freeman, S. Inhibiting the inflammasome: a chemical perspective. J. Med. Chem. 59, 1691-1710 (2016); reviewed in Mangan et al., Nat Rev Drug Discov. 2018 Aug;17(8):588-606).

These include various chemical classes such as sulfonylurea-based compounds (glyburide, CP-456,773 (aka CRID3 and MCC950) and its derivatives); fenamate classes of non-steroidal anti-inflammatory drugs; hydroxysulfonamide analogue JC-171; novel boron compound series; benzimidazole-containing structure Fc11a-2; polyketide spirodalesol; acrylate and acrylamide derivatives; 3,4-methylenedioxy-β-nitrostyrene; β-sulfonyl nitrile molecule OLT1177; CY-09; BOT-4-one; and Michael acceptors. Most of these compounds have a promiscuous mode of action and limited potency. Therefore there is a need to identify and develop specific NLRP3 inflammasome inhibitors with improved pharmacological and/or physiological and or physicochemical properties.

WO9832733 refers to aryl and heteroaryl substituted sulfonyl ureas that are useful inhibitors of interleukin-1α and interleukin-1β processing and release.

WO2016131098, WO2017/140778 and WO2018215818 refer to sulfonylurea and related compounds and their use in treating, or identifying a disease or condition responsive to inhibition of NLRP3 or inhibition of the activation of NLRP3 or related components of the inflammatory process.

WO2017184604, WO2017184623, WO2017184624, WO2019023145, WO2019023147 and WO2019079119 refer to chemical entities that are useful for treating a condition, disease or disorder in which a decrease or increase in NLRP3 activity contributes to the pathology and/or symptoms and/or progression of the condition, disease or disorder in a subject.

WO2018136890 refers to sulfonylurea and sulfonyl thiourea compounds and their use in treating a disease or condition responsive to modulation of cytokines such as IL-1β and IL-18, modulation of NLRP3 or inhibition of the activation of NLRP3 or related components of the inflammatory process.

WO2018225018 and WO2019043610 refer to NLRP3 modulators as well as to the use of the novel inhibitor compounds in the treatment of diseases or conditions as well as treatment of disease states mediated by NLRP3 as well as treatment of diseases or conditions in which interleukin 1β activity and interleukin-18 (IL-18) are implicated.

WO2019008025, WO2019008029, WO2019034686, WO2019034688, WO2019034690, WO2019034692, WO2019034693, WO2019034696, WO2019034697, WO2019068772, WO2019092170, WO2019092171 and WO2019092172 refer to novel compounds (e.g. sulfonylureas, sulfonylthioureas, sulfoximine ureas and sulfoximine thioureas), useful in the treatment and prevention of medical disorders and diseases, most especially by NLRP3 inhibition.

WO2018015445 refers to sulfonylurea compounds, or pharmaceutically-acceptable salt(s) thereof, which possess inflammasome inhibitory activity and are accordingly useful in methods of treatment of the human or animal body.

WO2020018975 discloses sulfonimidamide derivatives defined as inhibitors of interleukin-1 activity and NLRP3 modulators in connection with inflammatory processes.

WO9832733 refers to aryl and heteroaryl substituted sulfonyl ureas that are inhibitors of interleukin-1 alpha (α) and interleukin-1 beta (β) processing and release.

WO2020018970 discloses sulfonylureas defined as inhibitors of interleukin-1 activity.

The crosstalk between the NLRP3 inflammasome and Tau pathology has been recently deciphered. Ising et al. (Nature 2019 Nov;575(7784):669-673) relate the important role of microglia and NLRP3 inflammasome activation in the pathogenesis of tauopathies in the Tau22 mouse model of FrontoTemporal Dementia (FTD). Genetic ablation of components of the NLRP3 inflammasome in Tau22 mice reduced Tau aggregation/phosphorylation as well as improved cognition. Stancu et al. (Acta Neuropathol. 2019; 137(4): 599-617) investigated the role of inflammasome activation in prion-like or templated seeding of Tau pathology. Significant inhibition of exogenously seeded Tau pathology was found in ASC deficient - PS19 Tau transgenic mice. Furthermore it was demonstrated that chronic intracerebral administration of the NLRP3 inhibitor, MCC950, inhibits exogenously seeded Tau pathology. Finally, ASC deficiency also decreased nonexogenously seeded Tau pathology in PS19 mice.

WO 2020/102100 features chemical entities that are stated to be useful for treating a condition, disease or disorder in which a decrease or increase in NLRP3 activity contributes to the pathology and/or symptoms and/or progression of the condition, disease or disorder in a subject.

Maximilian Bremerich et al. describe additions to N-sulfinylamines as an approach for the metal-free synthesis of sulfonimidamides with O-benzotriazolyl sulfonimidates as activated intermediates (Angew. Chem. Int. Ed., 2019, 58, 19014-19020.)

Yantao Chen et al. report the synthesis and preclinical property comparisons of saccharin aza bioisosteres (ACS Med. Chem. Lett. 2017, 8, 672-677).

There is a need to identify and develop specific NLRP3 inflammasome inhibitors and/or modulators of interleukin activity with improved pharmacological and/or physiological and/or physicochemical properties.

The present invention surprisingly provides compounds of formula (I) capable of modulating NLRP3 inflammasome. Thus, such compounds are beneficial in the treatment of diseases or abnormalities associated with altered IL-18 levels that commonly lead to pathological inflammation.

### Summary of the invention

It was an object of the present invention to provide compounds that can be employed in the treatment, alleviation or prevention of a group of diseases, disorders and abnormalities responsive to modulation or inhibition of the activation of a component of the inflammasome pathway. In particular the component of the inflammasome pathway is NLRP3 inflammasome. The present inventors have surprisingly found that these objects can be achieved by the compounds of formula (I) as described hereinafter.

The compounds of formula (I) display a high capability in modulating or in inhibiting the activation of a component of the inflammasome pathway, in particular where the component of the inflammasome pathway is NLRP3 inflammasome. Due to their unique design features, these compounds display properties such as appropriate lipophilicity and molecular weight, brain uptake and pharmacokinetics, cell permeability, solubility and metabolic stability, in order to be a successful medicament for the treatment, alleviation or prevention of diseases, disorders and abnormalities responsive to inhibition of activation of the NLRP3 inflammasome.

The present invention discloses novel compounds of formula (I) having capabilities to inhibit the NLRP3 inflammasome. The present invention provides methods for the treatment of diseases, disorders and abnormalities associated with NLPR3 inflammasome, using a compound of formula (I) or a pharmaceutical composition thereof. The present invention further provides a pharmaceutical composition comprising a compound of formula (I) and at least one selected from pharmaceutically acceptable excipients, carriers, diluents and adjuvants. The present invention also provides such compounds for use in the treatment, alleviation or prevention of a disease, disorder or abnormality responsive to modulation or inhibition of the activation of a component of the inflammatory pathway.

The present invention is as described in the appended claims.

### Definitions

Within the meaning of the present application the following definitions apply:
"Alkyl" refers to a saturated straight or branched organic moiety consisting of carbon and hydrogen atoms. Examples of suitable alkyl groups have 1 to 6 carbon atoms, preferably 1 to 4 carbon atoms, and include methyl, ethyl, propyl, isopropyl, n-butyl, t-butyl and isobutyl. Within the present invention any alkyl group can be optionally substituted with one or more (preferably 1 or 2) substituents defined below as "optional substituent". The above definition of alkyl also applies if this moiety is part of a larger moiety such as "alkoxy". The same applies to the other moieties such as cycloalkyl, cycloalkenyl, aryl, heteroaryl, heterocyclyl, etc.

"Hal" or "halogen" refers to F, Cl, Br, and I.

"Cycloalkyl" refers to saturated monocyclic, bicyclic or tricyclic hydrocarbyl groups (each cycle having 3 to 8 ring carbon atoms). "Cycloalkenyl" refers to unsaturated, non-aromatic monocyclic, bicyclic or tricyclic hydrocarbyl groups (each cycle having 3 to 8 ring carbon atoms) having at least one (preferably 1 or 2) carbon-carbon double bonds within one ring. Examples thereof may be selected from the group consisting of cyclopropyl, cyclobutyl, cyclopentyl, cyclopentenyl, cyclohexyl, cyclohexenyl, cyclohexadienyl and the like. Any cycloalkyl or cycloalkenyl within this invention may be optionally substituted with one or more (preferably 1 or 2) substituents defined below as "optional substituents"; said optional substituents include for instance halo (e.g., Cl, F, Br, and I); halogenated alkyl (e.g., CF₃, 2-Br-ethyl, CH₂F,CH₂Cl, CH₂CF₃, or CF₂CF₃); hydroxyl; amino; carboxylate; carboxamido; alkylamino; arylamino; alkoxy; aryloxy; nitro; azido; cyano; thio; sulfonic acid; sulfate; phosphonic acid; phosphate; and phosphonate.

"Aryl" refers to a stable monocyclic, bicyclic, or tricyclic carbon ring of up to 8 members in each ring, wherein at least one ring is aromatic as defined by the Huckel rule. The term includes polycyclic systems comprising saturated carbon rings or heteroaryl or heterocyclic groups as long as at least one ring is an aromatic ring with up to 8 carbon ring atoms. Within the present invention any aryl group can be optionally substituted with one or more (preferably 1 or 2) substituents defined below as "optional substituent".

"Heteroaryl" refers to an aryl group (preferably with up to 8 ring members in each ring) containing one or more (particularly one to four) non-carbon ring atom(s) (particularly N, O or S or any combination thereof) in at least one ring therof. Heteroaryl rings may also be fused with one or more cyclic hydrocarbon, heterocyclic, aryl, or heteroaryl rings. Heteroaryl includes, but is not limited to, 5-membered heteroaryls having one heteroatom (e.g., thiophenes, pyrroles, furans); 5-membered heteroaryls having two heteroatoms in 1,2 or 1,3 positions (e.g., oxazoles, pyrazoles, imidazoles, thiazoles, purines); 5-membered heteroaryls having three heteroatoms (e.g., triazoles, thiadiazoles); 5-membered heteroaryls having four heteroatoms (e.g., tetrazoles); 6-membered heteroaryls with one heteroatom (e.g., pyridine, quinoline, isoquinoline, phenanthrine, 5,6-cycloheptenopyridine); 6-membered heteroaryls with two heteroatoms (e.g., pyridazines, cinnolines, phthalazines, pyrazines, pyrimidines, quinazolines); and 6-membered heretoaryls with three heteroatoms (e.g., 1,3,5-triazine). Within the present invention any heteroaryl group can be optionally substituted at one or more carbon ring atom and/or hetero ring atom with a substituent defined below as "optional substituent".

"Heterocyclyl" refers to a non-aromatic ring having 4 to 8 atoms in the ring and of those atoms 1 to 4 are heteroatoms (which can be independently selected from N, S and O). Heterocyclic rings may also be fused with one or more cyclic hydrocarbon, heterocyclic, aryl, or heteroaryl rings. Heterocyclic includes partially and fully saturated heterocyclic groups. Heterocyclic systems may be attached to another moiety via any number of carbon atoms or heteroatoms of the radical and may be both saturated and unsaturated. Non-limiting examples of heterocyclic moieties include pyrrolidinyl, pyrrolinyl, pyranyl, piperidinyl, piperazinyl, morpholinyl, tetrahydrofuranyl, tetrahydrothiophenyl, pyrazolinyl, dithiolyl, oxathiolyl, dioxanyl, dioxinyl, oxazinyl, azepinyl, diazepinyl, thiazepinyl, oxepinyl and thiapinyl, imidazolinyl, thiomorpholinyl, oxetanyl and the like.

"Optionally substituted" in reference to a certain group refers to said group as to optionally be substituted with one or more substituents (i.e., the substituent may be present or not). Such "optional substituents" may be selected from the group consisting of optionally substituted alkyl; optionally substituted cycloalkyl (e.g., optionally substituted cyclopropyl); optionally substituted hydroxyalkyl; optionally substituted alkoxy (e.g., optionally substituted alkoxy); optionally substituted alkenyl; optionally substituted alkynyl; optionally substituted aryl; aryloxy; optionally substituted heteroaryl; optionally substituted heterocyclyl; halo (e.g., F, Cl, Br, and I); hydroxyl; halogenated alkyl (e.g., CH₂F, CHF₂, CF₃, 2-Br-ethyl, CH₂CF₃, and CF₂CF₃); amino (e.g., NH₂, NR₃₀H, and NR₃₀R₃₁); alkylamino; arylamino; acyl; amido; CN; N₃; NO₂; CH₂OH; CONH₂; CONR₃₂R₃₃; CO₂R₃₂; CH₂OR₃₂; NHCOR₃₂; NHCO₂R₃₂; alkylthio; sulfate; sulfonic acid; sulfonate esters such as alkyl or aralkyl sulfonyl; phosphonic acid; phosphate; phosphonate; mono-, di-, or triphosphate esters; trityl or monomethoxytrityl; R₃₂SO; R₃₂SO₂; CF₃S; and CF₃SO₂; trialkylsilyl such as dimethyl-t-butylsilyl or diphenylmethylsilyl; wherein R₃₀, R₃₁, R₃₂ and R₃₃ are each independently selected from H and optionally substituted alkyl.

Compounds of the present invention having one or more optically active carbons can exist as racemates and racemic mixtures (including mixtures in all ratios), stereoisomers (including diastereomeric mixtures and individual diastereomers, enantiomeric mixtures and single enantiomers, mixtures of conformers and single conformers), tautomers, atropisomers, and rotamers. All isomeric forms are included in the present invention. Compounds described in this invention containing olefinic double bonds include E and Z geometric isomers. Also included in this invention are all pharmaceutically acceptable salts, polymorphs, hydrates and solvates of compounds of formula (I).

Tautomers are isomers of a compound which differ only in the position of the protons and electrons. The skeleton of the compound is unchanged. Common tautomeric pairs include: ketone - enol (H-O-C=CH 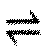 O=C-CH₂) and enamine - imine (H₂N-C=N 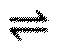 HN=C-NH). For the compounds of the present invention the tautomeric pairs are the following:

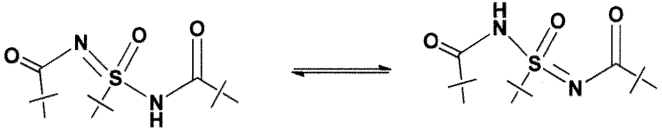

The term "polymorphs" refers to the various crystalline structures of the compounds of the present invention. This may include, but is not limited to, crystal morphologies (and amorphous materials) and all crystal lattice forms. Salts of the present invention can be crystalline and may exist as more than one polymorph.

Solvates, hydrates as well as anhydrous forms of the salt are also encompassed by the invention. The solvent included in the solvates is not particularly limited and can be any pharmaceutically acceptable solvent. Examples include water and C₁₋₄ alcohols (such as methanol or ethanol).

"Pharmaceutically acceptable salts" are defined as derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as, but not limited to, hydrochloric, hydrobromic, sulfuric, sulfamic, phosphoric, nitric acid and the like; and the salts prepared from organic acids such as, but not limited to, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic acid, and the like. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Organic solvents include, but are not limited to, nonaqueous media like ethers, ethyl acetate, ethanol, isopropanol, or acetonitrile. Lists of suitable salts can be found in Remington's Pharmaceutical Sciences, 18th ed., Mack Publishing Company, Easton, PA, 1990, p. 1445, the disclosure of which is hereby incorporated by reference.

"Pharmaceutically acceptable" is defined as those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio.

The "effective amount" of the compound which is to be administered to a subject is the dosage which according to sound medical judgement is suitable for treating, preventing or alleviating the disease, disorder or abnormality. The specific dose level and frequency of dosage can depend, e.g., upon a variety of factors including the activity of the specific compound employed, the metabolic stability and length of action of that compound, mode and time of administration, the rate of excretion, and drug combination. Patient-specific factors such as the age, body weight, general health, sex, diet, as well as the severity of the particular condition can also influence the amount which is to be administered.

The patients or subjects in the present invention are typically animals, particularly mammals, more particularly humans.

"NLRP3" as used herein refers to NOD-like receptor (NLR) family, pyrin-domain containing protein 3 component of inflammasome. Inflammasomes are intracellular supramolecular complexes comprising a sensor molecule, the adaptor apoptosis-associated speck-like protein containing a CARD (ASC) and the effector protease caspase 1. Upon activation of the inflammasome sensor molecule, ASC self-associates into a helical fibrillary assembly resulting in formation of the so-called ASC speck or pyroptosome, which acts as a molecular platform for the activation of pro-caspase 1 via proximity-induced autocatalytic activation. Active caspase 1 triggers the activation and release of interleukin-1 (IL-1) family proteins and enables the nonconventional secretion of numerous cytosolic proteins. Among the pro-inflammatory mediators released upon NLRP3 activation are IL-1β, IL-18, high-mobility group protein B1 (HMGB1), leukotrienes and prostaglandins.

"Tau Aggregation Inhibitor" means small molecule compounds that bind to Tau and directly reduce Tau aggregation or promote disaggregation of pre-formed Tau aggregates.

"Alpha-synuclein aggregation inhibitor" means small molecule compounds that bind to alpha-synuclein and directly reduce alpha-synuclein aggregation or promote disaggregation of pre-formed alpha-synuclein aggregates.

"TDP-43 aggregation inhibitor" means small molecule compounds that bind to TDP-43 and directly reduce TDP-43 aggregation or promote disaggregation of pre-formed TDP-43 aggregates.

| **Abbreviation** | **Meaning** |
|---|---|
| NALP1-14 | Nacht Leucine-rich-repeat Protein 1-14 (it is a synonym of NLRP) |
| IPAF | Ice Protease-Activating Factor |
| NAIP | Neuronal Apoptosis Inhibitory Protein |
| ASC | Apoptosis-associated Speck-like protein containing a CARD |
| nucleotide-binding NACHT domain | NACHT : NAIP (neuronal apoptosis inhibitory protein), CIITA (MHC class II transcription activator), HET-E (incompatibility locus protein from Podospora anserina) and TP1 (telomerase-associated protein) |
| IL | Interleukin |
| TNF-alpha | Tumor Necrosis Factor - alpha |

The definitions and preferred definitions given in the "Definition"-section apply to all of the embodiments described below unless stated otherwise.

### Detailed description of the invention

The compounds of the present invention will be described in the following. It is to be understood that all possible combinations of the following definitions are also envisaged.

In one embodiment, the present invention relates to a compound of formula (I): 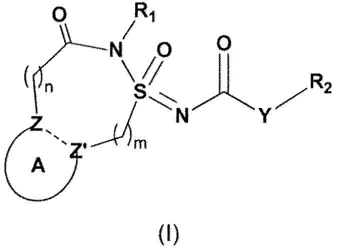 or stereoisomers, racemic mixtures, tautomers, pharmaceutically acceptable salts, hydrates, solvates and polymorphs thereof.

For **R₁** being hydrogen tautomeric rearrangement is possible resulting for instance in compounds of the following formula (Ia) which is subject matter of a preferred embodiment

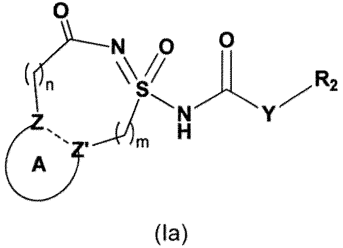

Further preferred embodiments of the compounds of formula (Ia) are compounds of the following formula (Ib)

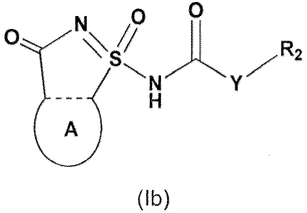

Further preferred embodiments of the compounds of formula (Ia) and (lb) are compounds of the following formula (Ic)

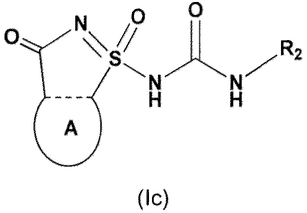

The following definitions apply to formulae (I), (Ia), (Ib) and (Ic) as appropriate.

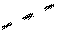 is a single or double bond.

**A** is selected from the group consisting of 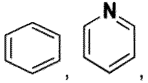 and 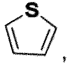 wherein each of these rings can be optionally substituted.

The optional substituent of **A** is preferably selected from the group consisting of optionally substituted C1-6 alkyl, halogen, hydroxyalkyl, NHₛ(alkyl)ₜ (wherein each of s and t can be 0, 1 or 2 as long as s+t=2;and wherein alkyl is C1-10 alkyl), optionally substituted heterocycloalkyl, optionally substituted cycloalkyl, haloalkyl and CN. More preferably the optional substituent of **A** can be

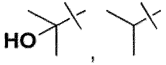

**R₁** is selected from the group consisting of hydrogen, alkyl and heterocyclyl (e.g. oxetanyl), preferably hydrogen and alkyl (e.g. Me); if R₁ is hydrogen tautomeric rearrangement is possible. Alkyl and heterocyclyl can be optionally substituted.

**R₂** is selected from the following ring systems 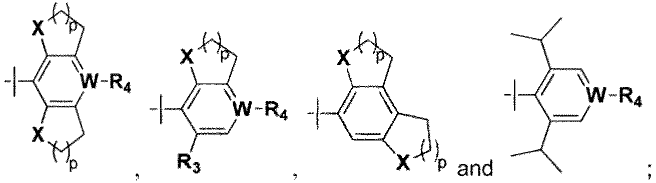 preferably it is selected from the following ring structures 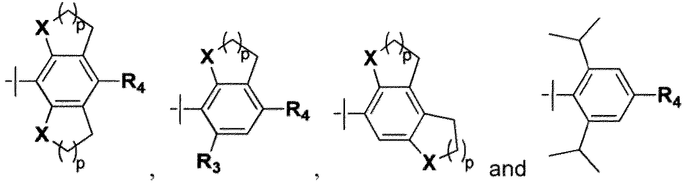 and more preferably **R₂** is selected from 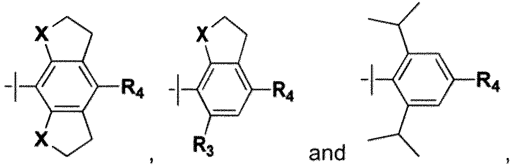 even more preferably **R₂** is selected from

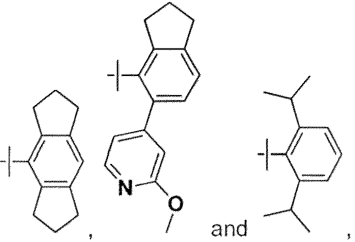

**R₃** is an optionally substituted heteroaryl, more preferably an optionally substituted pyridine; preferred substituents are -Oalkyl (e.g. -OCH₃).

**R₄** is selected from the group consisting of hydrogen, halogen, alkyl, alkyl-O-alkyl, aryl and heteroaryl. More preferably **R₄** is selected from the group consisting of hydrogen and halogen.

**W** is selected from the group consisting of C and N.

**X** are independently selected from the group consisting of CH₂ and O. More preferably **X** is CH₂.

**Y** is NH.

Each **R** is independently selected from the group consisting of hydrogen, halogen, CH₃, CH₂F, CHF₂, CF₃.

**R₁** is selected from the group consisting of hydrogen, alkyl and heterocyclyl, wherein alkyl and heterocyclyl can be optionally substituted; preferably **R₁** is hydrogen.

**m** is 0 or 1.

**n** is 0 or 1.

Preferably **n** and **m** are selected such that a 5- or 6-membered ring results.

Each **p** is independently 0 or 1; preferably at least one **p** is 1 and more preferably both **p** are 1.

The compounds of formula (I) include a moiety of the following formula 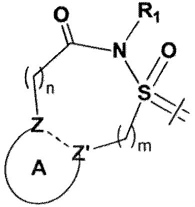 which is preferably selected from the group consisting of

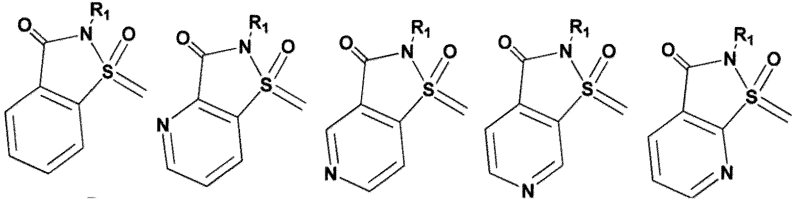

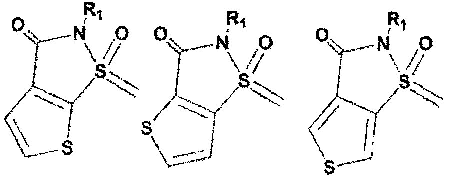

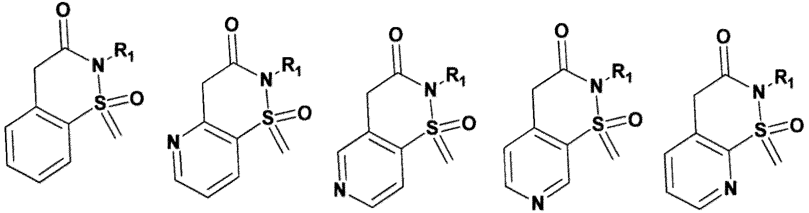

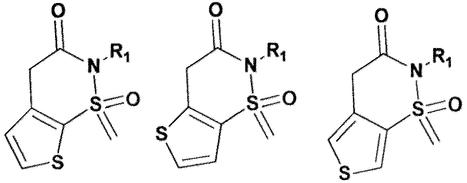

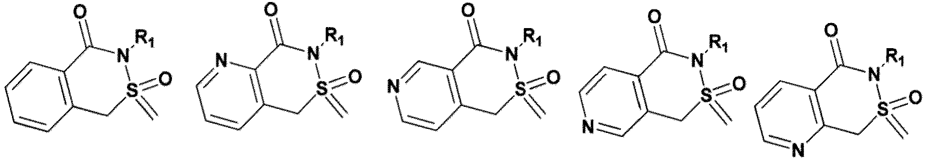

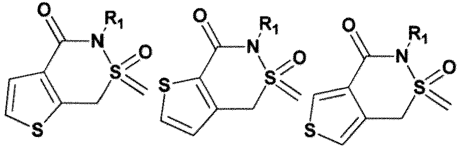

The compounds of formula (Ia) include a moiety of the following formula 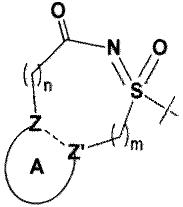 which is preferably selected from the group consisting of

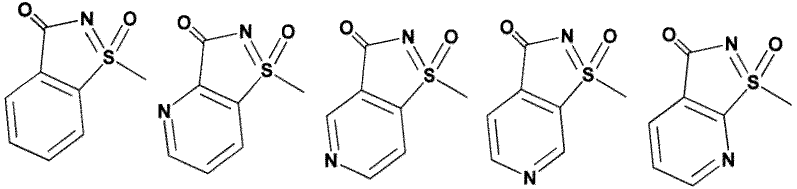

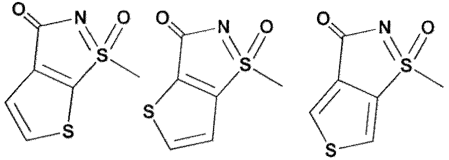

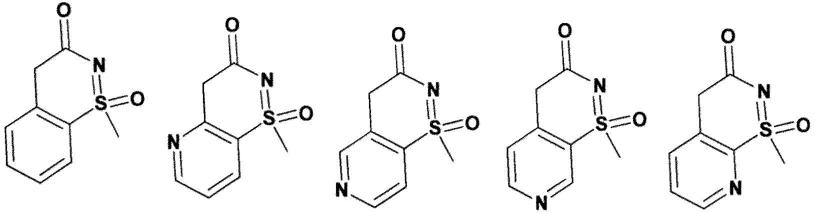

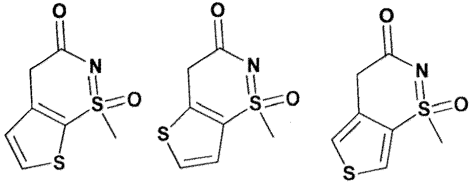

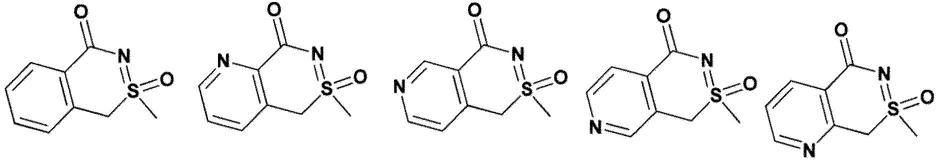

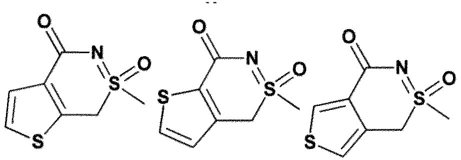

The moiety of the formula 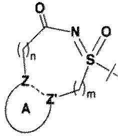 is preferably a moiety of the formula 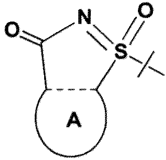 that is preferably selected from the group consisting of

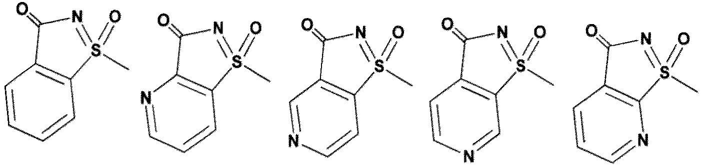

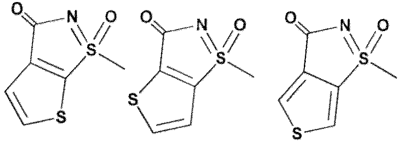

The ring **A** in the moieties 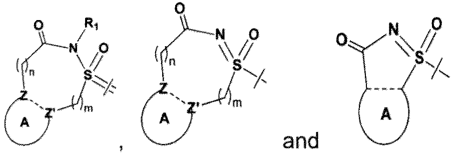 can be optionally substituted, meaning that any H included in the ring **A** can be replaced by a substituent.

Examples of compounds of formula (I) are

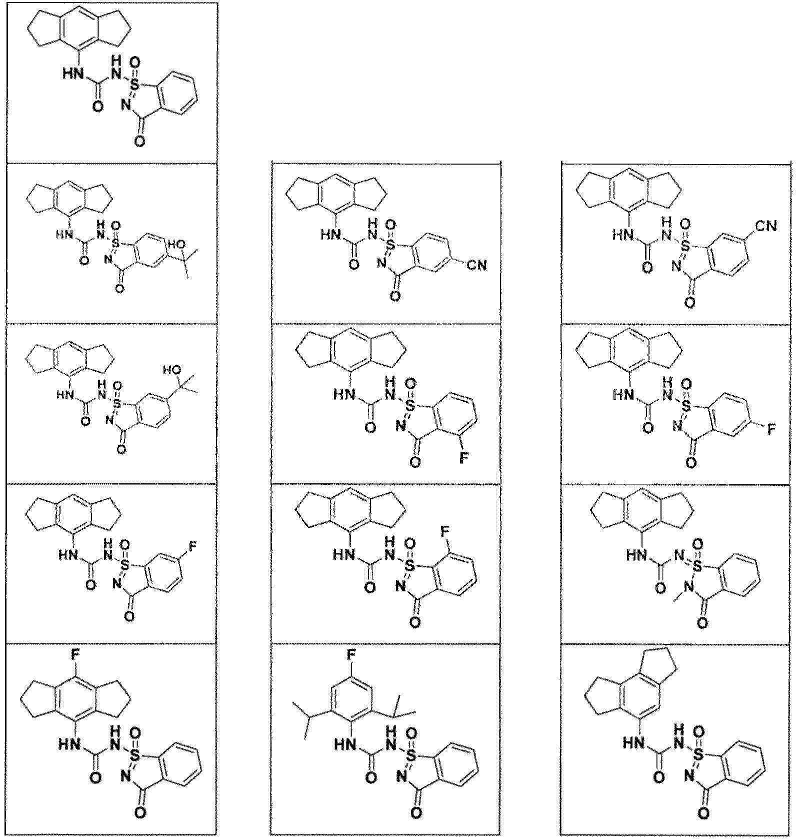

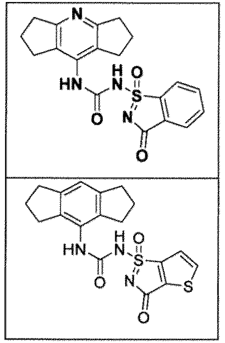

Preferred embodiments of the compound of formula (I) are as follows

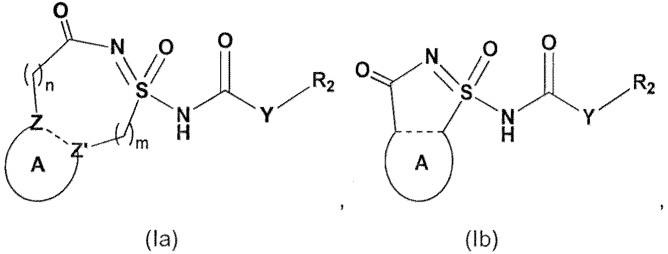

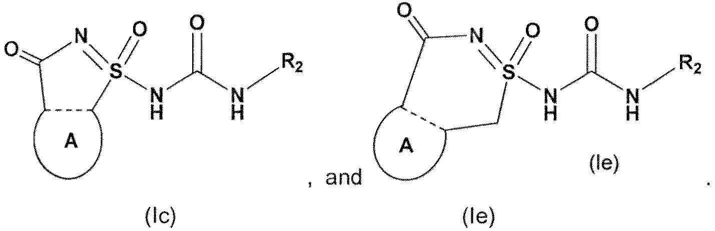

The following definitions apply to formulae (I), (Ia), (Ib), (Ic), (Id) and (Ie) as appropriate:
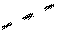 is a single or double bond, preferably a double bond.

**A** is selected from the group consisting of 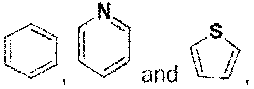 wherein each of these rings can be optionally substituted. Preferably, **A** is selected from the group consisting of 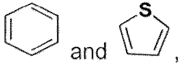 wherein each of these rings can be optionally substituted. More preferably, **A** is 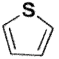 which can be optionally substituted.

It is understood that the aryl and heteroaryl can be attached at any available position.

The optional substituent of **A** is preferably selected from the group consisting of optionally substituted alkyl (such as methyl or isopropyl), halogen (such as Cl, F or Br), NHₛ(alkyl)ₜ (wherein each of s and t can be 0, 1 or 2 as long as s + t = 2), optionally substituted heteroaryl, optionally substituted aryl, optionally substituted heterocycloalkyl, optionally substituted cycloalkyl, -CN, -C(O)O-alkyl (such as -C(O)O-methyl), -S-alkyl (such as -S-methyl), and - O-alkyl (such as -O-methyl). The optional substituent of **A** is more preferably selected from the group consisting of alkyl, halogen, hydroxyalkyl, NHₛ(alkyl)ₜ (wherein each of s and t can be 0, 1 or 2 as long as s + t = 2), optionally substituted heteroaryl, optionally substituted aryl, optionally substituted heterocycloalkyl, optionally substituted cycloalkyl, haloalkyl, -CN, -C(O)O-alkyl, - S-alkyl and -O-alkyl. The optional substituent of **A** is further more preferably selected from the group consisting of alkyl, halogen, hydroxyalkyl, NHₛ(alkyl)ₜ (wherein each of s and t can be 0, 1 or 2 as long as s + t = 2), optionally substituted heteroaryl, optionally substituted aryl, optionally substituted heterocycloalkyl, optionally substituted cycloalkyl, haloalkyl, -CN, -S-alkyl, and -O-alkyl. Even more preferably the optional substituent of **A** is selected from the group consisting of alkyl, halogen, haloalkyl, alkyl-OH, NHₛ(alkyl)ₜ (wherein each of s and t can be 0, 1 or 2 as long as s + t = 2), -CN, -C(O)O-alkyl, and -O-alkyl. Even more preferably, the optional substituent of **A** is selected from the group consisting of -C(O)O-methyl, -CN, -CI, -Br, -F, methoxy, 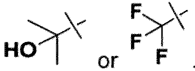 Most preferably the optional substituent of **A** can be 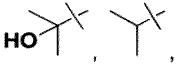 and CN.

In one embodiment, the optional substituent of **A** is selected from the group consisting of optionally substituted C1-6 alkyl, halogen, hydroxyalkyl, NHₛ(alkyl)ₜ (wherein each of s and t can be 0, 1 or 2 as long as s+t=2; and wherein alkyl is C1-10 alkyl), optionally substituted heterocycloalkyl, optionally substituted cycloalkyl, haloalkyl and CN. In a further embodiment, the optional substituent of **A** can be 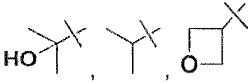 and CN.

**R₁** is selected from the group consisting of hydrogen, alkyl and heterocyclyl (e.g. oxetanyl). Preferably **R₁** is hydrogen or alkyl, more preferably hydrogen. If **R₁** is hydrogen tautomeric rearrangement is possible. Possible tautomers are shown, e.g. in formulae (Ia) to (le). Alkyl and heterocyclyl can be optionally substituted.

**R₂** is selected from the following ring systems

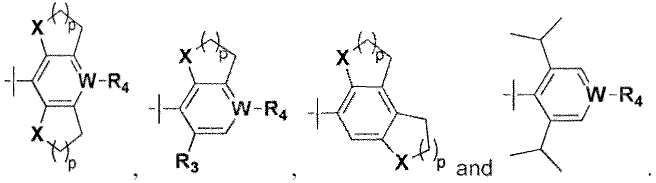

Preferably **R₂** is selected from the following ring structures 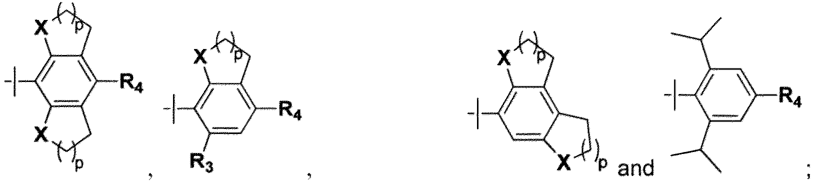 and more preferably **R₂** is selected from 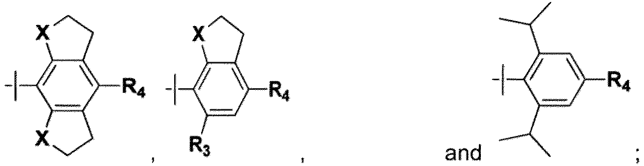 even more preferably **R₂** is selected from

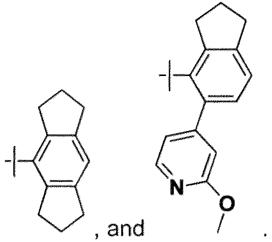

**R₃** is an optionally substituted heteroaryl, preferably an optionally substituted pyridine.

More preferably, **R₃** is optionally substituted pyridine, wherein the pyridine is, for example, substituted with -Oalkyl (e.g. -OCH₃).

The heteroaryl in **R₃** is preferably pyridine.

Preferred substituents of the heteroaryl in the **R₃** moiety are -Oalkyl (e.g. -OCH₃).

**R₄** is selected from the group consisting of hydrogen, halogen, alkyl, alkyl-O-alkyl, aryl and heteroaryl, for instance, **R₄** can be selected from the group consisting of hydrogen and halogen.

When **R₄** is alkyl, alkyl-O-alkyl, aryl or heteroaryl, it can be optionally substituted.

**W** is selected from the group consisting of C and N, preferably **W** is C. More preferably, when **W** is C then **R₄** is hydrogen. In the case of **W** being N, no **R₄** is present.

Each X is independently selected from the group consisting of CH₂ and O. More preferably **X** is CH₂.

**Y** is NH.

**Z** and **Z'** are independently selected from the group consisting of C and N, provided that when **m** is 0, **Z'** is C. Preferably **Z** and **Z'** are C.

Each **R** is independently selected from the group consisting of hydrogen, halogen, CH₃, CH₂F, CHF₂, CF₃, preferably **R** is hydrogen.

**m** is 0 or 1.

**n** is 0 or 1.

Preferably **n** and **m** are selected such that a 5- or 6-membered ring results.

Each **p**
is independently 0 or 1, preferably at least one **p** is 1.

In one embodiment, **R₂** is 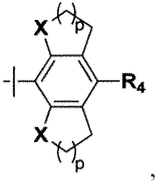
wherein **X** are independently selected from the group consisting of CH₂ and O,
each **p** is 1, and
**R₄** is selected from the group consisting of hydrogen, halogen, alkyl, alkyl-O-alkyl, aryl and heteroaryl.

In this embodiment, preferably, **X** is CH₂, and **R₄** is selected from the group consisting of hydrogen and halogen. In this embodiment, more preferably, **X** is CH₂,
and **R₄** is selected from the group consisting of hydrogen and F (fluoro).

In one embodiment, **R₂** is 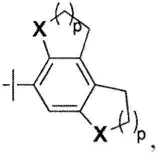 wherein **X** are independently selected from the group consisting of CH₂ and O, and each **p** is 1.

In this embodiment, preferably, **X** is CH₂.

In one embodiment, **R₂** is 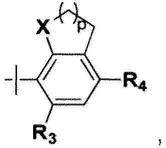
wherein **X** are independently selected from the group consisting of CH₂ and O,
each **p** is 1 ,
**R₄** is selected from the group consisting of hydrogen, halogen, alkyl, alkyl-O-alkyl, aryl, and heteroaryl, and
**R₃** is an optionally substituted heteroaryl.

In this embodiment, preferably, **X** is CH₂,
**R₃** is an optionally substituted heteroaryl, and **R₄** is selected from the group consisting of hydrogen and halogen. In this embodiment, more preferably, **X** is CH₂,
**R₃** is optionally substituted pyridine, wherein pyridine is preferably substituted with -Oalkyl (e.g. -OCH₃) and **R₄** is hydrogen or F (fluoro).

A further embodiment of the invention is directed to compounds of the following formula (Id) as far as they fall under the scope of the appended claims, 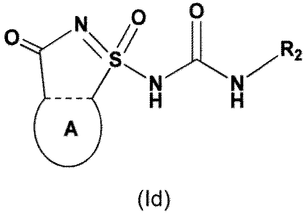 or stereoisomers, racemic mixtures, tautomers, pharmaceutically acceptable salts, hydrates, solvates and polymorphs thereof,
wherein
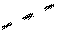 is a single or double bond,
**A** is selected from the group consisting of 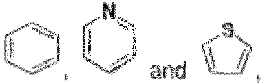
wherein each of these rings can be optionally substituted,
**R₂** is selected from the following ring systems 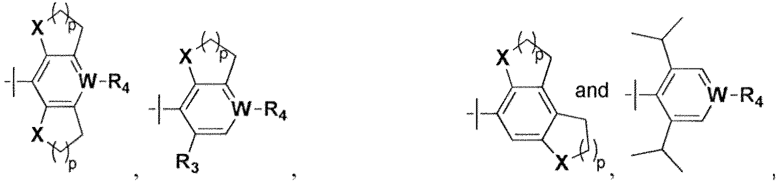
wherein
each **X** is independently selected from the group consisting of CH₂ and O,
each **p** is 1,
**W** is selected from the group consisting of C and N, in case of **W** being N no **R₄** is present,
**R₃** is an optionally substituted heteroaryl, and
**R₄** is selected from the group consisting of hydrogen, halogen, alkyl, alkyl-O-alkyl, aryl and heteroaryl.

Preferably, 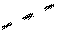 is a double bond.

**A** is selected from the group consisting of 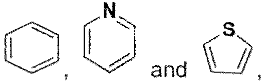 wherein each of these rings can be optionally substituted.

Preferably, **A** is selected from the group consisting of 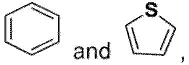 wherein each of these rings can be optionally substituted.

More preferably, **A** is 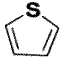 which can be optionally substituted.

The optional substituent of **A** is preferably selected from the group consisting of optionally substituted alkyl, halogen, hydroxyalkyl, NHₛ(alkyl)ₜ (wherein each of s and t can be 0, 1 or 2 as long as s + t = 2), optionally substituted heterocycloalkyl, optionally substituted cycloalkyl, haloalkyl, CN, alkyl, and alkoxy. More preferably, the optional substituent of **A** is selected from the group consisting of optionally substituted alkyl, preferably methyl or isopropyl; halogen, preferably Cl, F or Br; CN; alkyl carboxyl, preferably methyl carboxyl; and alkoxy, preferably methoxy. Further, alkyl can be optionally substituted with OH or halogen, preferably Cl, For Br. Even more preferably, the optional substituent of **A** is methyl carboxyl, CN, Cl, Br, F, methoxy,

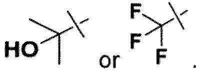

Preferably, **R₂** is selected from the following ring structures

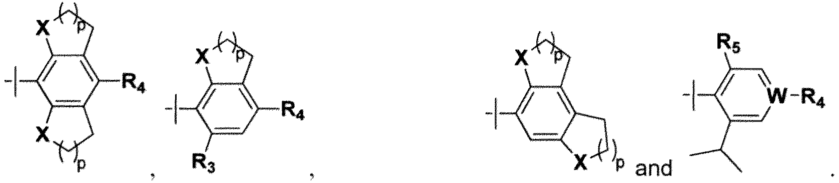

Preferably, X is CH₂.

Both p are 1.

**R₃** is an optionally substituted heteroaryl.

Preferably, **R₃** is an optionally substituted pyridine, wherein pyridine is preferably substituted with -Oalkyl (e.g. -OCH₃).

Preferably **R₄** is selected from the group consisting of hydrogen and halogen, preferably hydrogen or F (fluoro). More preferably, **R₄** is hydrogen.

Preferably, **W** is C. More preferably, when **W** is C then **R₄** is hydrogen.

In one embodiment, **R₂** is 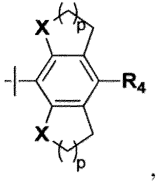 wherein
**X** are independently selected from the group consisting of CH₂ and O,
each **p** is 1 , and
**R₄** is selected from the group consisting of hydrogen, halogen, alkyl, alkyl-O-alkyl, carbocyclyl, heterocyclyl, aryl and heteroaryl.

Preferably, **X** is CH₂, and **R₄** is selected from the group consisting of hydrogen and halogen.

More preferably, **X** is CH₂, and **R₄** is selected from the group consisting of hydrogen and F (fluoro).

In one embodiment, **R₂** is 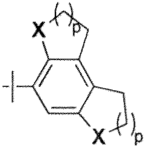 wherein **X** are independently selected from the group consisting of CH₂ and O, and each **p** is 1.

Preferably, **X** is CH₂.

In one embodiment, **R₂** is 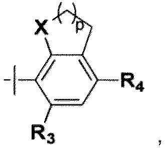
wherein **X** is selected from the group consisting of CH₂ and O,
   **p** is 1,
**R₄** is selected from the group consisting of hydrogen, halogen, alkyl, alkyl-O-alkyl, aryl and heteroaryl, and
**R₃** is an optionally substituted heteroaryl.

Preferably, **X** is CH₂, **R₃** is
an optionally substituted heteroaryl, and **R₄** is selected from the group consisting of hydrogen and halogen.

More preferably, **X** is CH₂, **R₃** is optionally substituted pyridine,
wherein pyridine is preferably substituted with -Oalkyl (e.g. -OCH₃) and **R₄** is hydrogen or F (fluoro).

Compounds of formula (I) are
Example 1 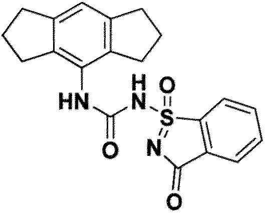 Example 2 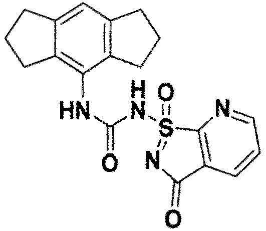
Example 4 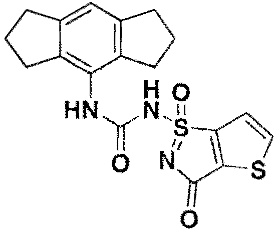 Example 5 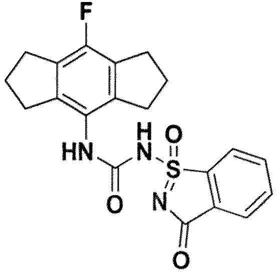
Example 6 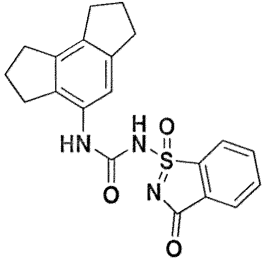 Example 7 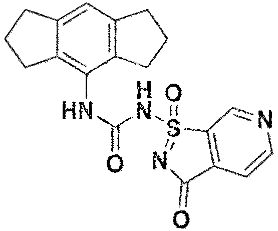
Example 8 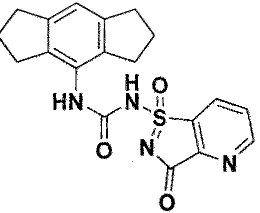 Example 9 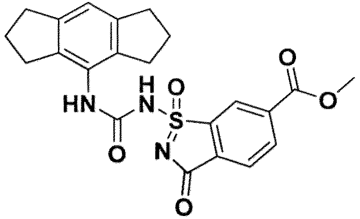
Example 10 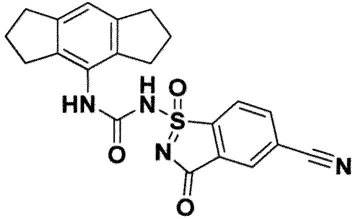 Example 11 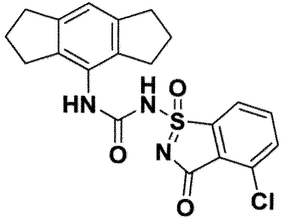
Example 12 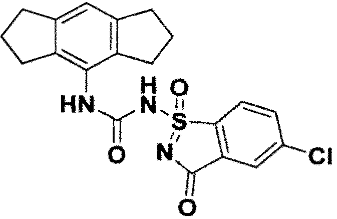 Example 13 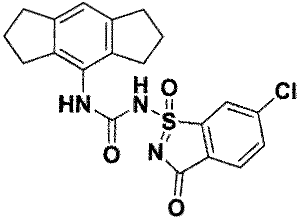
Example 14 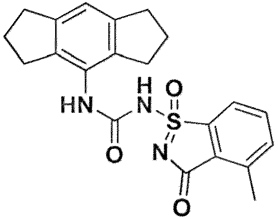 Example 15 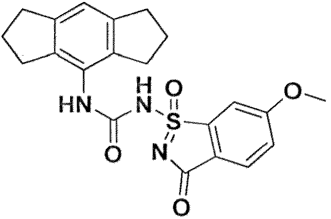
Example 18 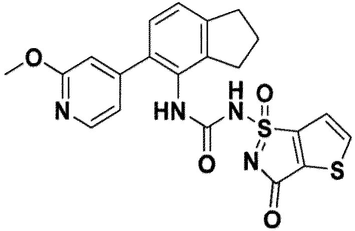 Example 19 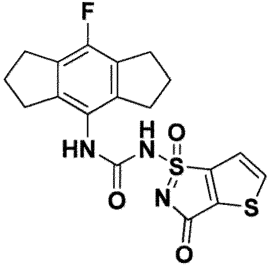 Example 22 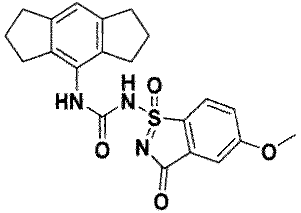
Example 23 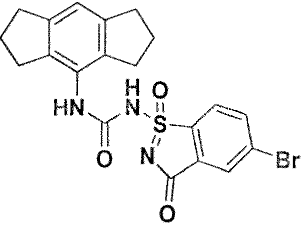 Example 24 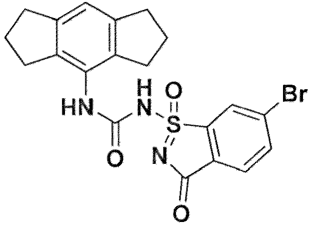
Example 25 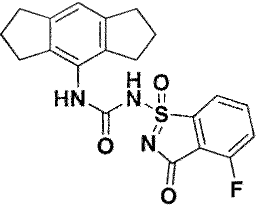 Example 26 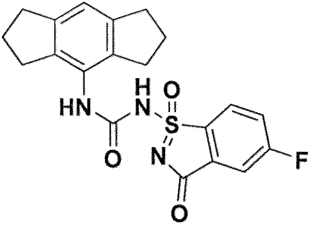
Example 27 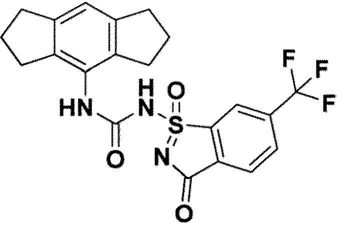 Example 28 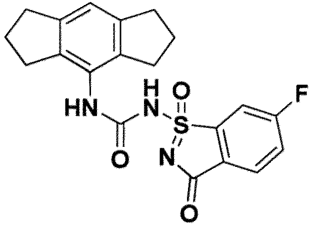
Example 29 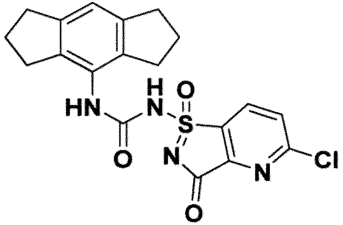 Example 30

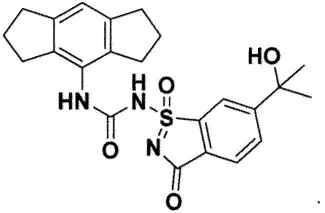

The following enantiomers were identified. The elution peak* is indicated:
Example 32 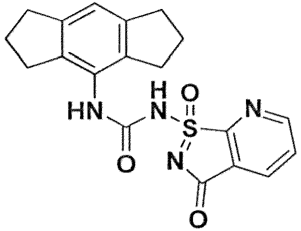 First*
Example 33 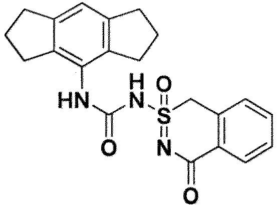 First*
Example 34 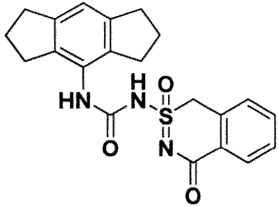 Second *
Example 35 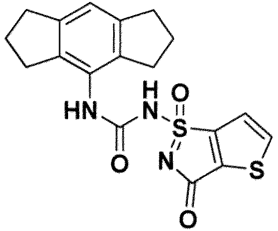 First*
Example 36 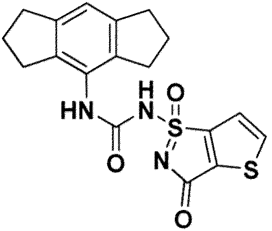 Second *
Example 37 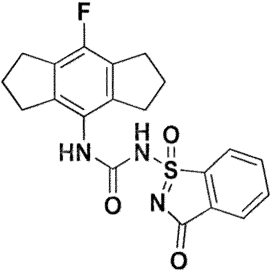 Second *
Example 38 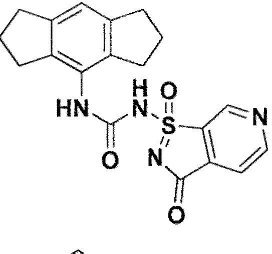 Second *
Example 39 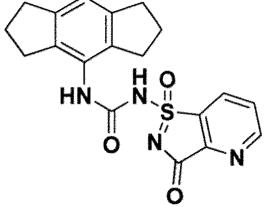 First*
Example 40 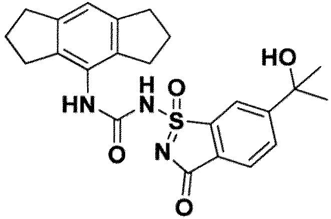 First*
Example 41 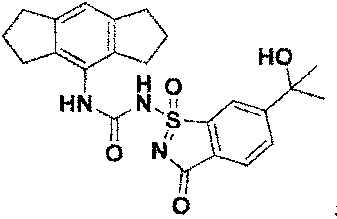 Second *
Example 42 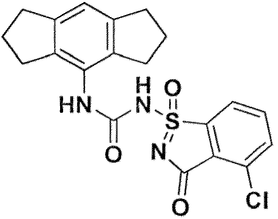 First*
Example 43 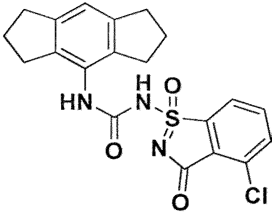 Second *
Example 44 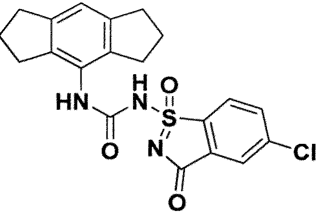 First*
Example 45 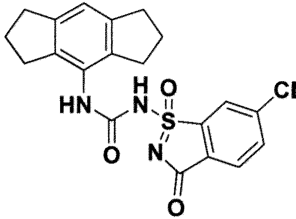 Second *
Example 46 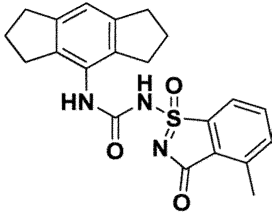 First*
Example 47 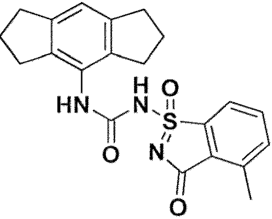 Second *
Example 48 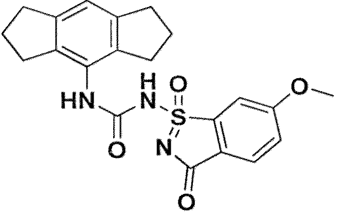 Second *
Example 52 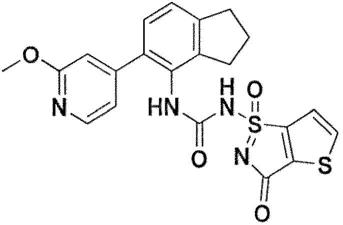 First*
Example 53 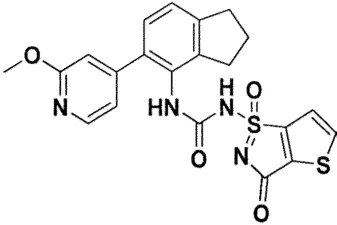 Second*
Example 54 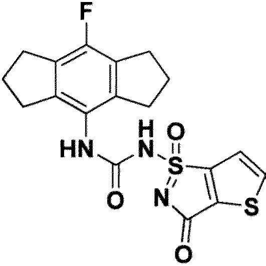 First *
Example 55 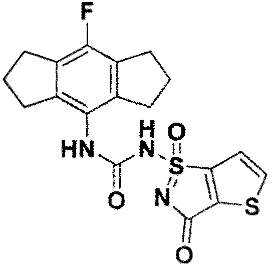 Second *
Example 57 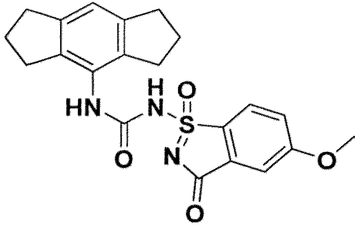 First *
Example 58 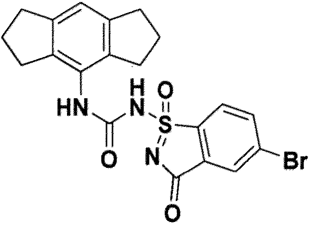 First*
Example 59 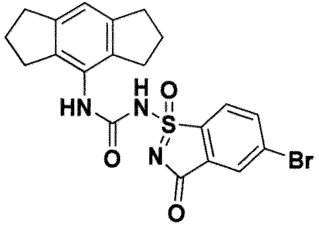 Second*
Example 60 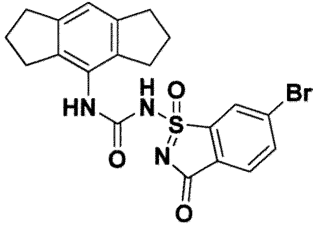 Second*
Example 61 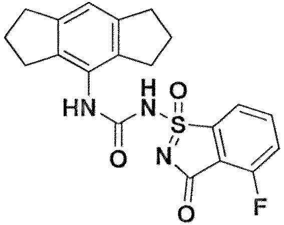 First *
Example 62 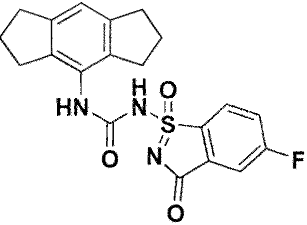 First *
Example 63 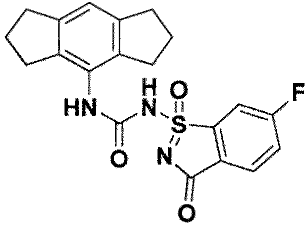 Second *
Example 64 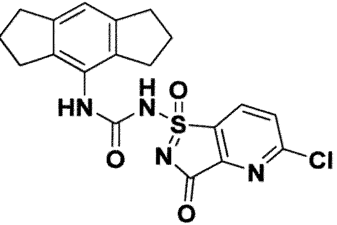 First *

A further embodiment of the invention is directed to compounds of the following formula (le) 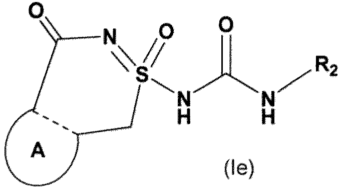
or stereoisomers, racemic mixtures, tautomers, pharmaceutically acceptable salts, hydrates, solvates and polymorphs thereof,
wherein
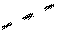 is a single or double bond,
**A** is selected from the group consisting of 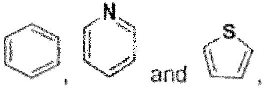
wherein each of these rings can be optionally substituted,
**R₂** is selected from the following ring systems 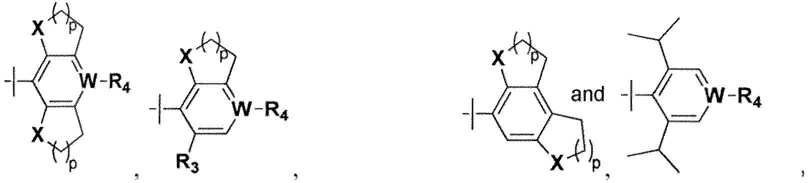
wherein
**X** are independently selected from the group consisting of CH₂ and O,
each **p** is 1,
**W** is selected from the group consisting of C and N, in case of **W** being N no **R₄** is present,
**R₄** is selected from the group consisting of hydrogen, halogen, alkyl, alkyl-O-alkyl,
aryl and heteroaryl, wherein alkyl, alkyl-O-alkyl, aryl and heteroaryl can be optionally substituted, and
**R₃** is an optionally substituted heteroaryl.

Preferred definitions which were given with respect to the compounds of formula (Id) apply here.

A preferred compound of formula (le) is
Example 3

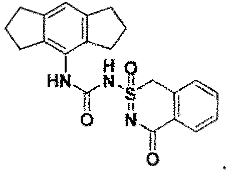

Any combination of the embodiments, preferred embodiments and more preferred embodiments disclosed herein is also envisaged in the present invention.

The present invention relates further to a pharmaceutical composition comprising a compound of formula (I), (Ia), (Ib), (Ic), (Id) or (Ie) and optionally at least one pharmaceutically acceptable excipient, carrier, diluent and/or adjuvant.

In one embodiment, the pharmaceutical composition comprises a compound of formula (Id) and optionally at least one pharmaceutically acceptable excipient, carrier, diluent and/or adjuvant.

In a further embodiment, the pharmaceutical composition comprises a compound of formula (le) and optionally at least one pharmaceutically acceptable excipient, carrier, diluent and/or adjuvant.

Embodiments as defined above for the compounds of formula (I), (Ia), (Ib), (Ic), (Id) or (le) apply here as well and can be combined with each other.

The present invention relates to a compound of formula (I), in particular (Ic) (Id) or (le) as defined herein, for use as a medicament.

The present invention relates to compounds of formula (I), in particular (Ic) (Id) or (le) as defined herein, for use in the treatment, alleviation or prevention of a disease, disorder or abnormality responsive to the modulation or inhibition of a component of the inflammasome pathway and/or for use in the modulation, in particular decrease, of IL-1 beta and/or IL-18 levels.

Preferably, the disease, disorder or the abnormality is responsive to the modulation of a component of the inflammasome pathway, in particular to the inhibition of the activation of a component of the inflammasome pathway. Preferably, the component of the inflammasome pathway is NLRP3 inflammasome.

More preferably, the disease, disorder or the abnormality is selected from Alzheimer's disease, Parkinson's disease, amyotrophic lateral sclerosis, demyelination, viral encephalitis, epilepsy, stroke, atherosclerosis, asthma, allergic inflammation, cryopyrin-associated periodic syndromes (CAPS), Muckle-Wells syndrome (MWS), familial cold autoinflammatory syndrome (FCAS), neonatal-onset multisystem inflammatory disease (NOMID), gout, pseudo-gout, inflammatory bowel disease, nonalcoholic fatty liver disease, nonalcoholic steatohepatitis, hypertension, myocardial infarction, oxalate-induced nephropathy, graft-versus host disease, type 1 diabetes, type 2 diabetes, rheumatoid arthritis, myelodysplastic syndrome, familial Mediterranean fever (FMF), TNF receptor associated periodic syndrome (TRAPS), mevalonate kinase deficiency (MKD), hyperimmunoglobulinemia D, periodic fever syndrome (HIDS), deficiency of interleukin 1 receptor (DIRA) antagonist, Majeed syndrome, pyogenic arthritis, pyoderma gangrenosum, and acne (PAPA), haploinsufficiency of A20 (HA20), PLCG2-associated antibody deficiency and immune dysregulation (PLAID), pediatric granulomatous arthritis (PGA), PLCG2-associated autoinflammation, antibody deficiency and immune dysregulation (APLAID), sideroblastic anemia with B-cell immunodeficiency, periodic fevers, developmental delay (SIFD), chronic nonbacterial osteomyelitis (CNO), Sweet's syndrome, chronic recurrent multifocal osteomyelitis (CRMO), synovitis, pustulosis, acne, hyperostosis, osteitis syndrome (SAPHO), multiple sclerosis (MS), psoriasis, Behcet's disease, Sjogren's syndrome, Schnitzler syndrome, chronic obstructive pulmonary disorder (COPD), steroid-resistant asthma, asbestosis, silicosis, cystic fibrosis, motor neuron disease, Huntington's disease, cerebral malaria, brain injury from pneumococcal meningitis, obesity, age-related macular degeneration (AMD), corneal infection, uveitis, dry eye, chronic kidney disease, diabetic nephropathy, alcoholic liver disease, skin contact hypersensitivity, sunburn, osteoarthritis, systemic juvenile idiopathic arthritis, adult-onset Still's disease, relapsing polychondritis, Chikungunya virus, Ross River virus, influenza, HIV, Coronaviruses, Dengue virus, Zika virus, hidradenitis suppurativa (HS), lung cancer metastasis, pancreatic cancers, gastric cancers, myelodisplastic syndrome, leukemia; polymyositis, colitis, helminth infection, bacterial infection, abdominal aortic aneurism, wound healing, depression, psychological stress, pericarditis including Dressler's syndrome, ischaemia reperfusion injury, frontotemporal dementia, HIV-associated neurocognitive disorder, Coronavirus-associated inflammatory pathologies, and traumatic brain injury.

Preferably the disease, disorder or the abnormality is selected from Alzheimer's disease, Parkinson's disease, amyotrophic lateral sclerosis, demyelination, viral encephalitis, epilepsy, stroke, atherosclerosis, asthma, allergic inflammation, cryopyrin-associated periodic syndromes (CAPS), gout, inflammatory bowel disease, nonalcoholic fatty liver disease, nonalcoholic steatohepatitis (NASH), hypertension, myocardial infarction, oxalate-induced nephropathy, graft-versus host disease, type 1 diabetes, type 2 diabetes, rheumatoid arthritis, myelodysplastic syndrome, anti-neutrophil cytoplasmic antibody-associated vasculitis (AAV), lupus nephritis, anti-glomerular basement membrane (GMB) disease, IgA nephropathy, glomerulonephritis (GN), systemic lupus erythematosus (SLE), Focal Segmental Glomerulosclerosis , Minimal change disease (MCD), Psoriatic Arthritis, and Hereditary Recurrent Fevers (HRFs).

Preferably, the disease, disorder or the abnormality is selected from Alzheimer's disease, Parkinson's disease, amyotrophic lateral sclerosis, demyelination, viral encephalitis, epilepsy, stroke, atherosclerosis, asthma and allergic inflammation, cryopyrin-associated periodic syndromes (CAPS), gout, inflammatory bowel disease, nonalcoholic fatty liver disease, nonalcoholic steatohepatitis (NASH), hypertension, myocardial infarction, oxalate-induced nephropathy, graft-versus host disease, type 1 and type 2 diabetes, rheumatoid arthritis, and myelodysplastic syndrome.

More preferably, the disease, disorder or the abnormality is selected from Alzheimer's disease, Parkinson's disease, cryopyrin-associated periodic syndromes (CAPS), nonalcoholic fatty liver disease, NASH, rheumatoid arthritis and gout.

Even more preferably, the disease, disorder or the abnormality is selected from Alzheimer's disease, Parkinson's disease, cryopyrin-associated periodic syndromes (CAPS), rheumatoid arthritis and gout.

In one embodiment, the present invention relates to compounds of formula (I), in particular (Ic), (Id) or (le) as defined herein, for use in the treatment, alleviation or prevention of a tauopathy by modulating a component of the inflammasome pathway, in particular, by modulating NLRP3 inflammasome.

In one embodiment, the present invention relates to compounds of formula (I), in particular (Ic), (Id) or (le) as defined herein, for use in the treatment, alleviation or prevention of a IL-18 and/or IL-1beta related disease or abnormality by modulating a component of the inflammasome pathway, in particular, by modulating NLRP3 inflammasome. The IL-18 and/or IL-1beta levels in a subject are typically decreased in result of the administration of compounds of formula (I), in particular (Ic), (Id) or (le) as defined herein.

IL-18 and/or IL-1 beta related diseases and abnormalities can be selected from chronic obstructive pulmonary disease (COPD), transfusion-related lung injury, bronchopulmonary dysplasia (BPD), acute respiratory distress syndrome (ARDS), pediatric autoinflammatory disease or condition, Still's disease, particularly Adult Still's disease or juvenile Still's disease, juvenile rheumatoid arthritis (JRA), juvenile idiopathic arthritis (JIA), systemic juvenile onset idiopathic arthritis (SoJIA), systemic juvenile idiopathic arthritis (sJIA), interstitial lung disease (ILD), macrophage activation syndrome (MAS) including primary, secondary and recurrent MAS, hemophagocytic lymphohistiocytosis (HLH), Familial (hereditary) hemophagocytic lymphohistiocytosis (FHLH) associated with gene defects in perforin, munc 13-4 and 18-2, synthaxin 11, immune deficiencies such as Chediak-Higashi syndrome (CHS), Griscelli syndrome (GS), X-linked lymphoproliferative syndrome (XLP2), X-linked inhibitor of apoptosis protein deficiency (XIAP), acquired hemophagocytic lymphohistiocytosis associated with infectious conditions especially Herpes virus such as EBV and other pathogens, autoinflammatory syndrome associated with NLRC4 mutations, Giant Cell Arteritis (GCA), Pyogenic arthritis, pyoderma gangrenosum, and acne (PAPA), pulmonary sarooidis, heart failure, ischemic heart disease, dry eye disease (DED), keratitis, corneal ulcer and abrasion, iritis, glaucoma, Sjogren's syndrome, autoimmune uveitis, Behcet's disease, conjunctivitis, allergic conjunctivitis, diabetes type 2, solid organ and hematologic stem cell transplantation, ischemia reperfusion injury, familial Mediterranean fever (FMF), tumor necrosis factor receptor 1- associated periodic syndromes (TRAPS), hyper-lgD syndromes (mevalonate kinase gene mutation), gout, Schnitzler syndrome, Wegener's granulomatosis also called granulomatosis with polyangitis (GPA), Hashimoto's thyroiditis, Crohn's disease, early onset inflammatory bowel disease (EOIBD), very EOIBD (VEOIBD), infantile IBD, neonatal IBD, ulcerative colitis and Blau syndrome (NOD-2 mutation).

The modulation of NLRP3 inflammasome appears to be beneficial in diseases, disorders and abnormalities with altered IL-18 levels, which lead to pathological inflammation.

Embodiments as defined above for the compounds of formula (I) such as (Ia), (Ib), (Ic), (Id) or (le) apply here as well and can be combined with each other.

The present invention relates to the compound of formula (I), in particular (Ic), (Id) or (le) as defined herein, that is a modulator of NLRP3 inflammasome activity and/or a modulator of IL-18 and/or IL-1b levels in subject.

The present invention relates to a pharmaceutical composition comprising a combination of a compound of formula (I), in particular (Ic), or (le) as defined herein, and at least one further biologically active compound differing from the compound of formula (I), in particular (Ic), or (le), and optionally at least one selected from pharmaceutically acceptable excipients, carriers, diluents or adjuvants.

In particular, the further biologically active compound can be one used for the treatment of a disease, disorder or abnormality which targets a different pathomechanism, e.g. an anti-amyloid beta antibody, anti-Tau antibody, amyloid beta small molecule inhibitor, Tau aggregation small molecule inhibitor, anti-alpha synuclein antibody or alpha-synuclein aggregation small molecule inhibitor, anti-TDP-43 antibody or anti-TDP-43 aggregation small molecule inhibitor among others. When a compound of the invention is used in combination with a further biologically active compound, the dose of each compound may differ from the dose if the compound were to be used as a monotherapy.

The present invention relates to the use of a compound of formula (I), in particular (Ic), or (le), as an analytical reference or an *in vitro* screening tool. The compounds of the present invention can be used as an analytical reference or an *in vitro* screening tool for characterization of cells with activated NLRP3 inflammasome and for testing of compounds targeting the NLRP3 inflammasome.

The present invention relates to a method for obtaining a compound of formula (I), in particular (Ic), or (le). In a further embodiment, the method is preferably suited for obtaining a compound of formula (le).

In one embodiment, the method comprises the step of isocyanate derivative coupling reaction of a compound of formula (IId) for producing a compound of formula (Ic) in the presence of a solvent and a base 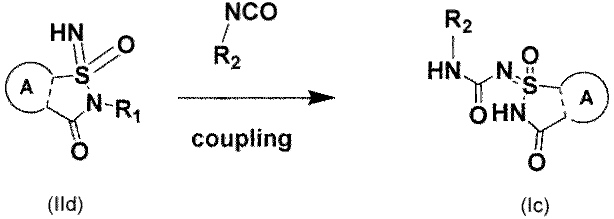 wherein **A, R₂** and **R₁** are as defined above.

The coupling reaction is conducted using a suitable solvent under strong basic conditions. The base is preferably NaH, triethylamine or pyridine. The solvent is preferably THF, DCM or CHCl₃.

The coupling is preferably conducted at room temperature (rt).

A method comprises the step of isocyanate derivative coupling reaction of a compound of formula (IId) for producing a compound of formula (Id) in the presence of a solvent and a base

In one embodiment, the method comprises the step of isocyanate derivative coupling reaction of a compound of formula (IIe) for producing a compound of formula (le) in the presence of a solvent and a base 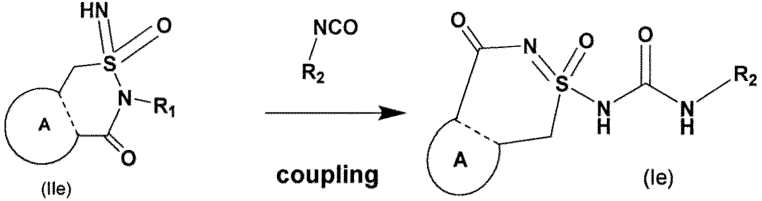 wherein **A, R₂** and **R₁** are as defined above.

The coupling reaction is conducted using a suitable solvent under strong basic conditions. The base is preferably NaH, triethylamine or pyridine. The solvent is preferably THF, DCM or CHCl₃. The coupling is preferably conducted at room temperature (rt).

The present invention further relates to compounds of formula (IId) and (IIe) as defined below 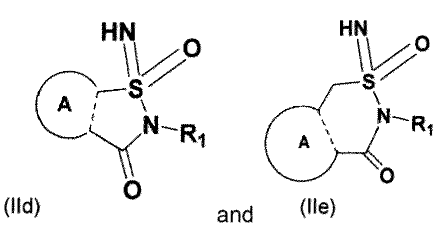 wherein **A,** and **R₁** are as defined above, when used in a method of producing a compound of formula (I) as outlined in the appended claims.

### Pharmaceutical compositions

While it is possible for the compounds of the present invention to be administered alone, it is preferable to formulate them into a pharmaceutical composition in accordance with standard pharmaceutical practice. Thus, the invention also provides a pharmaceutical composition which comprises a therapeutically effective amount of a compound of formula (I) optionally in admixture with a pharmaceutically acceptable carrier, diluent, adjuvant or excipient.

Pharmaceutically acceptable carriers, diluents, adjuvants and excipients are well known in the pharmaceutical art and are described, for example, in Remington's Pharmaceutical Sciences, 15th or 18th Ed. (Alfonso R. Gennaro, ed.; Mack Publishing Company, Easton, PA, 1990); Remington: the Science and Practice of Pharmacy 19th Ed.(Lippincott, Williams & Wilkins, 1995); Handbook of Pharmaceutical Excipients, 3rd Ed. (Arthur H. Kibbe, ed.; Amer. Pharmaceutical Assoc, 1999); Pharmaceutical Codex: Principles and Practice of Pharmaceutics 12th Ed. (Walter Lund ed.; Pharmaceutical Press, London, 1994); The United States Pharmacopeia: The National Formulary (United States Pharmacopeial Convention); Fiedler's "Lexikon der Hilfstoffe" 5th Ed., Edition Cantor Verlag Aulendorf 2002; "The Handbook of Pharmaceutical Excipients", 4th Ed., American Pharmaceuticals Association, 2003; and Goodman and Gilman's: the Pharmacological Basis of Therapeutics (Louis S. Goodman and Lee E. Limbird, eds.; McGraw Hill, 1992), the disclosures of which are hereby incorporated by reference.

The carriers, diluents, adjuvants and pharmaceutical excipients can be selected with regard to the intended route of administration and standard pharmaceutical practice. These compounds must be acceptable in the sense of being not deleterious to the recipient thereof.

Pharmaceutically useful excipients that may be used in the formulation of the pharmaceutical composition of the present invention may comprise, for example, vehicles, solvents (such as monohydric alcohols such as ethanol, isopropanol and polyhydric alcohols such as glycols), edible oils (such as soybean oil, coconut oil, olive oil, safflower oil, and cottonseed oil), oily esters (such as ethyl oleate and isopropyl myristate), binders (such as hydroxypropylmethyl cellulose (HPMC), hydroxypropyl cellulose (HPC), pregelatinzed starch and combinations thereof), solubilizers, thickening agents, stabilizers, disintegrants (such as carboxymethylcellulose calcium (CMC-Ca), carboxymethylcellulose sodium (CMC-Na), crosslinked PVP (e.g., crospovidone, Polyplasdone^{®} or Kollidon^{®} XL), alginic acid, sodium alginate, guar gum, cross-linked CMC (croscarmellose sodium, e.g. Ac-Di-Sol^{®}), carboxymethyl starch-Na (sodium starch glycolate) (e.g., Primojel^{®} or Explotab^{®}), preferably crosslinked PVP and/or croscarmellose sodium), glidants (such as colloidal SiO₂ (e.g., Aerosil^{®} 200), magnesium trisilicate, powdered cellulose, talc and combinations thereof), lubricating agents (such as magnesium stearate, aluminium or calcium silicate, stearic acid, hydrogenated castor oil, talc, glyceryl behenate, sodium stearate fumarate and combinations thereof), buffering agents, emulsifiers, wetting agents, suspending agents, sweetening agents, colorants, flavors, coating agents, preservatives, antioxidants, processing agents, drug delivery modifiers and enhancers (such as calcium phosphate), magnesium stearate, talc, monosaccharides, disaccharides, starch, gelatin, cellulose, methylcellulose, sodium carboxymethyl cellulose, dextrose, hydroxypropyl-β-cyclodextrin, polyvinylpyrrolidone, low melting waxes, and ion exchange resins.

The carrier is not particularly limited and will depend on the route of administration as well as the form of the pharmaceutical composition (i.e., solid, liquid, etc.). Suitable carriers include, without limitation, polyols such as mannitol, sorbitol, xylitol; disaccharides such as lactose, sucrose, dextrose and maltose; polysaccharides such as maltodextrine and dextranes; starches such as corn starch; celluloses such as microcrystalline cellulose, sodium carboxy methylcellulose, low-substituted hydroxypropyl cellulose, hydroxyl ethyl cellulose, hydroxypropyl cellulose or mixtures thereof; cylodextrines and inorganic agents such as dicalcium phosphate, calcium hydrogen phosphate; hydroxyapatite, tricalcium phosphate, talcum and silica. Microcrystalline cellulose, sucrose and/or lactose are preferred as carriers. Combinations thereof can also be employed. Carriers can include also protein and cell penetrating peptides which should be selected depending on the route of administration and target.

The diluent is not particularly limited and will depend on the route of administration as well as the form of the pharmaceutical composition (i.e., solid, liquid, etc.). Diluents include, for instance, water, ethanol, propylene glycol and glycerin, and combinations thereof.

An adjuvant is an additive which has few or no pharmacological effects by themselves, but that increases the efficacy or potency of the compounds of the invention if they are administered together.

The routes for administration (delivery) of the compounds of the invention include, but are not limited to, one or more of: oral (e. g. as a tablet, capsule, or as an ingestible solution), topical, mucosal (e. g. as a nasal spray or aerosol for inhalation), nasal, parenteral (e. g. by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, epidural and sublingual.

For example, the compounds can be administered orally in the form of tablets, capsules, ovules, elixirs, solutions or suspensions, which may contain flavoring or coloring agents, for immediate-, delayed-, modified-, sustained-, pulsed- or controlled-release applications.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, milk sugar e.g. lactose or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

If the compounds of the present invention are administered parenterally, then examples of such administration include one or more of: intravenously, intraarterially, intraperitoneally, intrathecally, intraventricularly, intraurethrally, intrasternally, intracranially, intramuscularly or subcutaneously administering the compounds; and/or by using infusion techniques. For parenteral administration, the compounds can be used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

As indicated, the compounds of the present invention can be administered intranasally or by inhalation and are conveniently delivered in the form of a dry powder inhaler or an aerosol spray presentation from a pressurized container, pump, spray or nebulizer with the use of a suitable propellant, e. g. dichlorodifluoromethane, trichlorofluoromethane, dichlorotetrafluoroethane, a hydrofluoroalkane such as 1,1,1,2-tetrafluoroethane (HFA134AT) or 1,1,1,2,3,3,3-heptafluoropropane (HFA 227EA), carbon dioxide or other suitable gas. In the case of a pressurized aerosol, the dosage unit may be determined by providing a valve to deliver a metered amount. The pressurized container, pump, spray or nebulizer may contain a solution or suspension of the active compound, e. g. using a mixture of ethanol and the propellant as the solvent, which may additionally contain a lubricant, e. g. sorbitan trioleate. Capsules and cartridges (made, for example, from gelatin) for use in an inhaler or insufflator may be formulated to contain a powder mix of the compound and a suitable powder base such as lactose or starch.

Alternatively, the compounds of the present invention can be administered in the form of a suppository or pessary, or it may be applied topically in the form of a gel, hydrogel, lotion, solution, cream, ointment or dusting powder. The compounds of the present invention may also be dermally or transdermally administered, for example, by the use of a skin patch.

They may also be administered by the pulmonary or rectal routes. They may also be administered by the ocular route. For ophthalmic use, the compounds can be formulated as micronized suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the compounds of the present invention can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, polyethylene glycol, liquid paraffin, polysorbate 60, cetyl ester wax, cetearyl alcohol, 2-octyldodecanol, benzyl alcohol and water.

Typically, a physician will determine the actual dosage which will be most suitable for an individual subject. The specific dose level and frequency of dosage for any particular individual may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

A proposed dose of the compounds according to the present invention for administration to a human (of approximately 70 kg body weight) is 0.1 mg to 1 g, preferably 1 mg to 500 mg of the active ingredient per unit dose. The unit dose may be administered, for example, 1 to 4 times per day. The dose will depend on the route of administration. It will be appreciated that it may be necessary to make routine variations to the dosage depending on the age and weight of the patient as well as the severity of the condition to be treated. The precise dose and route of administration will ultimately be at the discretion of the attendant physician or veterinarian.

The claimed compound can be used for the treatment, alleviation or prevention of the recited diseases, disorders or abnormality alone or in combination with one or more further biologically active compounds. In particular the further biologically active compound can be one used for the treatment of diseases, disorders or abnormalities which target a different pathomechanism, e.g. an anti-amyloid beta antibody and/or anti-Tau antibody or Tau aggregation small molecule inhibitor in Alzheimer's disease, anti-alpha synuclein antibody or alpha-synuclein aggregation small molecule inhibitor, anti-TDP-43 antibody or anti-TDP-43 aggregation small molecule inhibitor among others. When a compound of the invention is used in combination with a further biologically active compound against the same diseases, disorders or abnormality, the dose of each compound may differ from that when the compound were to be used alone.

The combinations referred to above may conveniently be presented for use in the form of a pharmaceutical formulation. The individual components of such combinations may be administered either sequentially or simultaneously in separate or combined pharmaceutical formulations by any convenient route. When administration is sequential, either the compound of the invention or the further biologically active compound may be administered first. When administration is simultaneous, the combination may be administered either in the same or different pharmaceutical composition. When combined in the same formulation it will be appreciated that the two compounds must be stable and compatible with each other and the other components of the formulation. When formulated separately they may be provided in any convenient formulation, conveniently in such manner as are known for such compounds in the art.

The pharmaceutical compositions of the invention can be produced in a manner known per se to the skilled person as described, for example, in Remington's Pharmaceutical Sciences, 15th Ed., Mack Publishing Co., New Jersey (1975).

Examples of the diseases, disorders or abnormalities which can be treated, alleviated or prevented include, but are not limited, to Alzheimer's disease, Parkinson's disease, amyotrophic lateral sclerosis, demyelination, viral encephalitis, epilepsy, stroke, atherosclerosis, asthma and allergic inflammation, cryopyrin-associated periodic syndromes (CAPS) (Muckle-Wells syndrome (MWS), familial cold autoinflammatory syndrome (FCAS) and neonatal-onset multisystem inflammatory disease (NOMID)), gout, pseudo-gout, inflammatory bowel disease, nonalcoholic fatty liver disease, nonalcoholic steatohepatitis, hypertension, myocardial infarction, oxalate-induced nephropathy, graft-versus host disease, type 1 and type 2 diabetes, rheumatoid arthritis, myelodysplastic syndrome, familial Mediterranean fever (FMF), TNF receptor associated periodic syndrome (TRAPS), mevalonate kinase deficiency (MKD), hyperimmunoglobulinemia D and periodic fever syndrome (HIDS), deficiency of interleukin 1 receptor (DIRA) antagonist, Majeed syndrome, pyogenic arthritis, pyoderma gangrenosum and acne (PAPA), haploinsufficiency of A20 (HA20), PLCG2-associated antibody deficiency and immune dysregulation (PLAID), pediatric granulomatous arthritis (PGA), PLCG2-associated autoinflammation, antibody deficiency and immune dysregulation (APLAID), sideroblastic anemia with B-cell immunodeficiency, periodic fevers, developmental delay (SIFD), chronic nonbacterial osteomyelitis (CNO), Sweet's syndrome, chronic recurrent multifocal osteomyelitis (CRMO) and synovitis, pustulosis, acne, hyperostosis, osteitis syndrome (SAPHO), multiple sclerosis (MS), psoriasis, Behcet's disease, Sjogren's syndrome and Schnitzler syndrome, chronic obstructive pulmonary disorder (COPD), steroid-resistant asthma, asbestosis, silicosis, cystic fibrosis, motor neuron disease, Huntington's disease, cerebral malaria, brain injury from pneumococcal meningitis, obesity, age-related macular degeneration (AMD), corneal infection, uveitis and dry eye, chronic kidney disease, diabetic nephropathy, alcoholic liver disease, skin contact hypersensitivity, sunburn, osteoarthritis, systemic juvenile idiopathic arthritis, adult-onset Still's disease, relapsing polychondritis, Chikungunya and Ross River viruses, flu, HIV, Dengue virus, Zika viruses, hidradenitis suppurativa (HS), lung cancer metastasis, pancreatic cancers, gastric cancers, myelodisplastic syndrome, leukemia; polymyositis, colitis, helminth infection, bacterial infection, abdominal aortic aneurism, wound healing, depression, psychological stress, pericarditis including Dressler's syndrome, ischaemia reperfusion injury, frontotemporal dementia, HIV-associated neurocognitive disorder, and traumatic brain injury; preferably the disease, disorder or abnormality is selected from Alzheimer's disease, Parkinson's disease, amyotrophic lateral sclerosis, demyelination, viral encephalitis, epilepsy, stroke, atherosclerosis, asthma and allergic inflammation, CAPS, gout, inflammatory bowel disease, nonalcoholic fatty liver disease, nonalcoholic steatohepatitis, hypertension, myocardial infarction, oxalate-induced nephropathy, graft-versus host disease, type 1 and type 2 diabetes, rheumatoid arthritis, and myelodysplastic syndrome.

The compounds of the present invention can be used as an analytical reference or an *in vitro* screening tool for characterization of cells with activated NLRP3 inflammasome and for testing of compounds targeting the NLRP3 inflammasome.

The compounds according to the present invention can also be provided in the form of a mixture with at least one further biologically active compound and/or a pharmaceutically acceptable carrier and/or a diluent and/or an excipient and/or adjuvant. The compound and/or the further biologically active compound are preferably present in a therapeutically effective amount.

The nature of the further biologically active compound will depend on the intended use of the mixture. The further biologically active substance or compound may exert its biological effect by the same or a similar mechanism as the compound according to the invention or by an unrelated mechanism of action or by a multiplicity of related and/or unrelated mechanisms of action.

The invention also includes all suitable isotopic variations of the compounds of the invention. An isotopic variation of the compound of the invention is defined as one in which at least one atom is replaced by an atom having the same atomic number but an atomic mass different from the atomic mass usually found in nature. Examples of isotopes that can be incorporated into compounds of the invention include isotopes of hydrogen, carbon, nitrogen, oxygen, sulfur, fluorine and chlorine such as ²H, ³H, ¹³C, ¹⁴C, ¹⁵N, ¹⁷O, ¹⁸O, ³⁵S, ¹⁸F and ³⁶Cl, respectively. Certain isotopic variations of the invention, for example, those in which a radioactive isotope such as ³H or ¹⁴C is incorporated, are useful in drug and/or substrate tissue distribution studies. Tritiated, i.e., ³H, and carbon-14, i.e., ¹⁴C, isotopes are particularly preferred for their ease of preparation and delectability. ¹⁸F-labeled compounds are particularly suitable for imaging applications such as PET. Further, substitution with isotopes such as deuterium, i.e., ²H, may afford certain therapeutic advantages resulting from greater metabolic stability, for example, increased *in vivo* half-life or reduced dosage requirements and hence may be preferred in some circumstances. Isotopic variations of the compounds of the invention can generally be prepared by conventional procedures such as by the illustrative methods or by the preparations described in the Examples and Preparations hereafter using appropriate isotopic variations of suitable reagents.

The compounds of the present invention can be synthesized by those skilled in the art by using commonly known preparation steps, for instance those of the general methods shown in the following schemes. These methods are only given for illustrative purposes and should not to be construed as limiting.

### General synthetic schemes for the preparation of illustrative compounds of this invention:

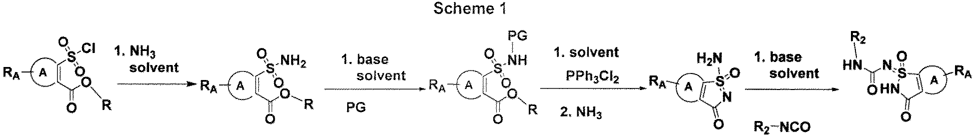

R_{A} is the optional substituent of A
R is a leaving group such as alkyl (e.g., Me, Et)
PG is a protective group

From commercially available chlorosulfonyl derivatives, sulfonamide can be synthetized using ammonia and an appropriate solvent. Appropriate (e.g., TBDMS, TBDPS) protection of the sulfonamide moiety can be achieved in a suitable solvent under basic conditions. A generic chlorinating agent can be used (e.g., Ph₃PCl₂, PCl₅, SOCl₂) followed by ammonia treatment. The derivative can undergo a deprotection step without isolation followed by intramolecular ring-closure (one-pot procedure) to afford a benzo[d]isothiazol-3-one 1-oxide derivative. Treatment with an appropriate isocyanate using a suitable solvent under strongly basic conditions can afford the desired urea derivative as a racemic mixture after purification. The enantiomers can be separated by chiral supercritical fluid chromatography (SFC) to obtain the desired single enantiomers, if desired. 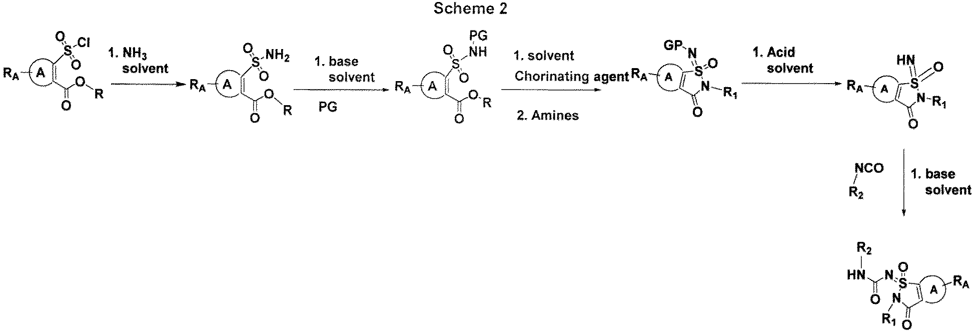
R_{A} is the optional substituent of A
R is a leaving group such as alkyl (e.g., Me, Et)
PG is a protective group

From commercially available chlorosulfonyl derivatives, sulfonamide can be synthetized using ammonia and an appropriate solvent. Appropriate (e.g., TBDMS, TBDPS) protection of the sulfonamide moiety can be achieved in a suitable solvent under basic conditions. A generic chlorinating agent can be used (e.g., Ph₃PCl₂, PCl₅, SOCl₂) followed by amine treatment. The derivatives can underdo a deprotection step without isolation followed by intramolecular ring-closure (one-pot procedure) to afford protected a benzo[d]isothiazol-3-one 1-oxide derivative. Deprotection in acid conditions using an appropriate solvent followed by treatment with an appropriate isocyanate using a suitable solvent under strong basic conditions can afford the desired urea derivative as a racemic mixture after purification. The enantiomers can be separated by chiral supercritical fluid chromatography (SFC) to obtain the desired single enantiomers, if desired.

### Preparative Examples

### Preparative Example 1

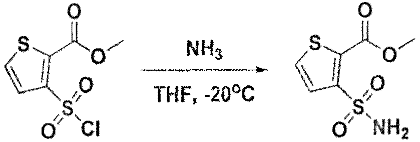

A solution of methyl 3-(chlorosulfonyl)thiophene-2-carboxylate (5.0 g, 20.77 mmol) in THF (500 mL) was cooled to -20° C and ammonia gas was purged slowly through the solution for 30 min. Then the reaction mixture was allowed to stir at room temperature for 30 min. After the completion of the reaction, solid was removed. The reaction mixture was filtered through a Buchner funnel and the filtrate was concentrated under reduced pressure to obtain a crude product. Then the crude product was triturated with diethyl ether (2 x 20 ml), the resulting solid was filtered through a Buchner funnel and dried to afford methyl 3-sulfamoylthiophene-2-carboxylate (1.8 g, 39 %) as a colorless crystalline solid.
MS: 220.1 [(M-H)]⁻

### Preparative Examples 2 to 14

Following the procedure of **Preparative Example 1** using appropriate reagents indicated in the table below, the following compounds were prepared.

**Table 1:**

| Starting Material | Product | 1. Yield |
|---|---|---|
| | **Preparative Example** | 2. ¹H-NMR |
| | | 3. MH⁺ (ESI) |
| 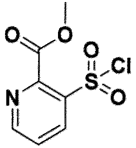 | 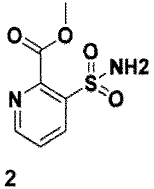 | 1.98 % |
| | | 2. ¹H NMR (400 MHz, DMSO-d6) δ = 8.79 (dd, 1H), 8.35 (dd, 1H), 7.81-7.78 (m, 1H), 3.86 (s, 3H). |
| | | 3. 217.1 |
| 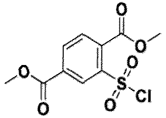 | 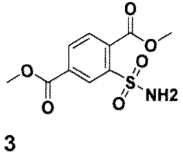 | 1.85% |
| | | 2. ¹H NMR (400 MHz, CDCI3) δ = 8.76 (d, 1H), 8.29 (dd, 1H), 7.90 (d, 1H), 5.69 (s, 2H), 4.02 (s, 3H), 3.98 (s, 3H). |
| | | 3. 272.2 |
| 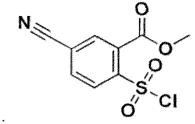 | 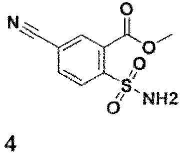 | 1.71 % |
| | | 2. ¹H NMR (400 MHz, DMSO-d6) δ = 8.24-8.21 (m, 2H), 8.11 (d, 1H), 7.68 (brs, 2H), 3.85(s.3H). |
| | | 3. 239.2 |
| 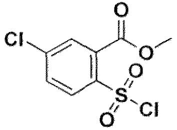 | 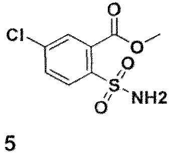 | 1.94 % |
| | | 2. ¹H NMR (400 MHz, DMSO-d6) δ = 7.96 (d, 1H), 7.84-7.81 (m, 1H), 7.77 (m, 1H), 7.38 (brs, 2H), 3.83 (s, 3H). |
| | | 3. 248.2 |
| 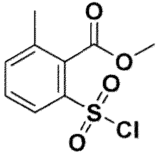 | 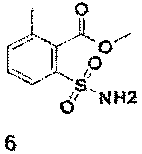 | 1. 91 % |
| | | 2. ¹H NMR (500 MHz, DMSO-d6) δ = 7.78 (d, 1H), 7.57-7.52 (m, 2H), 7.32 (brs, 2H), 3.81 (s, 3H), 2.27 (s, 3H). |
| | | 3. ND |
| 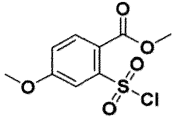 | 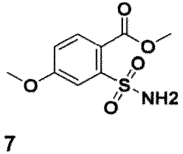 | 1.92 % |
| | | 2. ¹H NMR (400 MHz, CDCl3) δ = 7.91 (d, 1H), 7.71 (d, 1H), 7.06 (dd, 1H), 5.86 (s, 2H), 3.95 (s, 3H), 3.91 (s, 3H). |
| | | 3. 244.3 |
| 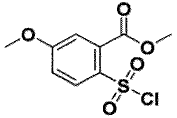 | 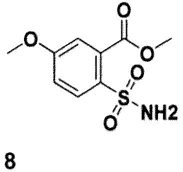 | 1.94% |
| | | 2. ¹H NMR (400 MHz, DMSO-d6) δ = 7.88 (d, 1H), 7.25-7.22 (m, 1H), 7.16-7.14 (m, 3H), 3.85 (s.3H), 3.82 (s, 3H). |
| | | 3. 246.2 |
| 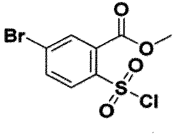 | 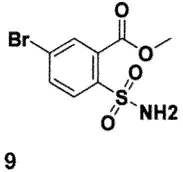 | 1.97% |
| | | 2. ¹H NMR (400 MHz, DMSO-d6) δ = 7.97 (dd, 1H), 7.89 (s, 1H), 7.88 (d, 1H), 7.30 (brs, 2H), 3.83 (s, 3H). |
| | | 3. 292.2 |
| 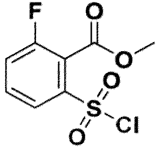 | 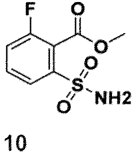 | 1. 91 % |
| | | 2. ¹H NMR (400 MHz, DMSO-d6) δ = 7.82 (m, 1H), 7.80-7.73 (m, 1H), 7.64-7.59 (m, 3H), 3.84 (s, 3H). |
| | | 3. 232.1 |
| 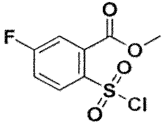 | 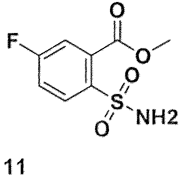 | 1.92% |
| | | 2. ND |
| | | 3. 235.3 |
| 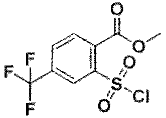 | 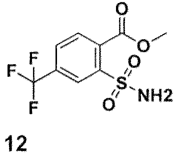 | 1.96 % |
| | | 2. ¹H NMR (400 MHz, DMSO-d6) δ = 8.29 (s, 1H), 8.11(d, 1H), 7.89 (dd, 1H), 7.34 (brs, 2H), 3.86 (s, 3H). |
| | | 3. 267.2 |
| 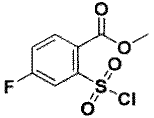 | 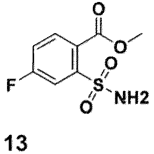 | 1.91 % |
| | | 2. ¹H NMR (500 MHz, DMSO-d6) δ = 7.79-7.75 (m, 2H), 7.60-7.56 (m, 1H), 7.32 (brs, 2H) 3.83 (s, 3H). |
| | | 3. 232.1 |
| 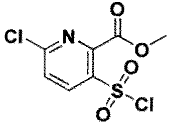 | 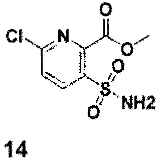 | 1.95% |
| | | 2. ¹H NMR (500 MHz, CDCl3) δ = 8.38 (dd, 1H), 7.96 (d, 1H), 7.83 (s, 2H), 3.87 (s, 3H) |
| | | 3. 251.2 |

### Preparative Example 15

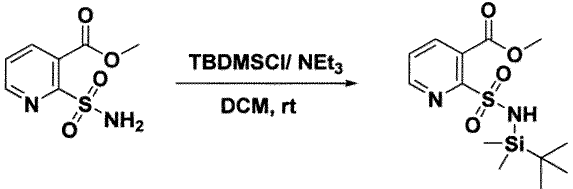

A solution of methyl 2-sulfamoylnicotinate (0.5 g, 2.31 mmol) in DCM (20 mL) was cooled to 0 °C and triethylamine (0.97 mL, 6.9444 mmol) was added, followed by TBDMSCI (0.418 g, 2.77 mmol). The reaction mixture was stirred at RT for 16h. After completion of the reaction, the reaction mixture was diluted with water and extracted with DCM (2 x 50 mL). The organic phase was separated, washed with water (50 ml), followed by saturated brine solution (50 ml), dried over Na₂SO₄ and subsequently filtered. The filtrate was concentrated under reduced pressure to obtain a crude product. The crude product was purified by flash chromatography (neutral alumina; 0 to 50% ethyl acetate in petroleum ether). The pure fractions were collected and concentrated under vacuum to afford the title methyl 2-(N-(tert-butyldimethylsilyl)sulfamoyl)-nicotinate (0.48 g, 63%) as an off-white solid.

MS: 331.27 (M+H) ⁺.

### Preparative Examples 16 to 34

Following the procedure of **Preparative Example 15** using appropriate reagents indicated in the table below, the following compounds were prepared.

**Table 2:**

| Starting Material | Product | 1. Yield |
|---|---|---|
| | **Preparative Example** | 2. ¹H-NMR |
| | | 3. MH⁺ (ESI) |
| 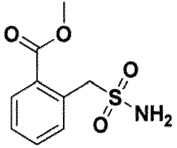 | 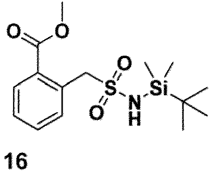 | 1.70% |
| | | 2. ¹H NMR (400 MHz, CDCl3) δ = 12.0 (bs, 2H), 8.11 (dd, 1H), 7.63 (m, 2H), 7.54 (m, 1H), 4.99 (s, 2H), 4.01(s, 3H), 0.92 (s, 9H), 0.2 (s, 6H). |
| | | 3. 344.2 |
| 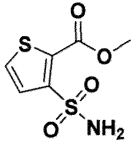 | 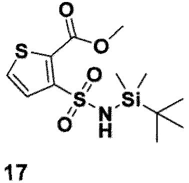 | 1.91 % |
| | | 2. ¹H NMR (400 MHz, CDCl3) δ = 7.56-7.53 (d, 1H), 7.50-7.49 (d, 1H), 6.31 (brs, 1H), 3.96 (s, 3H), 0.88 (s, 9H), 0.17 (s, 6H). |
| | | 3. 334.3 |
| 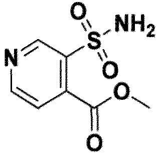 | 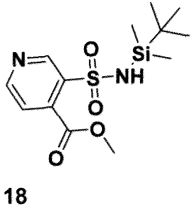 | 1.87% |
| | | 2. ND |
| | | 3. 331.2 |
| 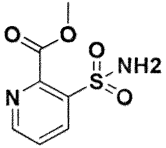 | 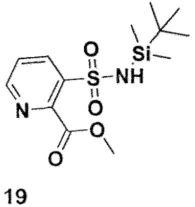 | 1.90% |
| | | 2. ND |
| | | 3. 331.2 |
| 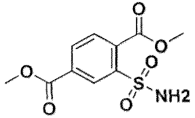 | 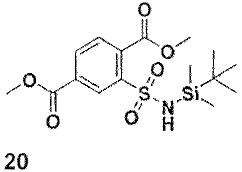 | 1.79% |
| | | 2. ¹H NMR (500 MHz, CDCI3) δ = 8.69 (d, 1H), 8.23 (dd, 1H), 7.87 (d, 1H), 6.04 (s, 1H), 4.00 (s, 3H), 3.97 (s, 3H), 0.9(m, 9H), 0.24(s, 6H). |
| | | 3. 386.4 |
| 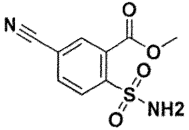 | 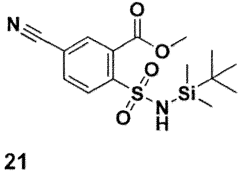 | 1. used crude without further purification |
| | | 2. ¹H NMR (400 MHz, CDCl3) δ = 8.21 (d, 1H), 8.12 (d, 1H), 7.91 (dd, 1H), 6.14 (brs, 1H), 0.,88 (s, 9H), 1.23 (s, 6H). |
| | | 3. ND |
| 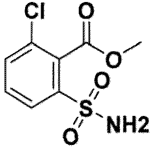 | 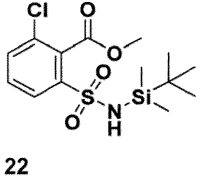 | 1.96 % |
| | | 2. ¹H NMR (400 MHz, CDCl3) δ = 7.86 (dd, 1H), 7.58 (dd, 1H), 7.47 (dd, 1H), 5.06 (s, 1H), 4.02 (s, 3H), 0.89 (s, 9H), 0.21 (s, 6H). |
| | | 3. 362.4 |
| 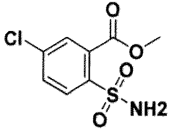 | 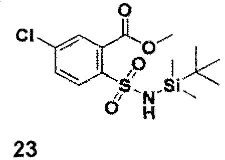 | 1. used crude without further purification |
| | | 2. ND |
| | | 3. 362.4 |
| 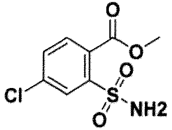 | 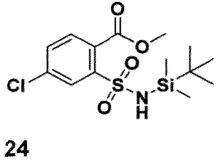 | 1.69% |
| | | 2. ND |
| | | 3. 363.9 |
| 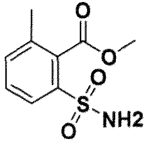 | 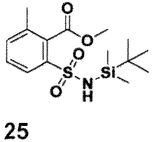 | 1.79% |
| | | 2. ¹H NMR (400 MHz, DMSO-d6) δ = 7.77 (d, 1H), 7.58-7.53 (m, 2H), 7.51-7.49 (brs, 1H), 3.81 (s, 3H), 2.26 (s, 3H), 0.86 (s, 9H), 0.06 (s, 6H). |
| | | 3. 342.4 |
| 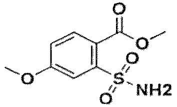 | 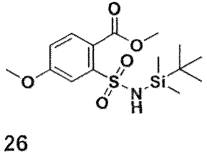 | 1. used crude |
| | | 2. ND |
| | | 3. 360.4 |
| 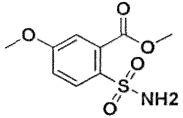 | 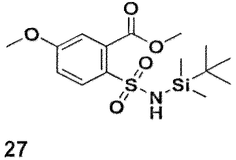 | 1. used crude |
| | | 2. ¹H NMR (500 MHz, CDCl3) δ = 8.00 (d, 1H), 7.32-7.27 (m, 1H), 7.05-7.03 (m, 1H), 6.04 (s, 1H), 3.98 (s, 3H), 3.90 (s, 3H), 0.91 (s, 9H), 0.21 (s, 6H). |
| | | 3. 360.2 |
| 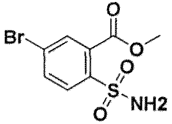 | 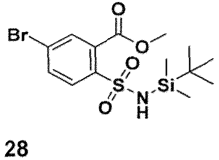 | 1. used crude |
| | | 2. ¹H NMR (400 MHz, CDCl3) δ = 7.96-7.94 (m, 2H), 7.75 (dd, 1H), 6.10 (s, 1H), 3.99 (s, 3H), 0.9 (s, 9H), 0.22 (s, 6H). |
| | | 3. 406.1 |
| 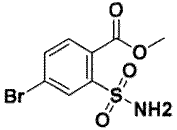 | 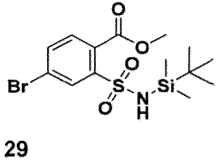 | 1.72% |
| | | 2. ND |
| | | 3. 406.3 |
| 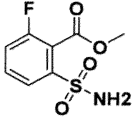 | 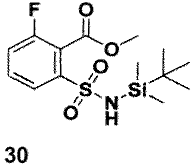 | 1. used crude |
| | | 2. ND |
| | | 3. 346.4 |
| 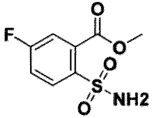 | 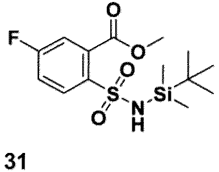 | 1. used crude |
| | | 2. ND |
| | | 3. 346.3 |
| 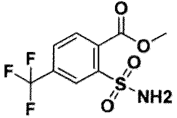 | 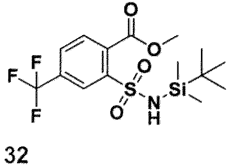 | 1.85% |
| | | 2. ¹H NMR (400 MHz, CDCl3) δ = 8.35 (s, 1H), 7.92 (d, 1H), 7.84 (dd, 1H), 3.98 (s, 3H), 0.87 (s, 9H), 0.05 (s, 6H). |
| | | 3. ND |
| 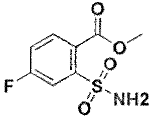 | 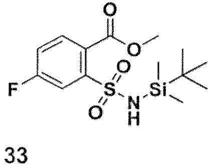 | 1.85 % |
| | | 2. ND |
| | | 3. 346.2 |
| 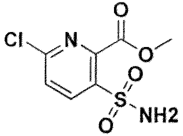 | 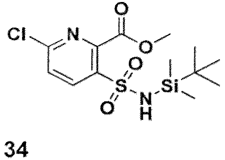 | 1.86% |
| | | 2. ND |
| | | 3. [-TBDMS] 249.0 |

### Preparative Example 35

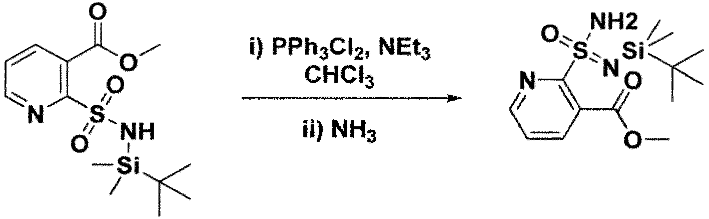

To a suspension of Ph₃PCl₂ (0.581 g, 1.74 mmol) in CHCl₃ (15 mL) was added triethylamine (0.61 mL, 4.36 mmol) and the mixture was stirred for 10 min at RT. Then the reaction mixture was cooled to 0°C, a solution of **Preparative Example 19** (0.480 g, 1.4545 mmol) in CHCl₃ was slowly added and the mixture was stirred for 20 min. Then the reaction mixture was cooled to - 20°C and ammonia gas was purged for 5 min. The mixture was warmed for 30 min at 0°C. After 30 min, a suspension was formed and the solid was removed by filtration. The filtrate was evaporated under reduced pressure to afford crude methyl 2-(N'-(tert-butyldimethylsilyl)-sulfamidimidoyl)nicotinate as an off-white solid (0.470 g crude). Although the compound was contaminated with PPh₃O it was directly taken to the next step without further purification.

### Preparative Examples 36 to 54

Following the procedure of **Preparative Example 35** using appropriate reagents indicated in the table below, the following compounds were prepared.

**Table 3**

| Starting Material | Product | 1. Yield |
|---|---|---|
| | **Preparative Example** | 2. MH⁺ (ESI) |
| 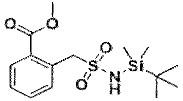 | 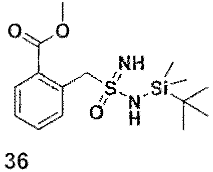 | 1. crude used to the next step |
| | | 2.343.2 |
| 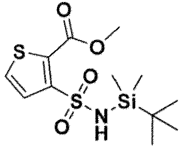 | 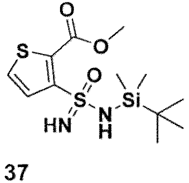 | 1.20% |
| | | 2.335.3 |
| 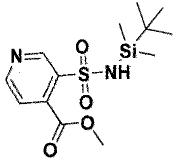 | 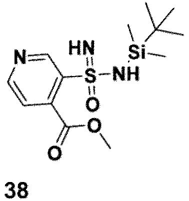 | 1. crude used to the next step |
| | | 2. ND |
| 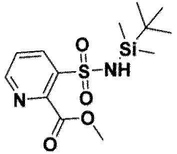 | 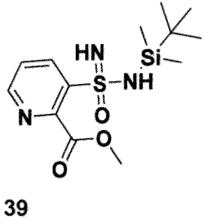 | 1. crude used to the next step |
| | | 2. 330.2 |
| 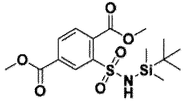 | 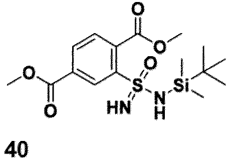 | 1. crude used to the next step |
| | | 2. 387.4 |
| 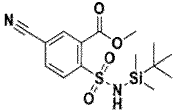 | 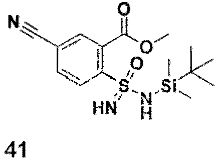 | 1. crude used to the next step |
| | | 2. ND |
| 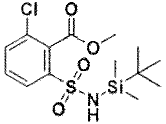 | 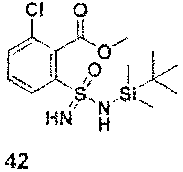 | 1. crude used to the next step |
| | | 2. 363.3 |
| 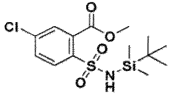 | 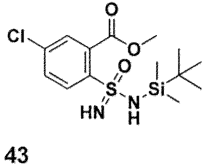 | 1. crude used to the next step |
| | | 2. ND |
| 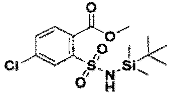 | 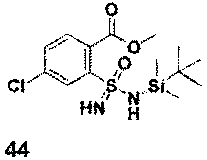 | 1. crude used to the next step |
| | | 2. ND |
| 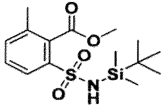 | 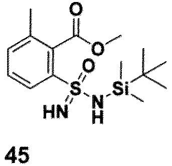 | 1. crude used to the next step |
| | | 2. ND |
| 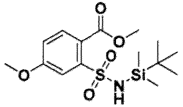 | 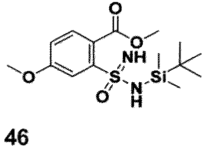 | 1. crude used to the next step |
| | | 2. 359.2 |
| 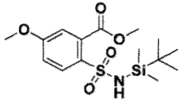 | 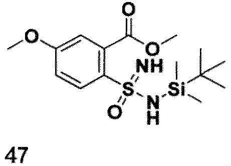 | 1. crude used to the next step |
| | | 2. ND |
| 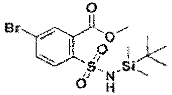 | 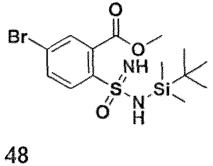 | 1. crude used to the next step |
| | | 2. 407.4 |
| 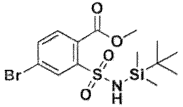 | 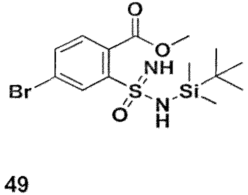 | 1. crude used to the next step |
| | | 2. ND |
| 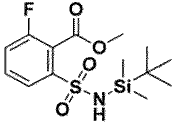 | 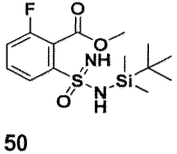 | 1. crude used to the next step |
| | | 2. ND |
| 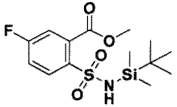 | 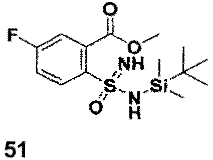 | 1. crude used to the next step |
| | | 2. 347.3 |
| 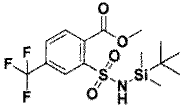 | 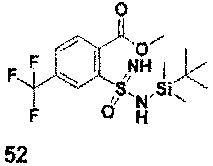 | 1. crude used to the next step |
| | | 2. ND |
| 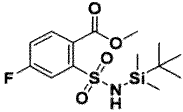 | 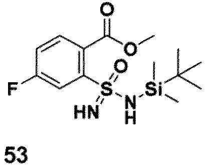 | 1. crude used to the next step |
| | | 2. ND |
| 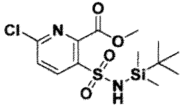 | 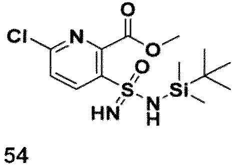 | 1. crude used to the next step |
| | | 2. 364.2 |

### Preparative Example 55

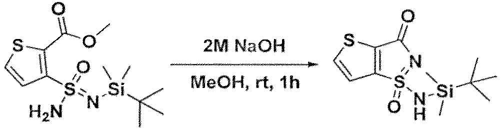

To a stirred solution of **Preparative Example 37** (1.4 g, 4.18 mmol ) in MeOH (15 mL) was added 2N NaOH solution (10.45 ml, 20.92 mmol) dropwise at 0°C and the mixture was stirred at RT for 1h. After completion of the reaction, the reaction mixture was diluted with brine solution (25 ml) and extracted with ethyl acetate (2 x 100 ml). The organic layer was separated and dried over Na₂SO₄, filtered and evaporated to afford 1-((tert-butyldimethylsilyl)amino)-3H-1,4-thieno[2,3-d]isothiazol-3-one 1-oxide (900mg, crude) as an off-white foam solid. The crude compound was used to the next step without further purfication
MS: 189.16 [(M-TBDMS)]⁺

### Preparative Example 56

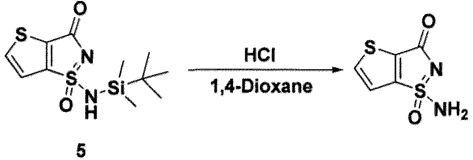

To a stirred solution of **Preparative Example 55** (0.9 g, 2.97 mmol) in 1,4-dioxane (15 mL) was added 4M HCl in 1,4-dioxane (2.97 ml, 11.90 mmol) dropwise at 0°C and the mixture was stirred for 25 min. After completion of the reaction, the reaction mixture was evaporated under reduced pressure to obtain a crude compound. The crude compound was purified by Grace automated purification (silica 24 g column, eluted with gradient mixture of MeOH in DCM (1 to 10%)). Collected pure fractions were concentrated under reduced pressure to afford 1-((tertbutyldimethylsilyl)amino)-3H-1,4-thieno[2,3-d]isothiazol-3-one 1-oxide (380 mg, 68% over two steps) as an off-white crystalline solid.
¹H NMR (400 MHz, DMSO-d6) δ = 8.24 (s, 2H), 8.14 (d, 1H), 7.41 (d, 1H).
MS: 189.1 [(M+H)]⁺

### Preparative Example 57

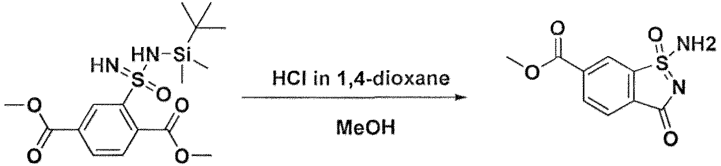

To a stirred solution of **Preparative Example 40** (350 mg, 0.9 mmol) in 1,4-dioxane (5 mL) was added dropwise a solution of 1M HCl in dioxane (0.5 ml) at 0°C and the mixture was allowed to stir at RT for 1 h. After completion of the reaction, the reaction mixture was evaporated under reduced pressure and the residue was purified by Grace automated purification (silica 12 g column, eluted with gradient mixture of MeOH in DCM (1 to 10%)). Collected pure fractions were concentrated under reduced pressure to afford methyl 1-amino-3-oxo-3H-1,4-benzo[d]isothiazole-6-carboxylate 1-oxide (60 mg, 40 % overall two steps) as a yellow solid.
1H-NMR, (400 MHz, DMSO-d6) δ = 8.43 (d, 1H), 8.36-8.38 (dd, 1H), 8.25 (s, 2H), 7.96 (d, 1H), 3.94 (s, 3H).
MS: 239.06 [(M-H)]+

### Preparative Examples 58 and 59

Following the procedure of **Preparative Example 57** using appropriate reagents indicated in the table below, the following compounds were prepared.

**Table 4:**

| Starting Material | Product | 1. Yield |
|---|---|---|
| | **Preparative Example** | 2. ¹H-NMR |
| | | 3. MH⁺ (ESI) |
| 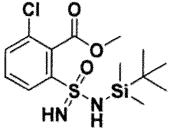 | 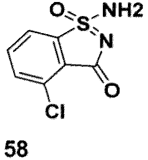 | 1.70 % |
| | | 2. ¹H NMR (400 MHz, DMSO-d6) δ = 8.20 (s, 2H), 7.90-7.93 (dd, 1H), 7.82-7.87 (m, 2H). |
| | | 3. 217.2 |
| 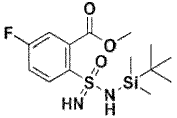 | 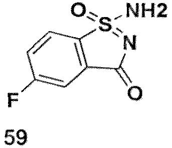 | 1.86 % |
| | | 2. ¹H NMR (400 MHz, DMSO-d6) δ = 8.01-7.98 (m, 1H), 7.83 (brs, 2H), 7.71-7.62 (m, 2H). |
| | | 3. 201.1 |

### Preparative Example 60

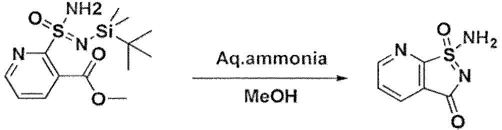

To a stirred solution of **Preparative Example 35** (0.47 g crude) in methanol (5 mL) was added NH₄OH solution (8 mL) slowly at rt and the mixture was heated to reflux for 3h. After the completion of the the reaction, solvent was evaporated under reduced pressure to obtain a crude product. The crude product was purified by Grace automated purifier (SiO₂, 12 g column; 0 to 5% methanol in DCM). Pure fractions were collected and concentrated under reduced pressure to afford 1-amino-3H-1,4-isothiazolo[5,4-b]pyridin-3-one 1-oxide (0.15 g, 57%).

MS: 184.18 (M+H) ⁺.

### Preparative Examples 61 to 75

Following the procedure of **Preparative Example 60** using appropriate reagents indicated in the table below, the following compounds were prepared.

**Table 5:**

| Starting Material | Product | 1. Yield |
|---|---|---|
| | **Preparative Example** | 2. ¹H-NMR |
| | | 3. MH⁺ (ESI) |
| 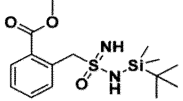 | 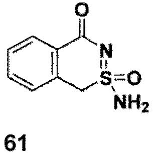 | 1.35 % |
| | | 2. ¹H NMR (500 MHz, DMSO-d6) δ = 7.96 (dd, 1H), 7.59-4.45 (m, 6H), 4.79 (d, H), 4.65 (d, 1H). |
| | | 3. 197.1 |
| 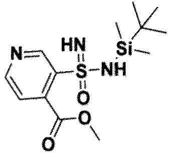 | 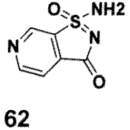 | 1. 5 % |
| | | 2. ¹H NMR (500 MHz, DMSO-d6) δ = 9.05 (s, 1H), 8.88 (d, 1H), 7.69 (d, 1H). |
| | | 3. 184.2 |
| 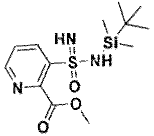 | 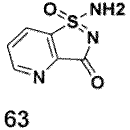 | 1. 60 % |
| | | 2. ¹H NMR (400 MHz, DMSO-d6) δ = 8.71 (dd, 1H), 8.18 (dd, 1H), 7.53 (dd, 1H), 6.39 (brs, 2H). |
| | | 3. 184.4 |
| 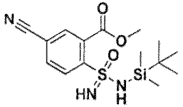 | 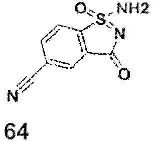 | 1. 44 % |
| | | 2. ND |
| | | 3. 208.8 |
| 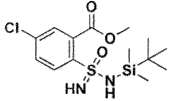 | 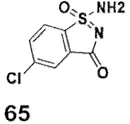 | 1. used crude |
| | | 2. ND |
| | | 3. 217.2 |
| 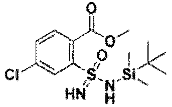 | 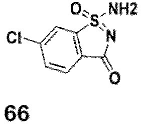 | 1. 50 % |
| | | 2. ND |
| | | 3. 217.2 |
| 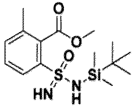 | 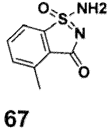 | 1. used crude |
| | | 2. ND |
| | | 3. +H20 197.3 |
| 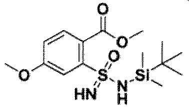 | 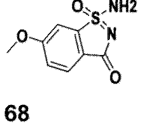 | 1. 23 % |
| | | 2. ND |
| | | 3. 213.1 |
| 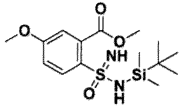 | 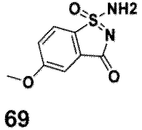 | 1. used crude |
| | | 2. ND |
| | | 3. 213.3 |
| 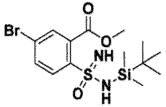 | 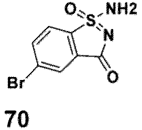 | 1. used crude |
| | | 2. ND |
| | | 3. 261.0 |
| 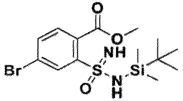 | 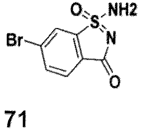 | 1. used crude |
| | | 2. ND |
| | | 3. 261.1 |
| 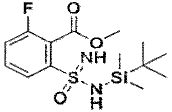 | 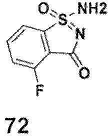 | 1. used crude |
| | | 2. ND |
| | | 3. ND |
| 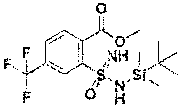 | 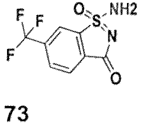 | 1. used crude |
| | | 2. ND |
| | | 3. ND |
| 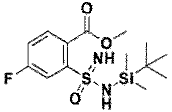 | 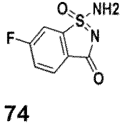 | 1. used crude |
| | | 2. ND |
| | | 3. 201.1 |
| 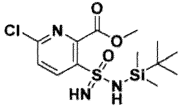 | 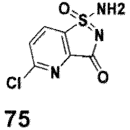 | 1. 68 % |
| | | 2. ND |
| | | 3. 218.1 |

### Examples

All reagents and solvents were obtained from commercial sources and used without further purification. ¹H-NMR spectra were recorded on Bruker 400 MHz-AVANCE III HD NMR and Bruker 500 MHz-AVANCE III HD NMR spectrometers in deuterated solvents. Chemical shifts (δ) are reported in parts per million and coupling constants (J values) in hertz. Spin multiplicities are indicated by the following symbols: s (singlet), d (doublet), t (triplet), q (quartet), m (multiplet), bs (broad singlet). Mass spectra were obtained on a Water ACQUITY SQD2 UPLC/MS system. GC-MS data were collected using an Agilent 7890B gas chromatograph and 5977A mass spectrometer. Chromatography was performed using silica gel (Acme: Silica gel 60, 0.063-0.2 mm) and suitable solvents as indicated in the specific examples. Flash purification was conducted with a Biotage Isolera one or Reveleris X₂ with KP-NH SNAP cartridges (Biotage) or Reveleris silica cartridges (Grace) and the solvent gradient indicated in the specific examples. Thin layer chromatography (TLC) was carried out on silica gel plates (Merck) with UV detection.

### Example 1

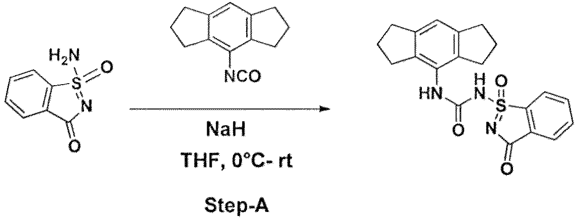

### Step A:

To a stirred solution of 1-amino-3H-1,4-benzo[d]isothiazol-3-one 1-oxide prepared following a literature procedure (ACS Med. Chem. Lett. 2017 May 1;8(6):672-677) in THF (8 mL) was added NaH (60%) (26.0 mg, 0.659 mmol) slowly at 0 °C and the mixture was stirred for 15 min. A solution of commercially available 4-isocyanato-1,2,3,5,6,7-hexahydro-s-indacene (0.109 g, 0.549 mmol) in THF (4 ml) was added to the reaction mixture dropwise at 0°C and the mixture was stirred at room temperature for 1h. After completion of the the reaction, the reaction was quenched with saturated NH₄Cl solution (15 ml) and the mixture was extracted with EtOAc (2 x 40ml). The organic phase was dried over Na₂SO₄, filtered and the filtrate was evaporated under reduced pressure to afford a crude product. The crude product was purified by flash chromatography (SiO₂ 230-400 mesh; 0-3% methanol in DCM). Pure compound fractions were collected and concentrated under reduced pressure to afford the racemic title compound **Example 1** (0.075 g, 93.68%) as an off-white solid.

1H-NMR, (400 MHz, DMSO-d6) δ = 8.07-8.05 (d, 1H), 7.68-7.6 (m, 3H), 6.85 (s, 1H), 2.79-2.75 (t, 4H), 2.67-2.64(t, 4H), 1.95-1.88(m, 4H).

MS: 381.9 (M+H)⁺.

### Examples 2 to 29

Following the procedure of **Example 1** using appropriate reagents indicated in the table below, the following compounds were prepared. THF solvent could be substituted by DCM or CHCl₃ and NaH by triethylamine or pyridine.

**Table 6:**

| Starting Material | Urea | Product | 1. Yield |
|---|---|---|---|
| | | **Example** | 2. ¹H-NMR |
| | | | 3. MH⁺ (ESI) |
| 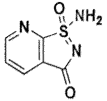 | 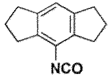 | 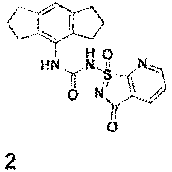 | 1. 19 % |
| | | | 2. ¹H NMR (400 MHz, DMSO-d6) δ = 8.66 (dd, 1H), 8.02-7.89 (m, 2H), 7.53 (dd, 1H), 6.77 (s, 1H), 2.71 (t, 4H), 2.67-2.54 (m, 4H), 1.91-1.87 (m, 4H). |
| | | | 3. 383.3 |
| 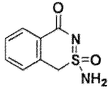 | 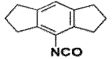 | 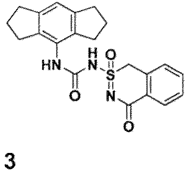 | 1. 32 % |
| | | | 2. ¹H NMR (400 MHz, DMSO-d6) δ = 7.87 (d, 1H), 7.72 (brs, 1H), 7.33-7.42 (m, 2H), 7.25 (d, 1H), 6.79 (s, 1H), 4.89 (d, 1H), 4.27 (d, 1H), 2.77-2.65 (m, 8H), 1.90 (m, 4H). |
| | | | 3. 396.3 |
| 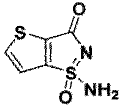 | 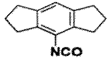 | 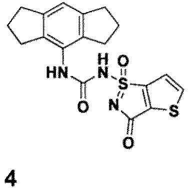 | 1. 68 % |
| | | | 2. ¹H NMR (500 MHz, DMSO-d6) δ = 8.08 (s, 1H), 7.77 (d, 1H), 7.25 (d, 1H), 6.82 (s, 1H), 2.76 (t, 4H), 2.68 (t, 4H), 1.94-1.88 (m, 4H). |
| | | | 3. 388.3 |
| 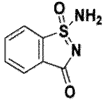 | 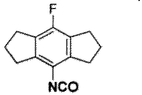 | 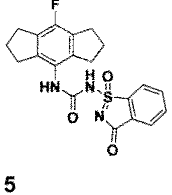 | 1. 26 % |
| | | | 2. ¹H NMR (500 MHz, DMSO-d6) δ =8.05-8.04 (m, 2H), 7.61-7.55 (m, 3H), 2.81-2.78 (t, 4H), 2.72-2.69 (t, 4H), 1.99-1.94 (m, 4H). |
| | | | 3. 400.3 |
| 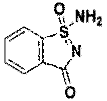 | 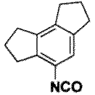 | 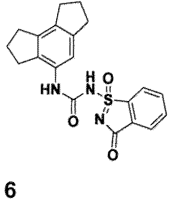 | 1. 36 % |
| | | | 2. ¹H NMR (500 MHz, DMSO-d6) δ = 8.07-8.05 (m, 1H), 7.73 (brs, 1H), 7.63-7.56 (m, 3H), 7.42 (s, 1H), 2.77-2.64 (m, 8H), 1.98-1.92 (m, 4H). |
| | | | 3. 382.3 |
| 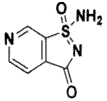 | 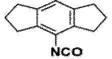 | 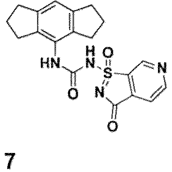 | 1. 23 % |
| | | | 2. ¹H NMR (500 MHz, DMSO-d6) δ = 9.24 (d, 1H), 8.82 (d, 1H), 8.19 (brs, 1H), 7.63 (dd, 1H), 6.83 (s, 1H), 2.76 (t, 4H), 2.65 (m, 4H), 1.92-1.89 (m, 4H). |
| | | | 3. 383.4 |
| 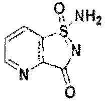 | 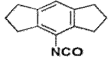 | 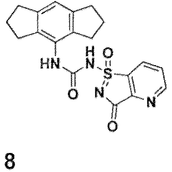 | 1. 33 % |
| | | | 2. ¹H NMR (500 MHz, DMSO-d6) δ = 8.79 (brs, 1H), 8.45 (d, 1H), 8.2 (brs, 1H), 7.58-7.56 (m, 1H), 6.82 (s, 1H), 2.76 (t, 4H), 2.67-2.64 (m, 4H), 1.93-1.88 (m, 4H). |
| | | | 3. 383.5 |
| 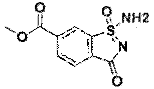 | 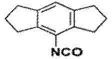 | 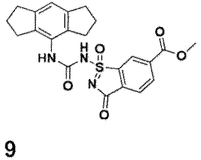 | 1. 33 % |
| | | | 2. ¹H NMR (500 MHz, DMSO-d6) δ = 8.64 (d, 1H), 8.37 (brs, 1H), 8.24-8.25 (dd, 1H), 6.85 (s, 1H), 3.94 (s, 3H), 2.78 (t, 4H), 2.65 (t, 4H), 1.91 (m, 4H). |
| | | | 3. 440.4 |
| 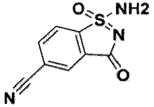 | 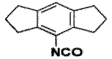 | 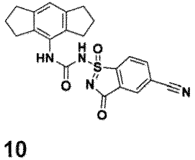 | 1. 26 % |
| | | | 2. ¹H NMR (500 MHz, DMSO-d6) δ = 8.34-8.22 (m, 2H), 8.11-8.07 (m, 2H), 6.83 (s, 1H), 2.68-2.64 (m, 4H), 1.93-1.88 (m, 4H), 2.77-2.74 (t, 4H). |
| | | | 3. 407,4 |
| 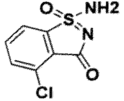 | 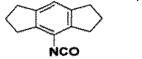 | 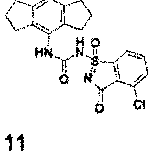 | 1. 92 % |
| | | | 2. ¹H NMR (500 MHz, DMSO-d6) δ = 8.06 (brs, 1H), 7.99-8.02 (m, 1H), 7.53-7.57 (m, 2H), 6.81 (s, 1H), 2.77 (t, 4H), 2.65 (t, 4H), 1.91 (m, 4H). |
| | | | 3. 416.3 |
| 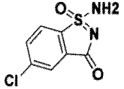 | 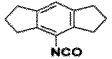 | 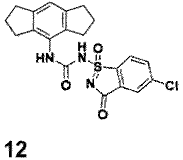 | 1. 24 % |
| | | | 2. ¹H NMR (500 MHz, DMSO-d6) δ = 8.10-8.05 (m, 2H), 7.65-7.63 (m, 1H), 7.57 (d, 1H), 6.82 (s, 1H), 2.77-2.74 (m, 4H), 2.67-2.64 (m, 4H), 1.92-1.89 (m, 4H). |
| | | | 3. 416.4 |
| 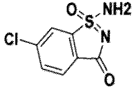 | 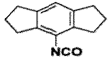 | 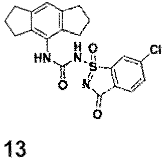 | 1. 24 % |
| | | | 2. ¹H NMR (500 MHz, DMSO-d6) δ = 8.15-8.10 (brs, 2H), 7.66-7.60 (m, 2H), 6.83 (s, 1H), 2.78-2.75 (m, 4H), 2.68-2.65 (m, 4H), 1.92-1.90 (m, 4H). |
| | | | 3. 416.4 |
| 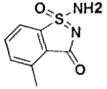 | 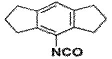 | 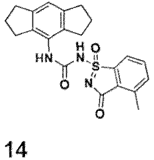 | 1. 39 % |
| | | | 2. ¹H NMR (500 MHz, DMSO-d6) δ = 7.93 (brs, 1H), 7.89 (d,1H), 7.41 (t, 1H), 7.33-7.31 (d, 1H), 6.80 (s,1H), 2.77-2.74 (m, 4H), 2.67-2.66 (m, 4H), 2.61(brs, 3H) 1.98-1.88(m, 4H). |
| | | | 3. 396.4 |
| 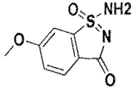 | 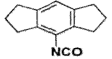 | 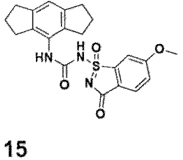 | 1. 40 % |
| | | | 2. ¹H NMR (500 MHz, DMSO-d6) δ = 8.02 (brs, 1H), 7.64 (d, 1H), 7.49 (d, 1H), 7.11 (dd, 1H), 6.82 (s, 1H), 3.82 (s, 3H), 2.64-2.78 (m, 8H), 1.90 (m, 4H). |
| | | | 3. 412.48 |
| 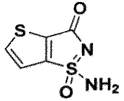 | 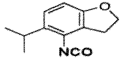 | 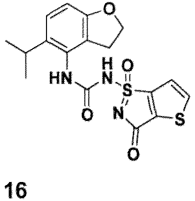 | 1. 51 % |
| | | | 2. ND |
| | | | 3. 392.2 |
| 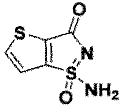 | 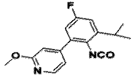 | 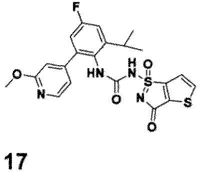 | 1. 41 % |
| | | | 2. ¹H NMR (500 MHz, DMSO-d6) δ = 8.37 (brs, 1H), 8.03 (brs, 1H), 7.21-7.17 (m, 2H), 7.00-6.98 (m, 2H), 6.75 (brs, 2H), 3.81 (brs, 3H), 3.14 (brs, 1H), 1.12-1.10 (m, 6H). |
| | | | 3.475.1 |
| 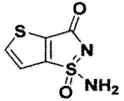 | 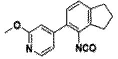 | 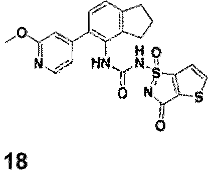 | 1. 60 % |
| | | | 2. ¹H NMR (500 MHz, DMSO-d6) δ = 7.97 (d, 2H), 7.75 (d, 1H), 7.14-7.10 (m, 2H), 7.06 (d, 1H), 6.90 (d, 1H), 6.71 (s, 1H), 3.78 (s, 3H), 2.92-2.89 (m,2H), 2.82-2.73 (m, 2H),1.99-1.96 (m, 2H) |
| | | | 3. 455.1 |
| 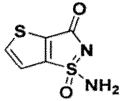 | 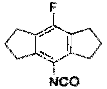 | 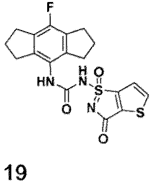 | 1. 56 % |
| | | | 2. ¹H NMR (500 MHz, DMSO-d6) δ = 8.67 (brs, 1H), 8.13 (d, 1H), 7.48 (d, 1H), 2.81 (t, 4H), 2.69 (t, 4H), 2.02-1.96 (m, 4H). |
| | | | 3. 406.4 |
| 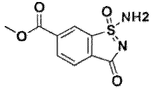 | 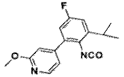 | 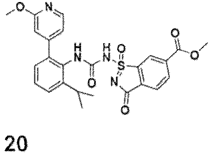 | 1. 46 % |
| | | | 2. ND |
| | | | 3. 527.3 |
| 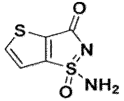 | 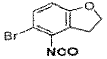 | 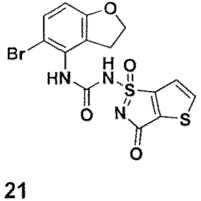 | 1. 67% |
| | | | 2. ¹H NMR (500 MHz, DMSO-d6) δ = 8.61 (brs, 1H), 8.06 (d,1H), 7.46 (d, 1H), 7.28 (d, 1H), 6.56 (.d, 1H), 4.53-4.49 (m, 2H), 3.09-3.02 (m, 2H). |
| | | | 3. 428.1 |
| 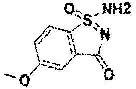 | 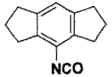 | 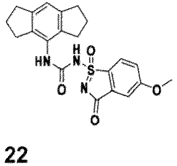 | 1. 40% |
| | | | 2. ¹H NMR (500 MHz, DMSO-d6) δ = 7.97-7.95 (m, 2H), 7.08-7.06 (m, 2H), 6.81 (s, 1H), 3.84 (s, 3H), 2.76 (t,4H), 2.65 (t, 4H), 1.93-1.87 (m, 4H). |
| | | | 3. 412.4 |
| 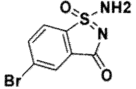 | 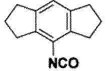 | 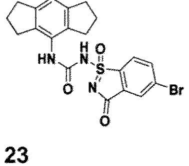 | 1. 8 % |
| | | | 2. ¹H NMR (500 MHz, DMSO-d6) δ = 8.09 (brs, 1H), 7.99 (d, 1H), 7.87 (dd,1H), 7.70 (d, 1H), 6.82 (s, 1H), 2.76 (t, 4H), 2.66 (t, 4H), 1.95-1.90 (m, 4H). |
| | | | 3. 460.1 |
| 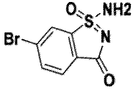 | 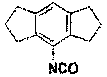 | 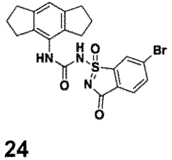 | 1. 8 % |
| | | | 2. ¹H NMR (500 MHz, DMSO-d6) δ = 8.26 (s, 1H), 8.15 (brs, 1H), 7.79 (dd,1H), 7.55 (d,1H), 6.83 (s, 1H), 2.77 (t, 4H), 2.67 (t, 4H), 1.93-1.90 (m, 4H). |
| | | | 3. 460.1 |
| 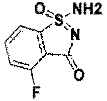 | 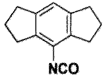 | 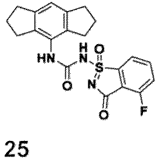 | 1. 57 % |
| | | | 2. ¹H NMR (400 MHz, DMSO-d6) δ = 8.06 (s, 1H), 7.86 (d, 1H), 7.63-7.58 (m, 1H), 7.36 (t, 1H), 6.81 (s, 1H), 2.76 (t, 4H), 2.65 (t, 4H), 1.93-1.86 (m, 4H). |
| | | | 3. 400.5 |
| 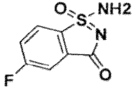 | 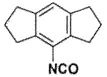 | 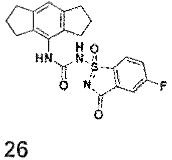 | 1. 61 % |
| | | | 2. ¹H NMR (400 MHz, DMSO) δ = 8.12-8.08 (m, 2H), 7.44-7.41 (m, 1H), 7.39-7.32 (m, 1H), 6.82 (s, 1H), 2.78-2.74 (m, 4H), 2.6 -2.64 (m, 4H), 1.94-1.87 (m, 4H). |
| | | | 3. 400.5 |
| 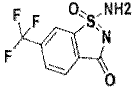 | 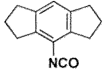 | 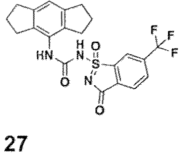 | 1. ND |
| | | | 2. ¹H NMR (400 MHz, DMSO) δ = 8.12-8.08 (m, 2H), 7.44-7.41 (m, 1H), 7.39-7.32 (m, 1H), 6.82 (s, 1H), 2.78-2.74 (m, 4H), 2.6 -2.64 (m, 4H), 1.94-1.87 (m, 4H). |
| | | | 3. 450.4 |
| 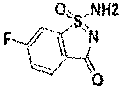 | 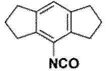 | 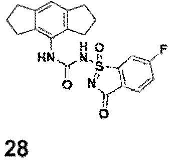 | 1. 18 % |
| | | | 2. ¹H NMR (500 MHz, DMSO-d6) δ = 8.12 (brs, 1H), 7.83 (d, 1H), 7.65-7.62 (m, 1H), 7.43 (t, 1H), 6.83 (s, 1H), 2.77 (t, 4H), 2.68-2.63 (m, 4H), 1.93-1.92 (m, 4H). |
| | | | 3. 400.2 |
| 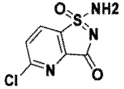 | 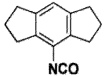 | 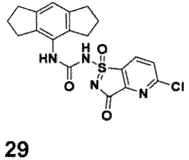 | 1. 18 % |
| | | | 2. ¹H NMR ((500 MHz, DMSO-d6) δ = 8.47 (d, 1H), 8.27 (brs, 1H), 7.71 (d, 1H), 6.83 (s, 1H), 2.76 (t, 4H), 2.65 (t, 4H), 1.94-1.88 (m, 4H). |
| | | | 3. 417.3 |

Examples 16, 17, 20, and 21 are reference examples.

### Example 30

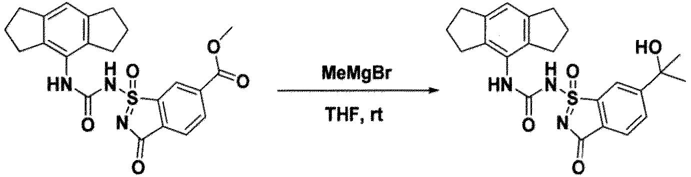

To a stirred solution of **Example 9** (15 mg, 0.034 mmol) in dry tetrahydrofuran (0.2 ml) was added a solution of 2M MeMgBr in THF (0.05 ml, 0.01 mmol) at room temperature and the mixture was stirred for 1 h. After the completion of the reaction, saturated NH₄Cl solution (1 ml) was added to the reaction mixture and the mixture was extracted with EtOAc (2 x 5 ml). The organic layer was dried over Na₂SO₄, filtered and evaporated under vacuum. The resulted residue was purified by Grace automated purifier (SiO₂ 4 g column; gradient elution of 0 to 10% MeOH in DCM). The collected pure compound fractions were evaporated and dried under vacuum to afford 1-(1,2,3,5,6,7-hexahydro-s-indacen-4-yl)-3-(6-(2-hydroxypropan-2-yl)-1-oxido-3-oxo-3H-1,4-benzo[d]isothiazol-1-yl)urea (4.4 mg, 29 %) as an off white solid.
¹H-NMR, (500 MHz, DMSO-d6) δ = 8.20 (d, 1H), 8.00 (bs, 1H), 7.70 (dd, 1H), 7.52 (d, 1H), 6.81 (s, 1H), 5.23 (s, 1H), 2.78-2.54 (m, 8H), 1.92-1.88 (m, 4H), 1.45 (s, 3H), 1.43 (s, 3H).
MS: 440.51(M+H)⁺

### Example 31

Following the procedure of **Example 30** using appropriate reagents indicated in the table below, the following compound was prepared.

**Table 7:**

| Starting Material | Product | 1. Yield |
|---|---|---|
| | **Example** | 2. ¹H-NMR |
| | | 3. MH⁺ (ESI) |
| 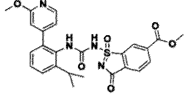 | 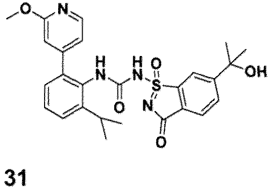 | 1. 23 % |
| | | 2. ¹H NMR (500 MHz, DMSO-d6) δ = 8.05 (s, 1H), 7.88 (s, 1H), 7.65 (s, 1H), 7.49 (s, 1H), 7.14 (s, 1H), 7.05 (d, 1H), 6.90 (d, 2 H), 6.74 (s, 1H), 4.78 (s, 1H), 3.80 (s, 3H), 3.20 (d, 1H), 1.45 (s, 6H), 1.09 (d, 6H). |
| | | 3. 527.5 |

Example 31 is a reference example.

### Separation of enantiomers:

The racemic mixture was subjected to chiral separation by SFC to afford the pure enantiomers.

### SFC Method:

| | |
|---|---|
| Column Name: | Chiralpak IC (4.6*250) mm, 5 µm; |
| Co-Solvent: | 40% |
| Co-Solvent Name: | 0.5% DEA in methanol |
| Injected Volume: | 10 µl |
| Flow Rate: | 4 ml/min |
| Outlet Pressure: | 100 bar |
| Temperature: | 30 °C |

### First eluting peak (Rt =3.34):

The fraction was evaporated under reduced pressure and the resulting residue was triturated with diethyl ether to remove diethyl amine contamination to afford **Example 1a** as an off white solid with 99.97% chiral purity.

1H-NMR, (400 MHz, DMSO-d6) δ = 8.06-8.04 (m, 1H), 7.86 (s, 1H), 7.61-7.5 (m, 3H), 6.80 (s, 1H), 2.77-2.74 (t, 4H), 2.68-2.66 (m, 4H), 1.98-1.87 (m, 4H).

MS: 382.28 (M+H)⁺.

### Second eluting peak (Rt=4.36):

The fraction was evaporated under reduced pressure and the resulting residue was triturated with diethyl ether to remove diethyl amine contamination to afford **Example 1b** as an off white solid with 97.76% chiral purity.

1H-NMR, (400 MHz, DMSO-d6) δ = 8.07-8.05 (m, 1H), 7.99 (s, 1H), 7.61-7.54 (m, 3H), 6.81 (s, 1H), 2.77-2.74 (t, 4H), 2.67-2.65 (m, 4H), 1.91-1.878 (m, 4H).

MS: 382.33 (M+H)⁺.

### Examples 32 to 64

Following the chiral separation of **Example 1,** enantiopure compounds were obtained. Different column types, eluent, temperature, pressure and flow rate could have been used.

**Table 8:**

| Racemate | **Enantiopure** | 1. ¹H-NMR |
|---|---|---|
| | **Example** | 2. MH⁺ (ESI) |
| | Eluting peak | |
| 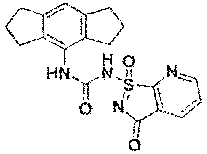 | **32** | 1. ¹H NMR (400 MHz, DMSO-d6) δ = 8.84 (dd, 1H), 8.47 (bs, 1H), 8.22 (dd, 1H), 7.72 (dd, 1H), 6.82 (s, 1H), 2.73 (t, 4H), 2.68-2.58 (m, 4H), 1.92-1.85 (m, 4H) |
| | **First** | |
| | | 2. 383.4 |
| 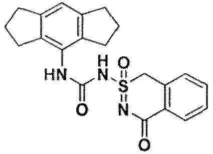 | **33** | 1. ¹H NMR (400 MHz, DMSO-d6) δ = 8.47 (brs, 1H), 8.01 (d, 1H), 7.67 (t, 1H), 7.56 (t, 1H), 7.51 (d, 1H), 6.88 (s, 1H), 5.32 (d, 1H), 5.00 (d, 1H), 2.77 (t, 4H), 2.63 (brs, 4H), 1.96-1.91 (m, 4H). |
| | **First** | |
| | | 2. 396.5 |
| 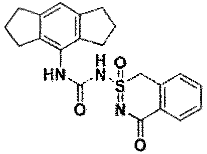 | **34** | 1. ¹H NMR (400 MHz, DMSO-d6) δ = 8.46 (brs, 1H), 8.01 (d, 1H), 7.66 (t, 1H), 7.56 (t, 1H), 7.50 (d, 1H), 6.88 (s, 1H), 5.32 (d, 1H), 5.01 (d, 1H), 2.77 (brs, 4H), 2.63 (brs, 4H), 1.91 (brs, 4H). |
| | **Second** | |
| | | 2. 396.5 |
| 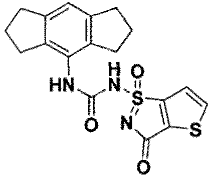 | **35** | 1. ¹H-NMR: (500 MHz, DMSO-d6) δ = 8.69 (bs, 1H), 8.16 (d, 1H), 7.51 (d, 1H), 6.88 (s, 1H), 2.77 (t, 4H), 2.65 (t, 4H), 1.94-1.88 (m, 4H). |
| | **First** | |
| | | 2. 388.3 |
| 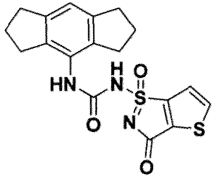 | **36** | 1. ¹H-NMR: (500 MHz, DMSO-d6) δ = 8.68 (bs, 1H), 8.16 (d, 1H), 7.51 (d, 1H), 6.88 (s, 1H), 2.77 (t, 4H), 2.65 (t, 4H), 1.94-1.88 (m, 4H). |
| | **Second** | |
| | | 2. 388.3 |
| 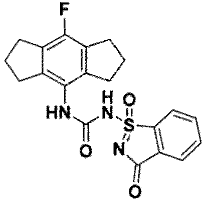 | **37** | 1. ¹H-NMR: (500 MHz, DMSO-d6) δ = 8.41 (brs, 1H), 8.11-8.09 (m,1H), 7.77-7.74 (m, 3H), 2.81-2.78 (t, 4H), 2.70-2.66 (m,4H), 2.00-1.94 (m, 4H). |
| | **Second** | |
| | | 2. 400.3 |
| 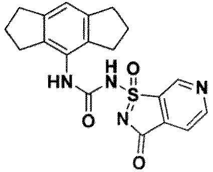 | **38** | 1. ¹H-NMR: (500 MHz, DMSO-d6) δ = 9.26 (d, 1H), 8.86 (d, 1H), 8.30 (bs, 1H), 7.68 (dd, 1H), 6.84 (s, 1H), 2.76 (t, 4H), 2.68-2.63 (m, 4H), 1.92-1.89 (m, 4H). |
| | **Second** | |
| | | 2. 383.4 |
| 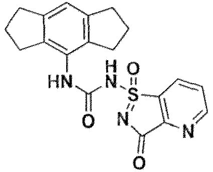 | **39** | 1. ¹H-NMR: (500 MHz, DMSO-d6) δ = 8.77 (brs, 1H), 8.44 (d, 1H), 8.13 (brs, 1H), 7.56 (s, 1H), 6.82 (s, 1H), 2.76 (t, 4H), 2.67-2.64 (m, 4H), 1.93-1.89 (m, 4H). |
| | **First** | |
| | | 2. 383.5 |
| 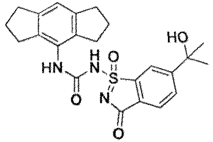 | **40** | 1. ¹H-NMR: (500 MHz, DMSO-d6) δ = 8.20 (d, 1H), 8.00 (brs, 1H), 7.70 (d, 1H), 7.52 (d, 1H), 6.81 (s, 1H), 5.23 (s, 1H), 2.78-2.54 (m, 8H), 1.93-1.86 (m, 4H), 1.45 (s, 3H), 1.43 (s, 3H). |
| | **First** | |
| | | 2. 440.4 |
| 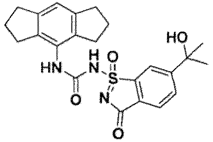 | **41** | 1. ¹H-NMR: (500 MHz, DMSO-d6) δ = 8.20 (d, 1H), 8.00 (brs, 1H), 7.70 (d, 1H), 7.52 (d, 1H), 6.81 (s, 1H), 5.23 (brs, 1H), 2.78-2.54 (m, 8H), 1.93-1.87 (m, 4H), 1.45 (s, 3H), 1.43 (s, 3H). |
| | **Second** | |
| | | 2. 440.4 |
| 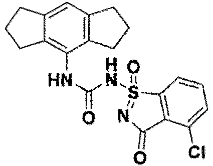 | **42** | 1. ¹H-NMR: ((500 MHz, DMSO-d6) δ = 8.31 (brs, 1H), 8.05-8.04 (m, 1H), 7.67-7.65 (m, 2H), 6.84 (s, 1H), 2.77 (t, 4H), 2.65-2.62 (m, 4H), 1.93-1.89 (m, 4H). |
| | **First** | |
| | | 2. 416.5 |
| 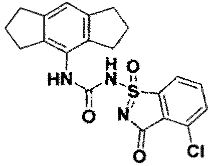 | **43** | 1. ¹H-NMR: (500 MHz, DMSO-d6) δ = 8.22 (brs, 1H), 8.05-8.04 (m, 1H), 7.64-7.61 (m, 2H), 6.83 (s, 1H), 2.77 (t, 4H), 2.64-2.62 (m, 4H), 1.93-1.89 (m, 4H). |
| | **Second** | |
| | | 2. 416.4 |
| 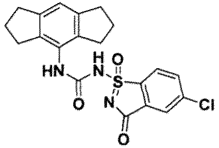 | **44** | 1. ¹H-NMR: (500 MHz, DMSO-d6) δ = 8.07-8.05 (m, 2H), 7.64 (d,1H), 7.57 (s,1H), 6.82 (s, 1H), 2.76 (t, 4H), 2.66-2.63 (m,4H), 1.92-1.89 (m, 4H). |
| | **First** | |
| | | 2. 416.4 |
| 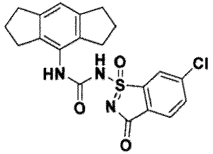 | **45** | 1. ¹H-NMR: (500 MHz, DMSO-d6) δ = 8.15-8.10 (brs, 2H), 7.67-7.60 (m,2H), 6.83 (s, 1H), 2.78-2.75 (m, 4H), 2.68-2.65 (m, 4H), 1.92-1.89 (m, 4H). |
| | **Second** | |
| | | 2. 416.2 |
| 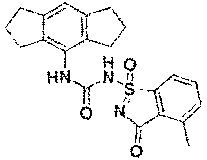 | **46** | 1. ¹H-NMR: (500 MHz, DMSO-d6) δ = 8.68 (brs, 1H), 7.95 (d,1H), 7.75 (t, 1H), 7.64 (d, 1H), 6.87(s,1H), 2.78-2.74 (m, 4H), 2.62-2.58 (m, 4H), 2.65 (bs, 3H), 1.94-1.89 (m, 4H). |
| | **First** | |
| | | 2. 396.2 |
| 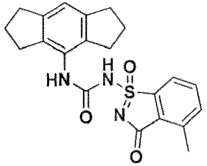 | **47** | 1. ¹H-NMR: (500 MHz, DMSO-d6) δ = 8.69 (brs, 1H), 7.95 (d,1H), 7.75 (t, 1H), 7.64 (d, 1H), 6.87 (s,1H), 2.78-2.74 (m, 4H), 2.62-2.60 (m, 4H), 2.65 (brs, 3H), 1.93-1.89 (m, 4H). |
| | **Second** | |
| | | 2. 396.2 |
| 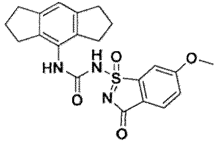 | **48** | 1. ¹H-NMR: (500 MHz, DMSO-d6) δ = 8.65 (brs,1H), 7.79 (d, 1H), 7.67 (d, 1H), 7.33 (d, 1H), 6.87 (s, 1H), 3.90 (s, 3H), 2.59-2.77 (m, 8H), 1.91 (m, 4H). |
| | **Second** | |
| | | 2. 412.5 |
| 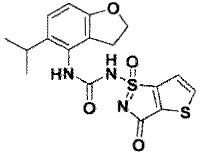 | **49** | 1. ¹H-NMR: (400 MHz, DMSO-d6) δ = 8.58 (brs,1H), 8.11 (d,1H), 7.45 (d,1H), 6.95 (d, 1H), 6.58 (d,1H), 4.46-4.41 (m, 2H), 3.07-2.93 (m, 3H), 1.06-1.03 (m, 6H). |
| | **Second** | |
| | | 2. 391.95 |
| 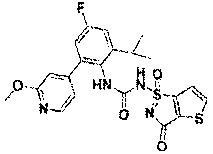 | **50** | 1. ¹H-NMR: (400 MHz, DMSO-d6) δ = 8.40 (brs, 1H), 8.03 (d, 2H), 7.22-7.17 (m, 2H), 7.01-6.98 (m, 2H), 6.74 (brs,1H), 3.81 (brs,3H),3.12 (brs, 1H), 1.12-1.10 (m,6H). |
| | **First** | |
| | | 2. 475.3 |
| 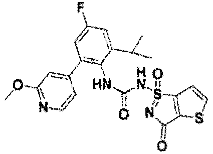 | **51** | 1. ¹H-NMR: (400 MHz, DMSO-d6) δ = 8.45 (brs,1H), 8.06 (d, 2H), 7.23-7.17 (m, 2H), 7.01-6.98 (m, 2H), 6.74 (brs, 1H), 3.81 (brs, 3H), 3.12 (brs, 1H), 1.13-1.10 (m,6H). |
| | **Second** | |
| | | 2. 475.4 |
| 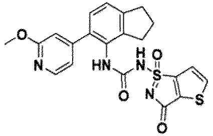 | **52** | 1. ¹H-NMR: (500 MHz, DMSO-d6) δ = 8.45 (brs, 1H), 8.0-7.99 (brs, 2H), 7.22 (d, 1H), 7.17 (d, 1H), 7.09 (d, 1H), 6.90 (d, 1H), 6.72 (s, 1H), 3.81 (s, 3H), 2.93-2.90 (m, 2H), 2.76-2.75 (m, 2H), 2.00-1.97 (m, 2H). |
| | **First** | |
| | | 2. 455.1 |
| 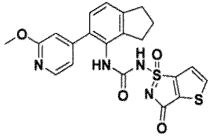 | **53** | 1. ¹H-NMR: (500 MHz, DMSO-d6) δ = 8.50 (brs, 1H), 8.02 (brs, 2H), 7.23 (d, 1H), 7.17 ( d, 1H), 7.10-7.09 (d, 1H), 6.90 (d, 1H), 6.72 (s,1H), 3.81 (s, 3H), 2.93-2.90 (m, 2H), 2.76-2.75 (m, 2H), 2.01-1.97 (m, 2H). |
| | **Second** | |
| | | 2. 455.1 |
| 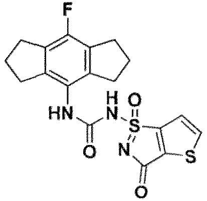 | **54** | 1. ¹H-NMR: (500 MHz, DMSO-d6) δ = 8.67 (brs, 1H), 8.13 (d, 1H), 7.48 (d, 1H), 2.81 (t, 4H), 2.69 (t, 4H), 2.02-1.96 (m, 4H). |
| | **First** | |
| | | 2. 406.4 |
| 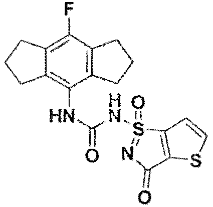 | **55** | 1. ¹H-NMR: (500 MHz, DMSO-d6) δ = 8.69 (brs, 1H), 8.15 (d, 1H), 7.50 (d, 1H), 2.81 (t, 4H), 2.69 (t, 4H), 2.02-1.96 (m, 4H). |
| | **Second** | |
| | | 2. 406.4 |
| 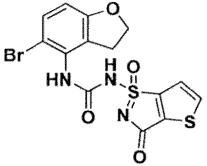 | **56** | 1. ¹H-NMR: (500 MHz, DMSO-d6) δ = 8.66 (brs, 1H), 8.10 (d,1H), 7.48 (d, 1H), 7.28 (d, 1H), 6.57 (d, 1H), 4.53-4.49 (m, 2H), 3.10-3.01 (m, 2H). |
| | **Second** | |
| | | 2. 428.03 |
| 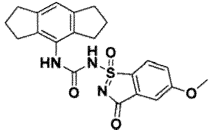 | **57** | 1. ¹H-NMR: (500 MHz, DMSO-d6) δ = 8.51 (brs, 1H), 8.03 (d, 1H), 7.32 (d, 2H), 6.87 (s, 1H), 3.90 (s, 3H), 2.75 (t, 4H), 2.67-2.55 (m, 4H), 1.94-1.88 (m, 4H). |
| | **First** | |
| | | 2. 412.4 |
| 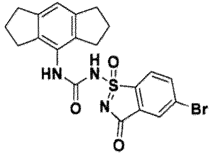 | **58** | 1. ¹H-NMR: (500 MHz, DMSO-d6) δ = 8.32 (brs, 1H), 8.03 (d, 1H), 7.87 (dd, 1H), 7.82 (d, 1H), 6.84 (s, 1H), 2.78 (t, 4H), 2.63 (t, 4H), 1.95-1.90 (m, 4H). |
| | **First** | |
| | | 2. 460.4 |
| 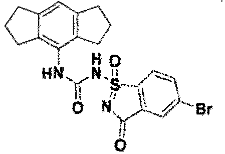 | **59** | 1. ¹H-NMR: (500 MHz, DMSO-d6) δ = 8.25 (brs, 1H), 8.02 (d, 1H), 7.84 (dd, 1H), 7.79 (d, 1H), 6.83 (s, 1H), 2.76 (t, 4H), 2.65 (t, 4H), 1.95-1.90 (m, 4H). |
| | **Second** | |
| | | 2. 460.4 |
| 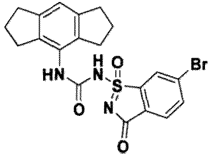 | **60** | 1. ¹H-NMR: (500 MHz, DMSO) δ = 8.45 (s, 1H), 8.33 (s, 1H), 7.93- 7.91 (m, 1H), 7.68 (d, 1H), 6.86 (s, 1H), 2.78 - 2.75 (t, 4H), 2.66 - 2.63 (m, 4H), 1.93 - 1.90 (m, 4H). |
| | **Second** | |
| | | 2. 460.1 |
| 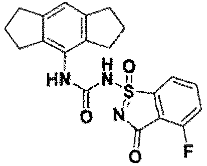 | **61** | 1. ¹H-NMR: (500 MHz, DMSO-d6) δ = 8.44 (brs,1H), 7.93 (d,1H), 7.79-7.76 (m, 1H), 7.53 (t, 1H), 6.85 (s, 1H), 2.76 (t, 4H), 2.65-2.61 (m,4H), 1.93-1.89 (m, 4H). |
| | **First** | |
| | | 2. 400.2 |
| 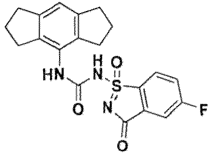 | **62** | 1. ¹H-NMR: (500 MHz, DMSO) δ = 8.49 (s, 1H), 8.20-8.17 (m, 1H), 7.63-7.58 (m, 2H), 6.86 (s, 1H), 2.76 (t, 4H), 2.65-2.61 (m, 4H), 1.92-1.88 (m, 4H). |
| | **First** | |
| | | 2. 400.3 |
| 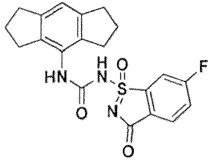 | **63** | 1. ¹H-NMR: (500 MHz, DMSO-d6) δ = 8.48 (brs,1 H), 7.98 (dd, 1H), 7.84-7.82 (m, 1H), 7.61-7.57 (m, 1H), 6.86 (s, 1H), 2.77 (t, 4H), 2.64 (brs,4H), 1.94-1.89 (m, 4H). |
| | **Second** | |
| | | 2. 400.2 |
| 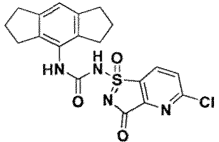 | **64** | 1. ¹H-NMR: (500 MHz, DMSO-d6) δ = 8.47 (d, 1H), 8.27 (brs, 1H), 7.72 (d,1H), 6.83 (s, 1H), 2.77-2.76 (t, 4H), 2.65 (t, 4H), 1.94-1.88 (m, 4H). |
| | **First** | |
| | | 2. 417.0 |

Enantiopure examples 49, 50, 51, and 56 are reference examples.

### BIOLOGICAL ASSAY DESCRIPTION

### NLRP3 inhibition assays

The following assays can be used to determine the inhibitory activity of test compounds on the NLRP3 inflammasome using common stimuli nigericin (Invivogen) or monosodium urate crystals (MSU) (Invivogen).

### Cell culture

Human monocyte-like cells are cultured in RPMI1640 Glutamax medium supplemented with 10% heat inactivated FCS and 50 U/ml penicillin-streptomycin (Life Technologies).

HMDM (Human Monocyte Derived Macrophage) are isolated from human blood with leucosep tubes and classical monocyte isolation kit from Miltenyi based on negative selection.

NLRP3 inflammasome activation assays Human monocyte-like cells are seeded at 75000 per well in a 96 well plate and differentiated overnight into macrophages with 10 ng/ml PMA (Phorbol Myristate Acetate). The following day, medium containing 10 ng/ml LPS (lipopolysaccharide) is added. After 3 h of LPS priming, concentrations of test compound in the range from 100 µM to 6 nM are added 30 min prior to NLRP3 inflammasome stimulation with Nigericin 3.75 µM or MSU 150 µg/ml for 3h.

Primary human monocyte-derived macrophages (HMDM) are seeded at 30000 per well in a 96 well plate and differentiated into macrophages with 10 ng/ml Human M-CSF (Macrophage Colony Stimulating Factor). At DIV 8 (Day in vitro) medium containing 10 ng/ml LPS is added. After 3h of LPS priming, concentrations of test compound in the range from 10 µM to 128 pM are added 30 min prior to NLRP3 inflammasome stimulation with Nigericin 2.5 µM or MSU 150 µg/ml for 3h.

### Measurement of IL-1beta

For IL-1β quantification, supernatants are analyzed using AlphaLISA kits according to the manufacturer's instructions (Perkin Elmer AlphaLISA AL220F).

### IC₅₀

IC₅₀ (concentration corresponding to 50% inhibition) were determined using GraphPad Prism 8.

The following example compounds were measured:

| **Examples** | **IC₅₀ Human monocyte-like cells MSU (µM)** | **IC₅₀ Human monocyte-like cells Niqericin (µM)** | **IC₅₀ HMDM cells MSU (µM)** | **IC₅₀ HMDM cells Nigericin (µM)** |
|---|---|---|---|---|
| **1** | **+++** | **++** | **+++** | **++** |
| **1b** | **+++** | **++** | **+++** | **++** |
| **2** | **+++** | **++** | **++** | |
| **3** | **+++** | **++** | **+++** | **++** |
| **4** | **+++** | **+++** | **+++** | **++** |
| **5** | **+++** | **++** | **+++** | **++** |
| **6** | **++** | **++** | **++** | |
| **7** | **+++** | **++** | **++** | |
| **8** | **++** | **++** | | |
| **9** | **++** | **++** | | |
| **10** | **+++** | **++** | | |
| **11** | **+++** | **++** | **++** | **++** |
| **12** | **++** | **++** | **++** | |
| **13** | **+++** | **++** | | |
| **14** | **+++** | **++** | **++** | **++** |
| **15** | **+++** | **+++** | | |
| **17** | **+++** | **+++** | | |
| **19** | **+++** | **+++** | **+++** | **+++** |
| **22** | **+++** | **++** | | |
| **23** | **+++** | **++** | | |
| **24** | **+++** | **++** | **++** | |
| **25** | **+++** | **++** | **+++** | |
| **26** | **+++** | **++** | **+++** | |
| **27** | **+++** | **++** | | |
| **28** | **+++** | **++** | | |
| **30** | **+++** | **+++** | **+++** | **+++** |
| **31** | **+++** | **+++** | **++** | **++** |
| **32** | **+++** | **++** | | |
| **33** | **+++** | **++** | | |
| **34** | **+++** | **+++** | **+++** | **++** |
| **35** | **+++** | **++** | **++** | |
| **36** | **+++** | **+++** | **+++** | **+++** |
| **37** | **+++** | **+++** | **+++** | **++** |
| **38** | **+++** | **++** | | |
| **39** | **++** | **++** | | |
| **40** | **+++** | **++** | **++** | |
| **41** | **+++** | **+++** | **+++** | **+++** |
| **42** | **+++** | **++** | **+++** | **++** |
| **43** | **+++** | **++** | | |
| **44** | **+++** | **++** | | |
| **45** | **+++** | **++** | | |
| **46** | **+++** | **++** | | |
| **47** | **+++** | **++** | | |
| **48** | **+++** | **+++** | **+++** | **+++** |
| **49** | **+++** | **++** | | |
| **50** | **++** | **++** | | |
| **51** | **+++** | **+++** | | |
| **52** | **++** | **++** | | |
| **53** | **+++** | **+++** | | |
| **54** | **+++** | **++** | **++** | |
| **55** | **+++** | **+++** | **+++** | **+++** |
| **56** | **++** | **+** | | |
| **57** | **+++** | **++** | | |
| **58** | **+++** | **++** | | |
| **59** | **++** | **++** | | |
| **60** | **+++** | **+++** | **+++** | **++** |
| **61** | **+++** | **++** | | |
| **62** | **+++** | **+++** | **+++** | **++** |
| **63** | **+++** | **++** | **++** | |
| **64** | **+++** | **+++** | | **++** |

| | | | | |
|---|---|---|---|---|
| Legend: +++ IC₅₀ < 1 µM; ++ IC₅₀ 1<x<10 µM; + IC₅₀ 10<x<30 µM. | | | | |

The tested compounds showed inhibition of IL-1beta release in human monocyte-like and HMDM cells using MSU or Nigericin as activators.

## Claims

1. A compound of formula (I): 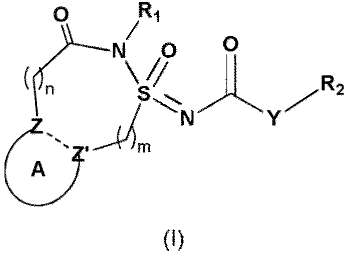
or stereoisomers, racemic mixtures, tautomers, pharmaceutically acceptable salts, hydrates, solvates and polymorphs thereof;
wherein 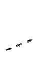 is a single or double bond;
A is selected from the group consisting of 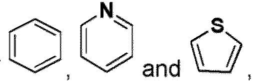 wherein each of these rings can be optionally substituted;
**R₁** is selected from the group consisting of hydrogen, alkyl, and heterocyclyl, wherein alkyl and heterocyclyl can be optionally substituted;
**R₂** is selected from the following ring systems 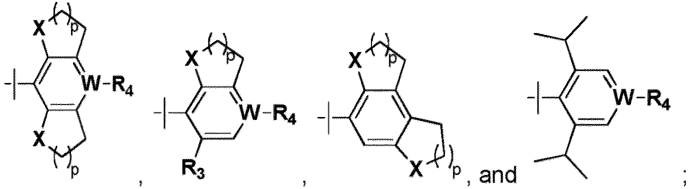
**R₃** is an optionally substituted heteroaryl;
**R₄** is selected from the group consisting of hydrogen, halogen, alkyl, alkyl-O-alkyl, aryl and heteroaryl, wherein alkyl, alkyl-O-alkyl, aryl and heteroaryl can be optionally substituted;
**W** is selected from the group consisting of C and N, in case of **W** being N no **R₄** is present;
each **X** is independently selected from the group consisting of CH₂ and O;
**Y** is NH;
each R is independently selected from the group consisting of hydrogen, halogen, CH₃, CH₂F, CHF₂, and CF₃;
**Z** and **Z'** are independently selected from the group consisting of C and N, provided that when **m** is 0, **Z'** is C;
**m** is 0 or 1;
**n** is 0 or 1; and
each **p** is independently 0 or 1.

2. The compound according to claim 1, which is a compound of formula (Ia), (Ib) or (Ic): 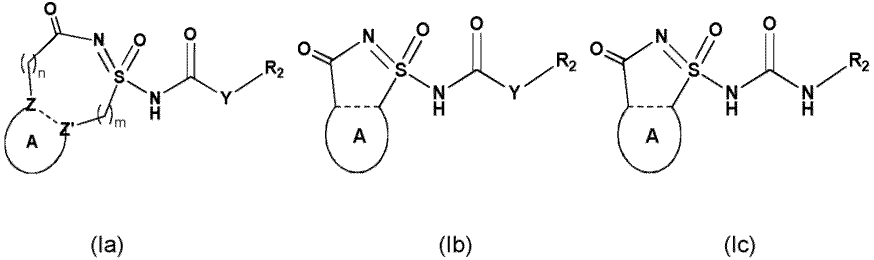 wherein 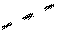 , A, **R₂**, **Y, Z, Z', m** and **n** are as defined in claim 1.

3. The compound according to claim 1, which is a compound of formula (le): 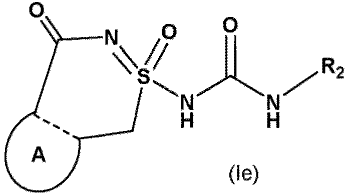 wherein 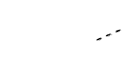 is a single or double bond,
**A** is selected from the group consisting of 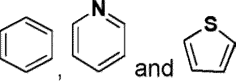 , wherein each of these rings can be optionally substituted;
**R₂** is selected from the following ring systems 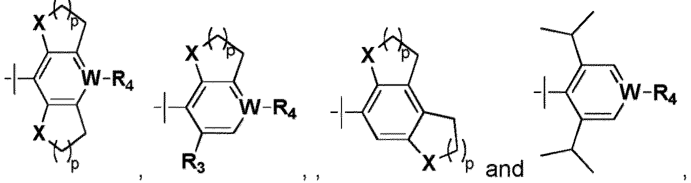 wherein
**X** are independently selected from the group consisting of CH₂ and O,
each **p** is 1,
**W** is selected from the group consisting of C and N, in case of **W** being N no **R₄** is present,
**R₃** is an optionally substituted heteroaryl, and
**R₄** is selected from the group consisting of hydrogen, halogen, alkyl, alkyl-O-alkyl, aryl and heteroaryl, wherein alkyl, alkyl-O-alkylaryl and heteroaryl can be optionally substituted.

4. The compound according to any one of claims 1 to 3, wherein **R₃** is an optionally substituted pyridine.

5. The compound according to any one of claims 1 to 4, which is selected from the list:
Example 1 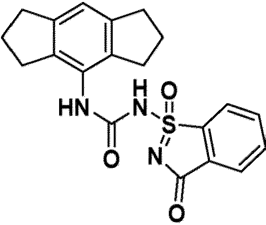 Example 4 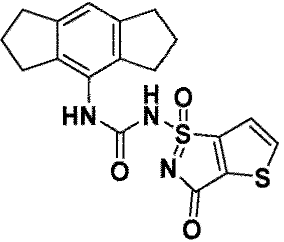
Example 5 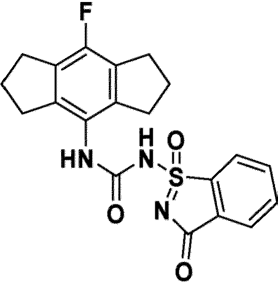 Example 6 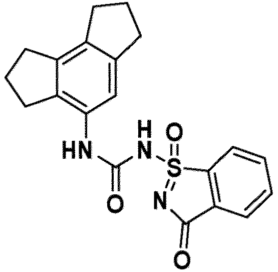
Example 10 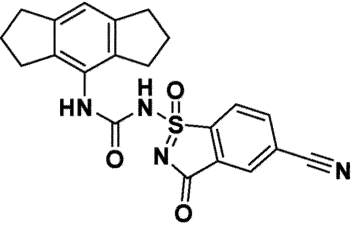 Example 25 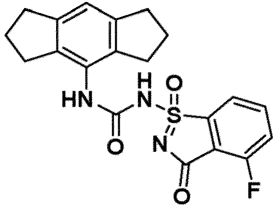
Example 26 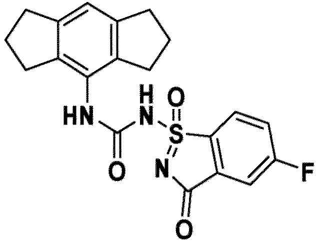 Example 28 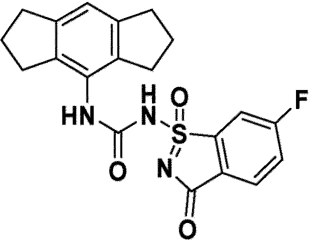
Example 30

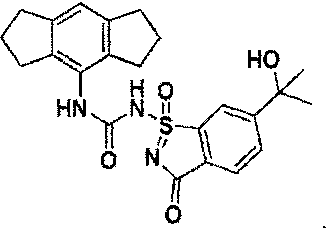

6. A pharmaceutical composition comprising a compound as defined in any one of claims 1 to 5 and optionally at least one selected from pharmaceutically acceptable excipients, carriers, diluents and adjuvants.

7. The compound as defined in any one of claims 1 to 5, or the pharmaceutical composition as defined in claim 6 for use as a medicament.

8. The compound as defined in any one of claims 1 to 5, or the pharmaceutical composition as defined in claim 6 for use in the treatment, alleviation or prevention of a disorder or abnormality responsive to modulation or inhibition of the activation of a component of the inflammasome pathway.

9. The compound for use or the pharmaceutical composition for use according to claim 8 which additionally modulates, in particular decreases, IL-1beta and/or IL-18 levels.

10. The compound for use or the pharmaceutical composition for use according to claim 8 or 9, wherein the component of the inflammasome pathway is NLRP3 inflammasome.

11. The compound as defined in any one of claims 1 to 5 or the pharmaceutical composition as defined in claim 6 for use in the treatment, alleviation or prevention of a disorder or abnormality responsive to inhibition of activation of the NLRP3 inflammasome.

12. The compound for use according to any one of claims 8 to 11, the pharmaceutical composition for use according to any one of claims 8 to 11, wherein the disease, disorder or abnormality is responsive to modulation of one or more of IL-1β, IL-17, IL-18, IL- 1 a, IL-37, IL-33 and Th17 cells.

13. The compound for use according to any one of claims 8 to 11, the pharmaceutical composition for use according to any one of claims 8 to 11, wherein the disease, disorder or abnormality is selected from Alzheimer's disease, Parkinson's disease, amyotrophic lateral sclerosis, demyelination, viral encephalitis, epilepsy, stroke, atherosclerosis, asthma, allergic inflammation, cryopyrin-associated periodic syndromes (CAPS), Muckle-Wells syndrome (MWS), familial cold autoinflammatory syndrome (FCAS), neonatal-onset multisystem inflammatory disease (NOMID), gout, pseudo-gout, inflammatory bowel disease, nonalcoholic fatty liver disease, nonalcoholic steatohepatitis, hypertension, myocardial infarction, oxalate-induced nephropathy, graft-versus host disease, type 1 diabetes, type 2 diabetes, rheumatoid arthritis, myelodysplastic syndrome, familial Mediterranean fever (FMF), TNF receptor associated periodic syndrome (TRAPS), mevalonate kinase deficiency (MKD), hyperimmunoglobulinemia D, periodic fever syndrome (HIDS), deficiency of interleukin 1 receptor (DIRA) antagonist, Majeed syndrome, pyogenic arthritis, pyoderma gangrenosum, and acne (PAPA), haploinsufficiency of A20 (HA20), PLCG2-associated antibody deficiency and immune dysregulation (PLAID), pediatric granulomatous arthritis (PGA), PLCG2-associated autoinflammation, antibody deficiency and immune dysregulation (APLAID), sideroblastic anemia with B-cell immunodeficiency, periodic fevers, developmental delay (SIFD), chronic nonbacterial osteomyelitis (CNO), Sweet's syndrome, chronic recurrent multifocal osteomyelitis (CRMO), synovitis, pustulosis, acne, hyperostosis, osteitis syndrome (SAPHO), multiple sclerosis (MS), psoriasis, Behcet's disease, Sjogren's syndrome, Schnitzler syndrome, chronic obstructive pulmonary disorder (COPD), steroid-resistant asthma, asbestosis, silicosis, cystic fibrosis, motor neuron disease, Huntington's disease, cerebral malaria, brain injury from pneumococcal meningitis, obesity, age-related macular degeneration (AMD), corneal infection, uveitis, dry eye, chronic kidney disease, diabetic nephropathy, alcoholic liver disease, skin contact hypersensitivity, sunburn, osteoarthritis, systemic juvenile idiopathic arthritis, adult-onset Still's disease, relapsing polychondritis, Chikungunya virus, Ross River virus, influenza, HIV, Coronaviruses, Dengue virus, Zika virus, hidradenitis suppurativa (HS), lung cancer metastasis, pancreatic cancers, gastric cancers, myelodisplastic syndrome, leukemia; polymyositis, colitis, helminth infection, bacterial infection, abdominal aortic aneurism, wound healing, depression, psychological stress, pericarditis including Dressler's syndrome, ischaemia reperfusion injury, frontotemporal dementia, HIV-associated neurocognitive disorder, Coronavirus-associated inflammatory pathologies, and traumatic brain injury.

14. The compound for use according to any one of claims 8 to 11, or the pharmaceutical composition for use according to any one of claims 8 to 11, wherein the disease, disorder or abnormality is selected from Alzheimer's disease, Parkinson's disease, cryopyrin-associated periodic syndromes (CAPS), nonalcoholic fatty liver disease, NASH and gout.

15. Use of the compound as defined in any of claims 1 to 5 as an analytical reference or an *in vitro* screening tool.

16. A method of producing a compound of formula (Ic) or (Id) comprising the step of isocyanate derivative coupling reaction with a compound of formula (IId) in the presence of a solvent and a base 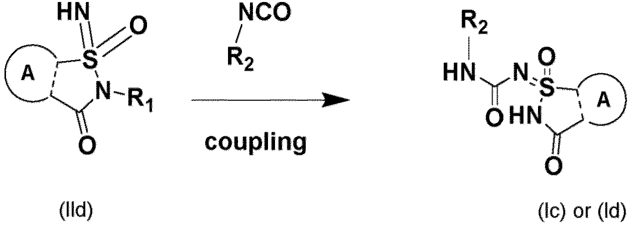 wherein **A, R₂** and **R₁** are as defined in any one of claims 1 to 4.

17. A method of producing a compound of formula (Ie) comprising the step of isocyanate derivative coupling reaction of a compound of formula (IIe) in the presence of a solvent and a base 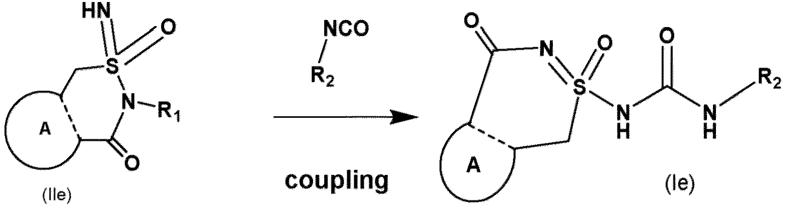 wherein **A, R₂** and **R₁** are as defined in any one of claims 1 to 4.

## Patentansprüche

1. Eine Verbindung der Formel (I): 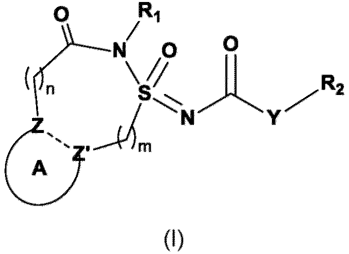
oder Stereoisomere, racemische Gemische, Tautomere, pharmazeutisch verträgliche Salze, Hydrate, Solvate und Polymorphe davon;
wobei
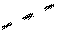 eine Einfach- oder Doppelbindung ist;
A ausgewählt ist aus der Gruppe bestehend aus 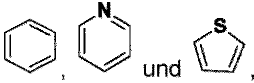 wobei jeder dieser Ringe gegebenenfalls substituiert sein kann;
**R₁** ausgewählt ist aus der Gruppe bestehend aus Wasserstoff, Alkyl und Heterocyclyl, wobei Alkyl und Heterocyclyl gegebenenfalls substituiert sein können;
**R₂** aus den nachstehenden Ringsystemen ausgewählt ist 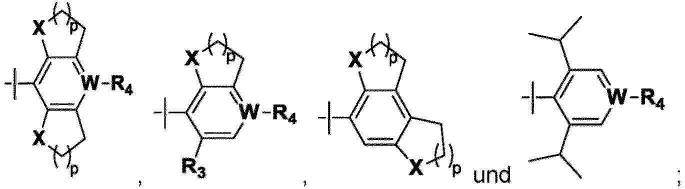
**R₃** ein gegebenenfalls substituiertes Heteroaryl ist;
**R₄** ausgewählt ist aus der Gruppe bestehend aus Wasserstoff, Halogen, Alkyl, Alkyl-O-Alkyl, Aryl und Heteroaryl, wobei Alkyl, Alkyl-O-Alkyl, Aryl und Heteroaryl gegebenenfalls substituiert sein können;
**W** ausgewählt ist aus der Gruppe bestehend aus C und N, wenn **W** für N steht, kein **R₄** vorhanden ist;
jedes **X** unabhängig ausgewählt ist aus der Gruppe bestehend aus CH₂ und O;
**Y** für NH steht;
jedes **R** unabhängig ausgewählt ist aus der Gruppe bestehend aus Wasserstoff, Halogen, CH₃, CH₂F, CHF₂ und CF₃;
**Z** und **Z'** unabhängig ausgewählt sind aus der Gruppe bestehend aus C und N, mit der Maßgabe, dass, wenn m gleich 0 ist, **Z'** für C steht;
**m** gleich 0 oder 1 ist;
**n** gleich 0 oder 1 ist; und
jedes **p** unabhängig 0 oder 1 bedeutet,

2. Die Verbindung gemäß Anspruch 1, welche eine Verbindung der Formel (la), (Ib) oder (Ic) ist: 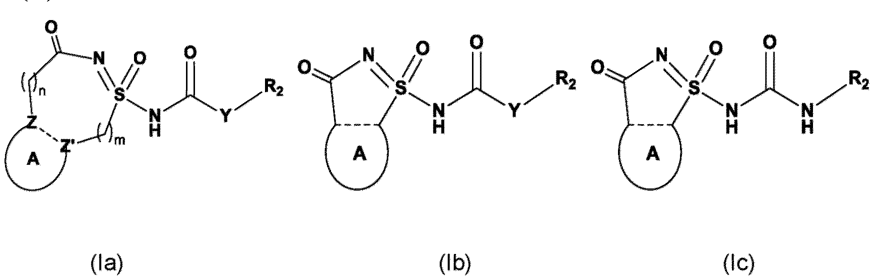 wobei 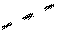 , **A, R₂, Y, Z, Z', m** und **n** wie in Anspruch 1 definiert sind.

3. Die Verbindung gemäß Anspruch 1, welche eine Verbindung der Formel (Ie) ist: 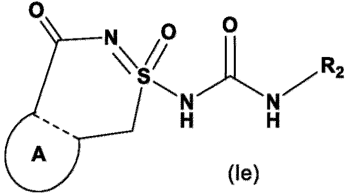 wobei
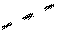 eine Einfach- oder Doppelbindung ist,
**A** ausgewählt ist aus der Gruppe bestehend aus 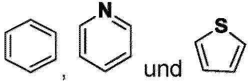 , wobei jeder dieser Ringe gegebenenfalls substituiert sein kann;
**R**² aus den nachstehenden Ringsystemen ausgewählt ist 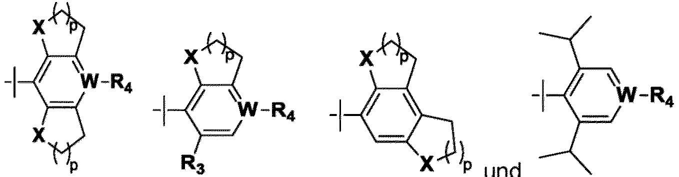 wobei
**X** unabhängig ausgewählt ist aus der Gruppe bestehend aus CH₂ und O,
jedes **p** gleich 1 ist,
**W** ausgewählt ist aus der Gruppe bestehend aus C und N, wenn **W** für N steht, kein **R₄** vorhanden ist,
**R³** ein gegebenenfalls substituiertes Heteroaryl ist, und
**R₄** ausgewählt ist aus der Gruppe bestehend aus Wasserstoff, Halogen, Alkyl, Alkyl-O-Alkyl, Aryl und Heteroaryl, wobei Alkyl, Alkyl-O-Alkyl, Aryl und Heteroaryl gegebenenfalls substituiert sein können.

4. Die Verbindung gemäß einem der Ansprüche 1 bis 3, wobei **R₃** ein gegebenenfalls substituiertes Pyridin ist.

5. Die Verbindung gemäß einem der Ansprüche 1 bis 4, welche ausgewählt ist aus der Liste:
Beispiel 1 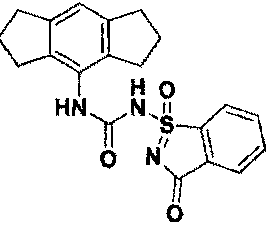 Beispiel 4 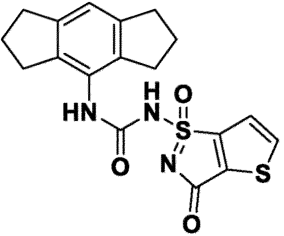
Beispiel 5 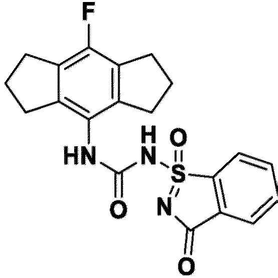 Beispiel 6 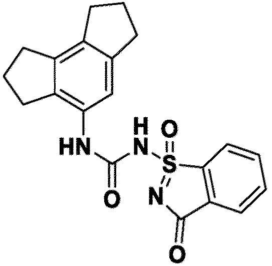
Beispiel 10 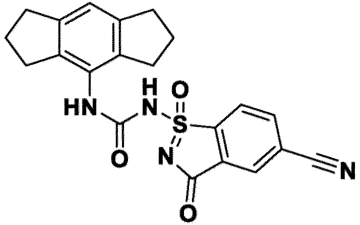 Beispiel 25 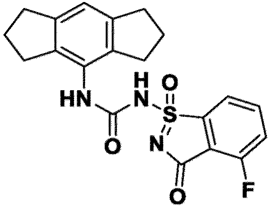
Beispiel 26 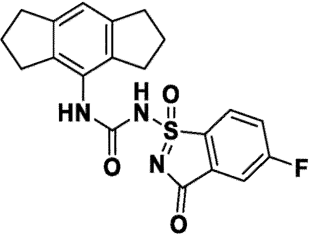 Beispiel 28 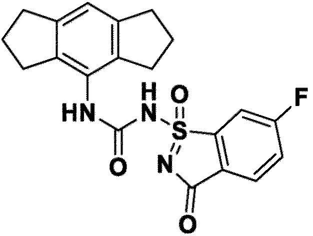
Beispiel 30

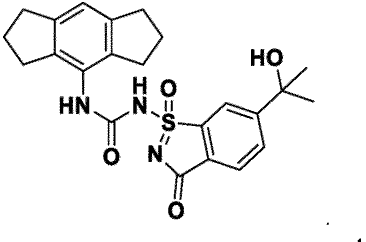

6. Ein Arzneimittel, umfassend eine Verbindung wie in einem der Ansprüche 1 bis 5 definiert und gegebenenfalls mindestens eines, ausgewählt aus pharmazeutisch verträglichen Exzipienten, Trägern, Verdünnungsmitteln and Hilfsmitteln.

7. Die Verbindung wie in einem der Ansprüche 1 bis 5 definiert oder das Arzneimittel wie in Anspruch 6 definiert zur Verwendung als ein Medikament.

8. Die Verbindung wie in einem der Ansprüche 1 bis 5 definiert oder das Arzneimittel wie in Anspruch 6 definiert zur Verwendung bei der Behandlung, Linderung oder Vorbeugung einer Störung oder Anomalie, welche auf Modulation oder Hemmung der Aktivierung einer Komponente des Inflammasom-Wegs anspricht.

9. Die Verbindung zur Verwendung oder das Arzneimittel zur Verwendung gemäß Anspruch 8, welches zusätzlich den IL-1beta- und/oder IL-18-Spiegel moduliert, insbesondere senkt.

10. Die Verbindung zur Verwendung oder das Arzneimittel zur Verwendung gemäß Anspruch 8 oder 9, wobei die Komponente des Inflammasom-Wegs NLRP3-Inflammasom ist.

11. Die Verbindung wie in einem der Ansprüche 1 bis 5 definiert oder das Arzneimittel wie in Anspruch 6 definiert zur Verwendung bei der Behandlung, Linderung oder Vorbeugung einer Störung oder Anomalie, welche auf die Hemmung der Aktivierung des NLRP3 Inflammasoms anspricht.

12. Die Verbindung zur Verwendung gemäß einem der Ansprüche 8 bis 11, das Arzneimittel zur Verwendung gemäß einem der Ansprüche 8 bis 11, wobei die Erkrankung, Störung oder Anomalie auf Modulation eines oder mehrerer von IL-1β, IL-17, IL-18, IL-1 a, IL-37, IL-33 und Th17-Zellen anspricht.

13. Die Verbindung zur Verwendung gemäß einem der Ansprüche 8 bis 11, das Arzneimittel zur Verwendung gemäß einem der Ansprüche 8 bis 11, wobei die Erkrankung, Störung oder Anomalie ausgewählt ist aus Alzheimer-Krankheit, Parkinson-Krankheit, amyotropher Lateralsklerose, Demyelisierung, viraler Enzephalitis, Epilepsie, Schlaganfall, Atherosklerose, Asthma, allergischer Entzündung, Kryopyrin-assoziierten periodischen Syndromen (CAPS), Muckle-Wells-Syndrom (MWS), familärem kälteinduziertem autoinflammatorischem Syndrom (FCAS), Neonatal-Onset Multisystem Inflammatory Disease (NOMID), Gicht, Pseudo-Gicht, Reizdarmsyndrom, nichtalkoholischer Fettlebererkrankung, nichtalkoholischer Steatohepatitis, Bluthochdruck, Myokardinfarkt, Oxalat-induzierte Nephropathie, Graftversus-Host-Erkrankung, Diabetes Typ 1, Diabetes Typ 2, rheumatoider Arthritis, myelodysplastischem Syndrom, familiärem Mittelmeerfieber (FMF), TNF-Rezeptor assoziiertem periodischem Syndrom (TRAPS), Mevalonatkinase-Defizienz (MKD), Hyperimmunoglobulinämie D, periodischem Fiebersyndrom (HIDS), Mangel an Interleukin-1-Rezeptor-Antagonisten (DIRA), Majeed-Syndrom, pyogener Arthritis, Pyoderma gangraenosum, und Akne (PAPA), Haploinsuffizienz von A20 (HA20), PLCG2-assoziertem Antikörpermangel und Immundysregulierung (PLAID), pädiatrischer granulomatöser Arthritis (PGA), PLCG2-assoziierter Autoinflammation, Mangel an Antikörpern und Immundysregulation (APLAID), sideroblastischer Anämie mit B-Zellen-Immunschwäche, periodischen Fiebern, Entwicklungsstörung (SIFD), chronischer nichtbakterieller Osteomyelitis (CNO), Sweet-Syndrom, chronischer rezidivierender multifokaler Osteomyelitis (CRMO), Synovitis, Pustulose, Akne, Hyperostose, SAPHO-Syndrom, Multipler Sklerose (MS), Psoriasis, Morbus Behcet, Sjogren-Syndrom, Schnitzler-Syndrom, chronisch obstruktiver Lungenerkrankung (COPD), Steroid-resistentem Asthma, Asbestose, Silikose, zystischer Fibrose, Motoneuronerkrankung, Chorea Huntington, zerebraler Malaria, Hirnschädigung durch Pneumokokkenmeningitis, Adipositas, altersbedingter Makuladegeneration (AMD), Hornhautentzündung, Uveitis, trockenem Auge, chronischer Nierenerkrankung, diabetischer Nephropathie, alkoholischer Lebererkrankung, Kontakthypersensibilitätshautreaktion, Sonnenbrand, Osteoarthritis, systemischer juveniler idiopathischer Arthritis, Morbus Still bei Erwachsenen, rezidivierender Polychondritis, Chikungunya-Virus, Ross-River-Virus, Grippe, HIV, Coronaviren, Dengue-Virus, Zika-Virus, Hidradenitis Suppurativa (HS), Lungenkrebsmetastasen, Bauchspeicheldrüsenkrebs, Magenkrebs, myelodisplastischem Syndrom, Leukämie; Polymyositis, Colitis, Wurminfektion, bakterieller Infektion, Bauchaortenaneurisma, Wundheilung, Depression, psychischem Stress, Perikarditis einschließlich Dressier-Syndrom, Ischämie-Reperfusionsverletzung, frontotemporaler Demenz, HIVassoziierter neurokognitiver Störung, Coronavirus-assoziierter entzündlicher Pathologien und traumatischer Hirnverletzung.

14. Die Verbindung zur Verwendung gemäß einem der Ansprüche 8 bis 11 oder das Arzneimittel zur Verwendung gemäß einem der Ansprüche 8 bis 11, wobei die Erkrankung, Störung oder Anomalie ausgewählt ist aus Alzheimer-Krankheit, Parkinson-Krankheit, Kryopyrin-assoziierter periodischer Syndrome (CAPS), nichtalkoholischer Fettlebererkrankung, NASH und Gicht.

15. Verwendung der Verbindung wie in einem der Ansprüche 1 bis 5 definiert als eine analytische Referenz oder ein *in vitro* Screening-Tool.

16. Ein Verfahren zur Herstellung einer Verbindung der Formel (Ic) oder (Id), umfassend den Schritt der Isocyanatderivat-Kupplungsreaktion mit einer Verbindung der Formel (IId) in Gegenwart eines Lösungsmittels und einer Base 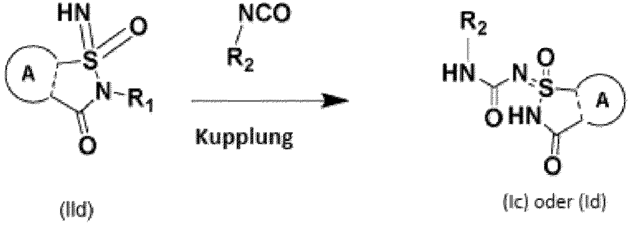 wobei **A, R₂** und **R₁** wie in einem der Ansprüche 1 bis 4 definiert sind.

17. Ein Verfahren zur Herstellung einer Verbindung der Formel (Ie), umfassend den Schritt der Isocyanatderivat-Kupplungsreaktion einer Verbindung der Formel (IIe) in Gegenwart eines Lösungsmittels und einer Base 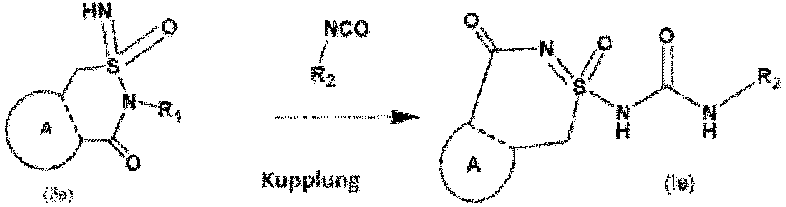 wobei **A, R₂** und **R₁** wie in einem der Ansprüche 1 bis 4 definiert sind.

## Revendications

1. Composé de formule (I) : 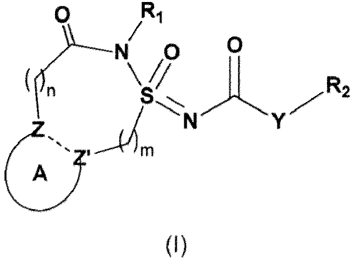
ou ses stéréoisomères, mélanges racémiques, tautomères, sels pharmaceutiquement acceptables, hydrates, solvates et polymorphes ;
dans lequel
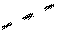 est une liaison simple ou double ;
A est choisi dans le groupe constitué par 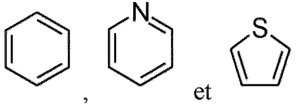 où chacun des ces cycles peut être éventuellement substitué ;
R₁ est choisi dans le groupe constitué par un hydrogène, alkyle, et hétérocyclyle, parmi lesquels les alkyle et hétérocyclyle peuvent être éventuellement substitués ;
R₂ est choisi parmi les systèmes cycliques suivants 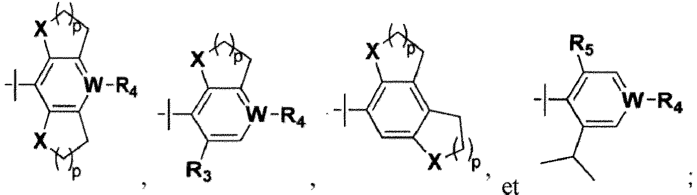
R₃ est un hétéroaryle éventuellement substitué ;
R₄ est choisi dans le groupe constitué par un hydrogène, halogène, alkyle, alkyl-O-alkyle, aryle et hétéroaryle, parmi lesquels les alkyle, alkyl-O-alkyle, aryle et hétéroaryle peuvent être éventuellement substitués ;
W est choisi dans le groupe constitué par C et N, dans le cas où W est N, aucun R₄ n'est présent,
chaque X est indépendamment choisi dans le groupe constitué par CH₂ et O ;
Y estNH;
chaque R est indépendamment choisi dans le groupe constitué par un hydrogène, halogène, CH₃, CH₂F, CHF₂, et CF₃ ;
Z et Z' sont indépendamment choisis dans le groupe constitué par C et N, sous réserve que, quand m vaut 0, Z' soit C ;
m vaut 0 ou 1 ;
n vaut 0 ou 1 ; et
chaque p vaut indépendamment 0 ou 1.

2. Composé selon la revendication 1, qui est un composé de formule (Ia), (Ib) ou (Ic) : 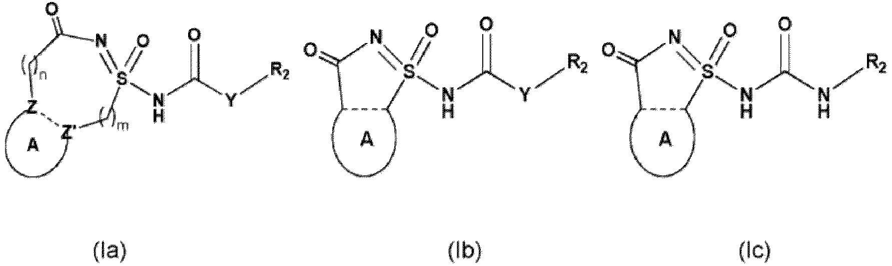 dans lequel 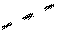 , A, R₂, Y, Z, Z', m et n sont tels que définis dans la revendication 1.

3. Composé selon la revendication 1, qui est un composé de formule (Ie) : 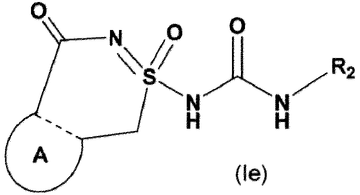 dans lequel 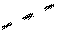
est une liaison simple ou double ;
A est choisi dans le groupe constitué par 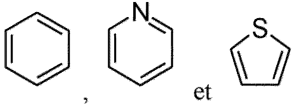 où chacun des ces cycles peut être éventuellement substitué ;
R₂ est choisi parmi les systèmes cycliques suivants 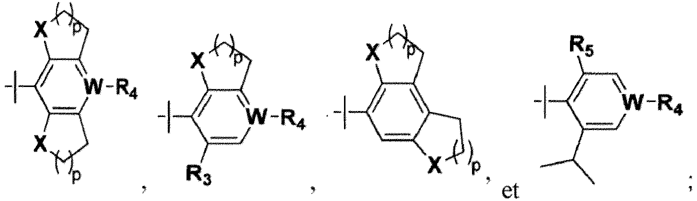 où
les X sont indépendamment choisis dans le groupe constitué par CH₂ et O, chaque p vaut 1,
W est choisi dans le groupe constitué par C et N, dans le cas où W est N, aucun R₄ n'est présent,
R₃ est un hétéroaryle éventuellement substitué, et
R₄ est choisi dans le groupe constitué par un hydrogène, halogène, alkyle, alkyl-O-alkyle, aryle et hétéroaryle, parmi lesquels les alkyle, alkyl-O-alkyle, aryle et hétéroaryle peuvent être éventuellement substitués.

4. Composé selon l'une quelconque des revendications 1 à 3, dans lequel R₃ est une pyridine éventuellement substituée.

5. Composé selon l'une quelconque des revendications 1 à 4, qui est choisi dans la liste suivante :
Exemple 1 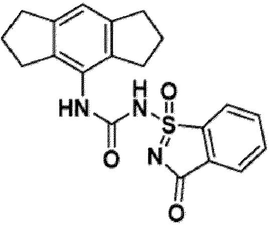 Exemple 4 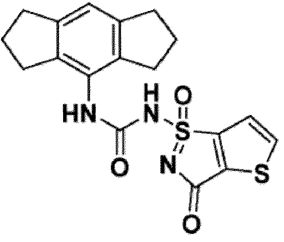 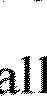
Exemple 5 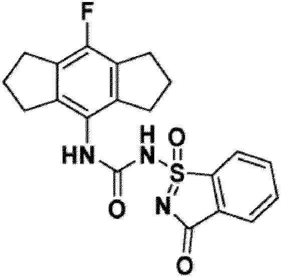 Exemple 6 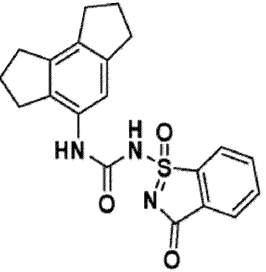
Exemple 10 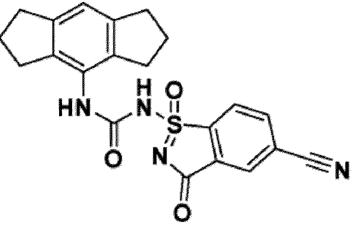 Exemple 25 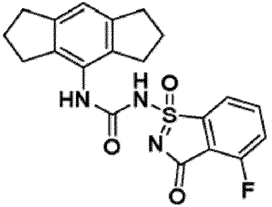
Exemple 26 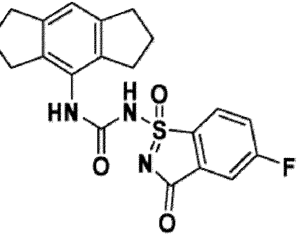 Exemple 28 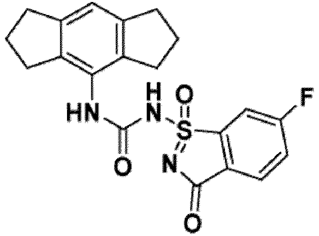
Exemple 30

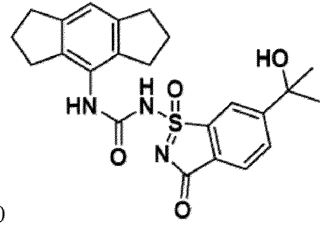

6. Composition pharmaceutique comprenant un composé tel que défini dans l'une quelconque des revendications 1 à 5 et éventuellement au moins l'un choisi parmi les excipients, véhicules, diluants et adjuvants pharmaceutiquement acceptables.

7. Composé selon l'une quelconque des revendications 1 à 5, ou composition pharmaceutique selon la revendication 6, pour une utilisation en tant que médicament.

8. Composé selon l'une quelconque des revendications 1 à 5, ou composition pharmaceutique selon la revendication 6, pour une utilisation dans le traitement, le soulagement ou la prévention d'un trouble ou d'une anomalie sensible à une modulation ou à une inhibition de l'activation d'un composant de la voie de l'inflammasome.

9. Composé pour une utilisation ou composition pharmaceutique pour une utilisation selon la revendication 8, qui de plus module, en particulier diminue, les niveaux d'IL-1bêta et/ou d'IL-18.

10. Composé pour une utilisation ou composition pharmaceutique pour une utilisation selon la revendication 8 ou 9, dans lequel/laquelle le composant de la voie de l'inflammasome est l'inflammasome NLRP3.

11. Composé selon l'une quelconque des revendications 1 à 5, ou composition pharmaceutique selon la revendication 6, pour une utilisation dans le traitement, le soulagement ou la prévention d'un trouble ou d'une anomalie sensible à une inhibition de l'activation de l'inflammasome NLRP3.

12. Composé pour une utilisation selon l'une quelconque des revendications 8 à 11 ou composition pharmaceutique selon l'une quelconque des revendications 8 à 11, dans lequel/laquelle la maladie, le trouble ou l'anomalie est sensible à la modulation d'une ou plusieurs cellules parmi IL-1β, IL-17, IL-18, IL-la, IL-37, IL-33 et Th17.

13. Composé pour une utilisation selon l'une quelconque des revendications 8 à 11 ou composition pharmaceutique selon l'une quelconque des revendications 8 à 11, dans lequel/laquelle la maladie, le trouble ou l'anomalie est choisi parmi une maladie d'Alzheimer, une maladie de Parkinson, une sclérose latérale amyotrophique, une démyélinisation, une encéphalite virale, une épilepsie, un accident vasculaire cérébral, une athérosclérose, un asthme, une inflammation allergique, des syndromes périodiques associés à la cryopyrine (CAPS), un syndrome de Muckle-Wells (MWS), un syndrome auto-inflammatoire froid familial (FCAS), une maladie inflammatoire multisystémique à révélation néonatale (NOMID, syndrome CINCA), une goutte, une pseudo-goutte, une maladie intestinale inflammatoire, une hépatite graisseuse non alcoolique, une stéatose hépatique non alcoolique, une hypertension, un infarctus du myocarde, une néphropathie induite par les oxalates, une réaction du greffon contre l'hôte, un diabète de type 1, un diabète de type 2, une polyarthrite rhumatoïde, un syndrome myélodysplasique, une fièvre méditerranéenne familiale (FMF), un syndrome périodique associé au récepteur de TNF (TRAPS), une insuffisance en mévalonate kinase (MKD), une hyperimmunoglobulinémie D, un syndrome de fièvre périodique (HIDS), une insuffisance en antagoniste de récepteur d'interleukine 1 (DIRA), un syndrome de Majeed, un syndrome d'arthrite pyogénique, pyodermite phagédénique et acné (PAPA), une haplo-insuffisance en A20 (HA20), une dérégulation immunitaire avec insuffisance en anticorps associés à PLCG2 (PLAID), une arthrite granulomateuse pédiatrique (PGA), une auto-inflammation associée à PLCG2, une dérégulation immunitaire avec insuffisance en anticorps (APLAID), un syndrome d'anémie sidéroblastique avec insuffisance en cellules B, fièvres périodiques et retard de développement (SIFD), une ostéomyélite non bactérienne chronique (CNO), un syndrome de Sweet, une ostéomyélite multifocale récurrente chronique (CRMO), un syndrome de synovite, pustulose, acné, hyperostose et ostéite (SAPHO), une sclérose en plaques (SEP), un psoriasis, une maladie de Behcet, un syndrome de Sjogren, un syndrome de Schnitzler, une broncho-pneumopathie chronique obstructive (BPCO), un asthme résistant aux stéroïdes, une amiantose, une silicose, une fibrose kystique, une maladie des neurones moteurs, une maladie de Huntington, un paludisme cérébral, une lésion cérébrale suite à une méningite à pneumocoques, une obésité, une dégénérescence maculaire liée à l'âge (AMD), une infection de la cornée, une uvéite, une sécheresse oculaire, une maladie rénale chronique, une néphropathie diabétique, une maladie hépatique alcoolique, une hypersensibilité de contact de la peau, un coup de soleil, une arthrose, une arthrite idiopathique juvénile systémique, une maladie de Still se révélant à l'âge adulte, une polychondrite récurrente, le virus Chikungunya, le virus Ross River, une grippe, le VIH, les coronavirus, un virus de la Dengue, le virus Zika, une hidradénite suppurée (HS), des métastases de cancer du poumon, des cancers du pancréas, des cancers gastriques, un syndrome myélodysplasique, une leucémie, une polymyosite, une colite, une infection par des helminthes, une infection bactérienne, un anévrisme aortique abdominal, une cicatrisation, une dépression, un stress psychologique, une péricardique y compris un syndrome de Dressler, une lésion par reperfusion ischémique, une démence fronto-temporale, un trouble neurocognitif associé au VIH, des pathologies inflammatoires associées à un coronavirus, et un traumatisme crânien.

14. Composé pour une utilisation selon l'une quelconque des revendications 8 à 11 ou composition pharmaceutique selon l'une quelconque des revendications 8 à 11, dans lequel/laquelle la maladie, le trouble ou l'anomalie est choisi parmi une maladie d'Alzheimer, une maladie de Parkinson, des syndromes périodiques associés à la cryopyrine (CAPS), une hépatite graisseuse non alcoolique, une NASH et une goutte.

15. Utilisation du composé tel que défini dans l'une quelconque des revendications 1 à 5 en tant que référence analytique ou outil de criblage *in vitro.*

16. Méthode de production d'un composé de formule (Ic) ou (Id), comprenant l'étape de réaction de couplage d'un dérivé isocyanate avec un composé de formule (IId) en présence d'un solvant et d'une base 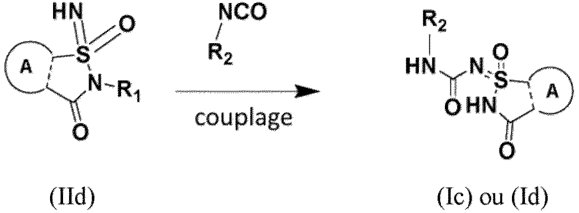 dans laquelle A, R₂ et R₁ sont tels que définis dans l'une quelconque des revendications 1 à 4.

17. Méthode de production d'un composé de formule (Ie), comprenant l'étape de réaction de couplage avec un dérivé isocyanate d'un composé de formule (IIe) en présence d'un solvant et d'une base 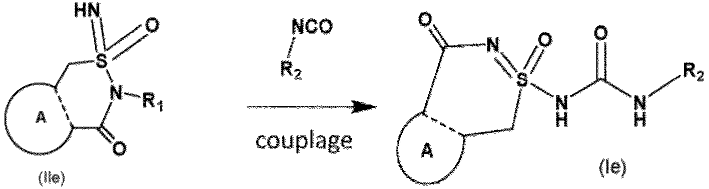 dans laquelle A, R₂ et Ri sont tels que définis dans l'une quelconque des revendications 1 à 4.
